# EUROPEAN PATENT APPLICATION

(11) **EP 4 840 329 A2**
(43) Date of publication of application: **18.11.2026**
(21) Application number: 26179290.7
(22) Date of filing: 24.11.2021
(51) Int. Cl.: A24B 15/30

(54) **ORAL POUCH PRODUCT**

(30) Priority: 06.04.2021 US 202117223773
(62) Document number(s) of the earlier application(s) in accordance with Art. 76 EPC: 21831155.3 / 4 319 570
(71) Applicant: Altria Client Services LLC, Richmond, VA 23230 (US)
(72) Inventor: Black, Shannon M., Richmond, 23219 (US); Gao, Feng, Richmond, 23219 (US); Crawford, Danielle R., Richmond, 23219 (US)
(74) Representative: Richardt Patentanwälte PartG mbB

(57) **Abstract**

An oral pouch product includes an outer wrapper defining a cavity and an inner filling material in the cavity. The outer wrapper includes a hydrophobic material or an elastomeric material. The inner filling material includes a dry mixture and a liquid mixture. The dry mixture includes a cellulosic material. The liquid mixture includes a triglyceride and liquid nicotine dissolved in the triglyceride. The triglyceride is included in the oral pouch product in an amount of 10% to 30% by weight based on the weight of the oral pouch product.

## Description

### BACKGROUND

### Field

The present disclosure relates to oral pouch products including nicotine.

### Description of Related Art

Oral nicotine products are available in a variety of formats, such as gums, sprays, lozenges, dissolvable tablets, non-dissolvable chews, films, gels, capsules, sticks (e.g., coated wooden dowels or singular dissolvable sticks), and pouches (e.g., containing fibers or granules). Oral products may have nicotine levels that create a familiar experience for adult tobacco consumers.

### SUMMARY

At least one example embodiment relates to an oral pouch product.

In at least one example embodiment, an oral pouch product comprises an outer wrapper defining a cavity and an inner filling material in the cavity. The outer wrapper includes a hydrophobic material. The inner filling material includes a dry mixture and a liquid mixture. The dry mixture includes a cellulosic material. The liquid mixture includes a triglyceride and liquid nicotine dissolved in the triglyceride. The triglyceride included in the oral pouch product in an amount of 10% to 30% by weight based on the weight of the oral pouch product.

In at least one example embodiment, the triglyceride includes a medium-chain triglyceride. In at least one example embodiment, the liquid mixture is free of water. In at least one example embodiment, the oral pouch product has a moisture content of 4% to 8%.

In at least one example embodiment, the liquid mixture is absorbed in the cellulosic material. In at least one example embodiment, the cellulosic material includes microcrystalline cellulose.

In at least one example embodiment, the liquid nicotine is present in the oral pouch product in an amount ranging from 4 mg per pouch to 12 mg per pouch.

In at least one example embodiment, the filler further comprises at least one additive. In at least one example embodiment, the additive comprises a sweetener, a pH adjuster, a flavorant, a sensate, or any combination thereof.

In at least one example embodiment, the outer wrapper includes at least one seam. In at least one example embodiment, the oral pouch product weighs 0.25 gram to 2.0 grams. In at least one example embodiment, the outer wrapper is a non-woven material.

At least one example embodiment relates to an oral pouch product.

In at least one example embodiment, an oral pouch product includes an outer wrapper defining a cavity and an inner filling material in the cavity. The outer wrapper includes an elastomeric material. The inner filling material includes a dry mixture and a liquid mixture. The dry mixture includes a cellulosic material. The liquid mixture includes a triglyceride and liquid nicotine dissolved in the triglyceride. The triglyceride is included in the oral pouch product in an amount of 10% to 30% by weight based on the weight of the oral pouch product. The oral pouch product has a moisture content of 4% to 8%.

In at least one example embodiment, the triglyceride includes a medium-chain triglyceride.

In at least one example embodiment, the liquid mixture is free of water.

In at least one example embodiment, the elastomeric material is a non-woven material including polyurethane. In at least one example embodiment, the outer wrapper has no seams.

In at least one example embodiment, the filler further comprises at least one additive. In at least one example embodiment, the additive comprises a sweetener, a pH adjuster, a flavorant, a sensate, or any combination thereof.

In at least one example embodiment, the oral pouch product weights about 0.25 gram to 2.0 grams.

### BRIEF DESCRIPTION OF THE DRAWINGS

The various features and advantages of the non-limiting embodiments herein may become more apparent upon review of the detailed description in conjunction with the accompanying drawings. The accompanying drawings are merely provided for illustrative purposes and should not be interpreted to limit the scope of the claims. The accompanying drawings are not to be considered as drawn to scale unless explicitly noted. For purposes of clarity, various dimensions of the drawings may have been exaggerated.
FIG. 1 is a flow diagram illustrating a method for forming a nicotine-containing powder in accordance with at least one example embodiment.
FIG. 2 is flow diagram illustrating a method for forming a nicotine-containing powder in accordance with at least one example embodiment.
FIG. 3 is a flow diagram illustrating a method for forming a nicotine-containing powder in accordance with at least one example embodiment.
FIG. 4 is a flow diagram illustrating a method for forming nicotine-containing powder in accordance with at least one example embodiment.
FIG. 5 is a cross-sectional illustration of an encapsulated nicotine granule in accordance with at least one example embodiment.
FIG. 6 is a cross-sectional illustration of an encapsulated nicotine granule in accordance with at least one example embodiment.
FIG. 7 is a flow diagram illustrating a method for forming encapsulated nicotine granules in accordance with at least one example embodiment.
FIG. 8 is a cross-sectional illustration of an encapsulated sweetener in accordance with at least one example embodiment.
FIG. 9 is a cross-sectional illustration of an encapsulated sweetener granules in accordance with at least one example embodiment.
FIG. 10 is a flow diagram illustrating a method for forming encapsulated sweetener granules in accordance with at least one example embodiment.
FIGS. 11A-11C depict chemical structures of nicotine in different forms. FIG. 11A is a chemical structure of free-base nicotine. FIG. 11B is a chemical structure of mono-protonated nicotine. FIG. 11C is a chemical structure of diprotonated nicotine.
FIGS. 12A-12B are a graphs depicting buccal nicotine disposition for different nicotine solutions.
FIG. 13A is a table depicting partition coefficient data for nicotine in different oil and aqueous phases. FIG. 13A is a table depicting partition coefficient data for nicotine in different oil and water phase combinations. FIG. 13B depicts of a chemical structure of triacetin (C2). FIG. 13C depicts a chemical structure of MCT (C8-C10). FIG. 13D depicts a chemical structure of triolein (C18).
FIG. 14 is a perspective view of a pouched product according to at least one example embodiment.
FIG. 15 is a perspective view of a dissolvable film according to at least one example embodiment.
FIG. 16A is a perspective view of an oral product having a circular cross section according to at least one example embodiment.
FIG. 16B is a perspective view of an oral product having an oval-shaped cross section according to at least one example embodiment.
FIG. 16C is a perspective view of an oral product having a rectangular cross section according to at least one example embodiment.
FIG. 16D is a perspective view of an oral product having an elongated rectangular cross section according to at least one example embodiment.
FIG. 16E is a perspective view of an oral product having a lens or football shaped cross section according to at least one example embodiment.
FIG. 16F is a perspective view of an oral product having a boomerangshaped cross section according to at least one example embodiment.
FIG. 16G is a perspective view of an oral product having a shield-shaped cross section according to at least one example embodiment.
FIG. 17 is a perspective view of an oral product according to at least one example embodiment.
FIG. 18 is a perspective view of a chewing gum according to at least one example embodiment.
FIG. 19A is a perspective view of a chewing gum having an oval-shaped cross section according to at least one example embodiment.
FIG. 19B is a perspective view of a chewing gum having a rectangular cross section according to at least one example embodiment.
FIG. 19C is a perspective view of a chewing gum having an elongated rectangular cross section according to at least one example embodiment.
FIG. 19D is a perspective view of a chewing gum having a lens or football shaped cross section according to at least one example embodiment.
FIG. 19E is a perspective view of a chewing gum having a boomerangshaped cross section according to at least one example embodiment.
FIG. 19F is a perspective view of a chewing gum having a shield-shaped cross section according to at least one example embodiment.
FIG. 20 is a perspective view of a chewing gum according to at least one example embodiment.
FIG. 21 is a cross-sectional view of a chewing gum with a coating according to at least one example embodiment.
FIG. 22 is a perspective view of an oral pouch product according to at least one example embodiment.
FIG. 23 is a cross-sectional view of the oral pouch product along line II-II of FIG. 22 according to at least one example embodiment.
FIG. 24 is a cross-sectional view of the oral pouch product along line III-III of FIG. 22 according to at least one example embodiment.
FIG. 25 is a side view of an oral pouch product according to at least one example embodiment.
FIG. 26 is a cross-sectional view along line VII-VII of the oral pouch product of FIG. 25 according at least one example embodiment.
FIG. 27 is a cross-sectional view of an oral pouch product according to at least one example embodiment.

### DETAILED DESCRIPTION OF EXAMPLE EMBODIMENTS

Some detailed example embodiments are disclosed herein. However, specific structural and functional details disclosed herein are merely representative for purposes of describing example embodiments. Example embodiments may, however, be embodied in many alternate forms and should not be construed as limited to only the example embodiments set forth herein.

Accordingly, while example embodiments are capable of various modifications and alternative forms, example embodiments thereof are shown by way of example in the drawings and will herein be described in detail. It should be understood, however, that there is no intent to limit example embodiments to the particular forms disclosed, but to the contrary, example embodiments are to cover all modifications, equivalents, and alternatives falling within the scope of example embodiments. Like numbers refer to like elements throughout the description of the figures.

It should be understood that when an element or layer is referred to as being "on," "connected to," "coupled to," or "covering" another element or layer, it may be directly on, connected to, coupled to, or covering the other element or layer or intervening elements or layers may be present. In contrast, when an element is referred to as being "directly on," "directly connected to," or "directly coupled to" another element or layer, there are no intervening elements or layers present. Like numbers refer to like elements throughout the specification. As used herein, the term "and/or" includes any and all combinations of one or more of the associated listed items.

It should be understood that, although the terms first, second, third, etc., may be used herein to describe various elements, regions, layers and/or sections, these elements, regions, layers, and/or sections should not be limited by these terms. These terms are only used to distinguish one element, region, layer, or section from another region, layer, or section. Thus, a first element, component, region, layer, or section discussed below could be termed a second element, region, layer, or section without departing from the teachings of example embodiments.

Spatially relative terms (e.g., "beneath," "below," "lower," "above," "upper," "inside," "outside," and the like) may be used herein for ease of description to describe one element or feature's relationship to another element(s) or feature(s) as illustrated in the figures. It should be understood that the spatially relative terms are intended to encompass different orientations of the device in use or operation in addition to the orientation depicted in the figures. For example, if the device in the figures is turned over, elements described as "below" or "beneath" other elements or features would then be oriented "above" the other elements or features. Thus, the term "below" may encompass both an orientation of above and below. The device may be otherwise oriented (rotated 90 degrees or at other orientations) and the spatially relative descriptors used herein interpreted accordingly.

The terminology used herein is for the purpose of describing various example embodiments only and is not intended to be limiting of example embodiments. As used herein, the singular forms "a," "an," and "the" are intended to include the plural forms as well, unless the context clearly indicates otherwise. It will be further understood that the terms "includes," "including," "comprises," and/or "comprising," specify the presence of stated features, integers, steps, operations, and/or elements, but do not preclude the presence or addition of one or more other features, integers, steps, operations, elements, and/or groups thereof.

Example embodiments are described herein with reference to cross-sectional illustrations that are schematic illustrations of example embodiments. As such, variations from the shapes of the illustrations are to be expected. Thus, example embodiments should not be construed as limited to the shapes of regions illustrated herein but are to include deviations and variations in shapes. When the terms "about" or "substantially" are used in connection with a numerical value, it is intended that the associated numerical value include a tolerance of ±10% around the stated numerical value unless the context indicates otherwise.

Unless otherwise defined, all terms (including technical and scientific terms) used herein have the same meaning as commonly understood by one of ordinary skill in the art to which example embodiments belong. It will be further understood that terms, including those defined in commonly used dictionaries, should be interpreted as having a meaning that is consistent with their meaning in the context of the relevant art and will not be interpreted in an idealized or overly formal sense unless expressly so defined herein.

In at least one example embodiment, the present disclosure provides methods of enhancing flavor and/or sensory effects of nicotine in oral products. In at least one example embodiment, a method includes spray drying nicotine. In at least one example embodiment, a method includes encapsulating nicotine. In at least one example embodiment, a method includes encapsulating a sweetener, such as can be included in an oral product including a nicotine-containing material.

In at least one example embodiment, the oral product is an oral tobacco product, an oral non-tobacco product, an oral cannabis product, or any combination thereof. The oral product may be in a form of loose material (e.g., loose cellulosic material), shaped material (e.g., plugs or twists), pouched material, tablets, lozenges, chews, gums, films, any other oral product, or any combination thereof.

The oral product may include chewing tobacco, snus, moist snuff tobacco, dry snuff tobacco, other smokeless tobacco and non-tobacco products for oral consumption, or any combination thereof.

Where the oral product is an oral tobacco product including smokeless tobacco product, the smokeless tobacco product may include tobacco that is whole, shredded, cut, granulated, reconstituted, cured, aged, fermented, pasteurized, or otherwise processed. Tobacco may be present as whole or portions of leaves, flowers, roots, stems, extracts (e.g., nicotine), or any combination thereof.

In at least one example embodiment, the oral product includes a tobacco extract, such as a tobacco-derived nicotine extract, and/or synthetic nicotine. The oral product may include nicotine alone or in combination with a carrier (e.g., white snus), such as a cellulosic material. The carrier may be a non-tobacco material (e.g., microcrystalline cellulose) or a tobacco material (e.g., tobacco fibers having reduced or eliminated nicotine content, which may be referred to as "exhausted tobacco plant tissue or fibers"). In some example embodiments, the exhausted tobacco plant tissue or fibers can be treated to remove at least 25%, 40%, 50%, 60%, 70%, 75%, 80%, 85%, 90%, or 95% of the nicotine. For example, the tobacco plant tissue can be washed with water or another solvent to remove the nicotine.

In other example embodiments, the oral product may include cannabis, such as cannabis plant tissue and/or cannabis extracts. In at least one example embodiment, the cannabis material includes leaf and/or flower material from one or more species of cannabis plants and/or extracts from the one or more species of cannabis plants. The one or more species of cannabis plants may include *Cannabis sativa*, *Cannabis indica*, and/or *Cannabis ruderalis*. In at least one example embodiment, the cannabis may be in the form of fibers. In at least one example embodiment, the cannabis may include a cannabinoid, a terpene, and/or a flavonoid. In at least one example embodiment, the cannabis material may be a cannabis-derived cannabis material, such as a cannabis-derived cannabinoid, a cannabis-derived terpene, and/or a cannabis-derived flavonoid.

The oral product (e.g., the oral tobacco product, the oral non-tobacco product, or the oral cannabis product) may have various ranges of moisture. In at least one example embodiment, the oral product is a dry oral product having a moisture content ranging from 5% by weight to 10% by weight. In at least one example embodiment, the oral product has a medium moisture content, such as a moisture content ranging from 20% by weight to 35% by weight. In at least one example embodiment, the oral product is a wet oral product having a moisture content ranging from 40% by weight to 55% by weight.

In at least one example embodiment, oral product may further include one or more elements such as a mouth-stable polymer, a mouth-soluble polymer, a sweetener (e.g., a synthetic sweetener and/or a natural sweetener), an energizing agent, a soothing agent, a focusing agent, a plasticizer, mouth-soluble fibers, an alkaloid, a mineral, a vitamin, a dietary supplement, a nutraceutical, a coloring agent, an amino acid, a chemesthetic agent, an antioxidant, a foodgrade emulsifier, a pH modifier, a botanical, a tooth-whitening agent, a therapeutic agent, a processing aid, a stearate, a wax, a stabilizer, a disintegrating agent, a lubricant, a preservative, a filler, a flavorant, flavor masking agents, a bitterness receptor site blocker, a receptor site enhancers, other additives, or any combination thereof.

### SPRAY-DRIED NICOTINE POWDER

In at least one example embodiment, nicotine-containing powders suitable for inclusion in oral products can be prepared using spray-drying techniques. Such nicotine-containing powders can include a plurality of substantially uniform nicotine particles.

In at least some example embodiments, the substantially uniform nicotine particles have an average particle size (90% distribution) ranging from about 5 *µ*m to about 200 *µ*m. For example, the plurality of substantially uniform nicotine particles may have an average particle size greater than or equal to about 5 *µ*m (e.g., great than or equal to about 10 *µ*m, greater than or equal to about 20 *µ*m, greater than or equal to about 30 *µ*m, greater than or equal to about 40 *µ*m, greater than or equal to about 50 *µ*m, greater than or equal to about 60 *µ*m, greater than or equal to about 70 *µ*m, greater than or equal to about 80 *µ*m, greater than or equal to about 90 *µ*m, greater than or equal to about 100 *µ*m, greater than or equal to about 110 *µ*m, greater than or equal to about 120 *µ*m, greater than or equal to about 130 *µ*m, greater than or equal to about 140 *µ*m, greater than or equal to about 150 *µ*m, greater than or equal to about 160 *µ*m, greater than or equal to about 170 *µ*m, greater than or equal to about 180 *µ*m, or greater than or equal to about 190 *µ*m). The plurality of substantially uniform nicotine particles may have an average particle size less than or equal to about 200 *µ*m (e.g., less than or equal to about 190 *µ*m, less than or equal to about 180 *µ*m, less than or equal to about 170 *µ*m, less than or equal to about 160 *µ*m, less than or equal to about 150 *µ*m, less than or equal to about 140 *µ*m, less than or equal to about 130 *µ*m, less than or equal to about 120 *µ*m, less than or equal to about 110 *µ*m, less than or equal to about 100 *µ*m, less than or equal to about 90 *µ*m, less than or equal to about 80 *µ*m, less than or equal to about 70 *µ*m, less than or equal to about 60 *µ*m, less than or equal to about 50 *µ*m, less than or equal to about 40 *µ*m, less than or equal to about 30 *µ*m, less than or equal to about 20 *µ*m, or less than or equal to about 10 *µ*m).

In at least some example embodiments, the substantially uniform nicotine particles have a moisture content less than or equal to 10 %. For example, the substantially uniform nicotine particles have a moisture content less than or equal to 10 %, less than or equal to 9 %, less than or equal to 8 %, less than or equal to 7 %, less than or equal to 5 %, less than or equal to 4 %, less than or equal to 3 %, less than or equal to 2 %, or less than or equal to 1 %.

In at least some example embodiments, the substantially uniform nicotine particles have a nicotine content less than or equal to 30 wt.%. For example, the substantially uniform nicotine particles may have a nicotine content ranging from about 10 wt.% to about 30 wt.%. The substantially uniform nicotine particles may have a nicotine content greater than or equal to about 10 wt.% (e.g., greater than or equal to about 11 wt.%, greater than or equal to about 12 wt.%, greater than or equal to about 13 wt.%, greater than or equal to about 14 wt.%, greater than or equal to about 15 wt.%, greater than or equal to about 16 wt.%, greater than or equal to about 17 wt.%, greater than or equal to about 18 wt.%, greater than or equal to about 19 wt.%, greater than or equal to about 20 wt.%, greater than or equal to about 21 wt.%, greater than or equal to about 22 wt.%, greater than or equal to about 23 wt.%, greater than or equal to about 24 wt.%, greater than or equal to about 25 wt.%, greater than or equal to about 26 wt.%, greater than or equal to about 27 wt.%, greater than or equal to about 28 wt.%, or greater than or equal to about 29 wt.%). The substantially uniform nicotine particles may have a nicotine content less than or equal to about 30 wt.% (e.g., less than or equal to about 29 wt.%, less than or equal to about 28 wt.%, less than or equal to about 27 wt.%, less than or equal to about 26 wt.%, less than or equal to about 25 wt.%, less than or equal to about 24 wt.%, less than or equal to about 23 wt.%, less than or equal to about 22 wt.%, less than or equal to about 21 wt.%, less than or equal to about 20 wt.%, less than or equal to about 19 wt.%, less than or equal to about 18 wt.%, less than or equal to about 17 wt.%, less than or equal to about 16 wt.%, less than or equal to about 15 wt.%, less than or equal to about 14 wt.%, less than or equal to about 13 wt.%, less than or equal to about 12 wt.%, or less than or equal to about 11 wt.%).

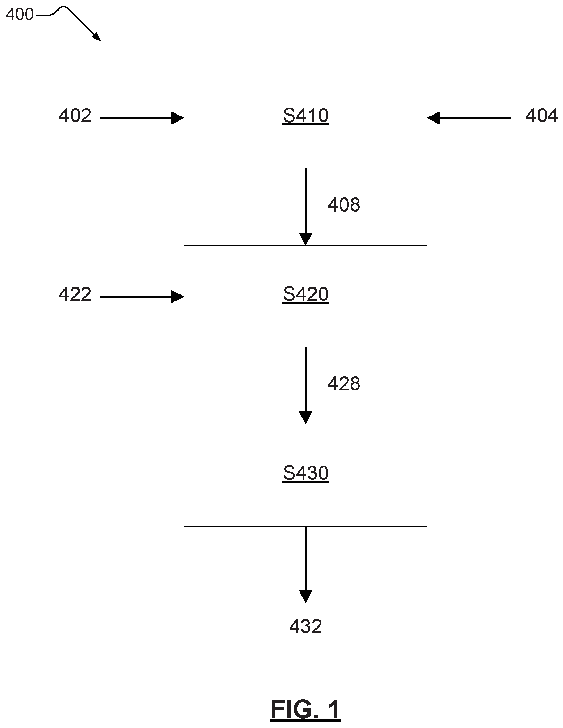

FIG. 1 is a flow chart illustrating a method 400 for forming a nicotine-containing powder 432 for inclusion in oral products such as gums, sprays, lozenges, dissolvable tablets, non-dissolvable chews, films, gels, capsules, and pouches (e.g., containing fibers or granules). The method 400 includes providing S410 a first solution 408 (i.e., carrier solution). The first solution 408 may have a viscosity suitable for subsequent processing.

The first solution 408 may include a carrier 402 and a solvent 404. In at least some example embodiments, providing S410 includes contacting the carrier 402 and the solvent 404 to form the first solution 408 (i.e., carrier solution). In at least one example embodiment, the contacting includes dissolving the carrier 402 in the solvent 404 to form the first solution 408 (i.e., carrier solution). In each instance, the solvent 404 may include water, ethanol, or both water and ethanol. The carrier 402 may include a biopolymer, a natural polymer, a synthetic polymer, a bulk sweetener, a pyrrolidone polymer, a methacrylate copolymer, or any combination thereof.

The biopolymer may include a polysaccharide, a bulk sweetener, or both a polysaccharide and a bulk sweetener, by way of example. In some example embodiments, the polysaccharide includes, for example, starches, methyl cellulose, hydroxyl propyl cellulose (HPC), hydroxyl methyl propyl cellulose (HPMC), high-methylated pectin, low-methylated pectin, amidated pectin, carboxyl methyl cellulose (CMC), dextrin, maltodextrin, isomalt, xanthan gum, agar, carrageenan, guar gum, alginate, isomalt, or any combination thereof. In some example embodiments, the bulk sweetener includes, for example, sucrose, dextrose, fructose, lactose, raffinose, trehalose, maltose, maltodextrins, isomalt, sugar alcohol, or any combination thereof. In some example embodiments, the biopolymer includes, for example, starches, methyl cellulose, hydroxyl propyl cellulose (HPC), hydroxyl methyl propyl cellulose (HPMC), high-methylated pectin, low-methylated pectin, amidated pectin, carboxyl methyl cellulose (CMC), dextrin, xanthan gum, agar, carrageenan, guar gum, alginate, sucrose, dextrose, fructose, lactose, raffinose, trehalose, maltose, maltodextrins, isomalt, or any combination thereof.

In at least some example embodiments, the natural and synthetic polymers include, for example, pectin, starches, gum arabic, or any combination thereof. In some example embodiments, the bulk sweetener include, for example, a sugar alcohol, which may include a sorbitol, mannitol, xylitol, maltitol, lactitol, isomalt, hydrogenated isomaltulose, hydrogenated starch hydrolyzates, or any combination thereof. In some example embodiments, the pyrrolidone polymer include, for example, polyvinylpyrrolidone (PVP), copolymers of polyvinylpyrrolidone (PVP) and vinyl acetate, or any combination thereof. In some example embodiments, the methacrylate copolymer include, for example, copolymers of methacrylate and acrylic acid.

In at least some example embodiments, the first solution 408 an amount of the carrier 402 ranging from about 3 wt.% to about 45 wt.%. For example, the first solution 408 may include greater than or equal to about 3 wt.% of the carrier 402 (e.g., greater than or equal to about 4 wt.%, greater than or equal to about 5 wt.%, greater than or equal to about 10 wt.%, greater than or equal to about 15 wt.%, greater than or equal to about 20 wt.%, greater than or equal to about 25 wt.%, greater than or equal to about 30 wt.%, greater than or equal to about 35 wt.%, greater than or equal to about 40 wt.%, greater than or equal to about 41 wt.%, greater than or equal to about 42 wt.%, greater than or equal to about 43 wt.%, or greater than or equal to about 44 wt.%). The first solution 408 may include less than or equal to about 45 wt.% of the carrier 402 (e.g., less than or equal to about 44 wt.%, less than or equal to about 43 wt.%, less than or equal to about 42 wt.%, less than or equal to about 41 wt.%, less than or equal to about 40 wt.%, less than or equal to about 35 wt.%, less than or equal to about 30 wt.%, less than or equal to about 25 wt.%, less than or equal to about 20 wt.%, less than or equal to about 15 wt.%, less than or equal to about 10 wt.%, less than or equal to about 5 wt.%, or less than or equal to about 4 wt.%). An amount of the carrier 402 and an amount of the solvent 404 within the first solution 408 may be adjusted so that the first solution 408 has a viscosity suitable for subsequent processing. For example, in at least some example embodiments, such as where the carrier 402 includes pectin, the first solution 508 may include about 5 wt.% of the carrier 402. In other example embodiments, such as where the carrier 403 includes maltodextrin, the first solution 508 may include about 30 wt.% of the carrier 402.

In at least some example embodiments, the first solution 408 includes an amount of the solvent 404 ranging from about 55 wt.% to about 97 wt.%. For example, the first solution 408 may include greater than or equal to about 55 wt.% of the solvent 404 (e.g., greater than or equal to about 56 wt.%, greater than or equal to about 57 wt.%, greater than or equal to about 58 wt.%, greater than or equal to about 59 wt.%, greater than or equal to about 60 wt.%, greater than or equal to about 65 wt.%, greater than or equal to about 70 wt.%, greater than or equal to about 75 wt.%, greater than or equal to about 80 wt.%, greater than or equal to about 85 wt.%, greater than or equal to about 90 wt.%, greater than or equal to about 95 wt.%, or greater than or equal to about 96 wt.%). The first solution 408 may include less than or equal to about 97 wt.% of the solvent 404 (e.g., less than or equal to about 96 wt.%, less than or equal to about 95 wt.%, less than or equal to about 90 wt.%, less than or equal to about 85 wt.%, less than or equal to about 80 wt.%, less than or equal to about 75 wt.%, less than or equal to about 70 wt.%, less than or equal to about 65 wt.%, less than or equal to about 60 wt.%, less than or equal to about 59 wt.%, less than or equal to about 58 wt.%, less than or equal to about 57 wt.%, or less than or equal to about 56 wt.%).

In at least one example embodiment, the method 400 includes contacting S420 the first solution 408 with a nicotine-containing formulation 422 to form a second mixture 428 (*i.e.*, feed solution). In some example embodiments, the nicotine-containing formulation 422 includes nicotine, a nicotine complex (such as, nicotine polacrilex), a nicotine salt, or any combination thereof. The nicotine salt may include nitrate, monotartrate, bitartrate, bitartrate dihydrate, salicylate, sulfate or bisulfate, phosphate or acid phosphate, acetate, lactate, succinate, maleate, fumarate, gluconate, saccharate, benzoate, methanesulfonate, hydrochloride, hydrobromide, hydroiodide, or any combination thereof.

In at least some example embodiments, the second mixture 428 includes an amount of the first solution 408 ranging from about 85 wt.% to about 99 wt.% of the first solution 408. For example, the second mixture 428 may include greater than or equal to about 85 wt.% of the first solution 408 (e.g., greater than or equal to about 86 wt.%, greater than or equal to about 87 wt.%, greater than or equal to about 88 wt.%, greater than or equal to about 89 wt.%, greater than or equal to about 90 wt.%, greater than or equal to about 91 wt.%, greater than or equal to about 92 wt.%, greater than or equal to about 93 wt.%, greater than or equal to about 94 wt.%, greater than or equal to about 95 wt.%, greater than or equal to about 96 wt.%, greater than or equal to about 97 wt.%, or greater than or equal to about 98 wt.%). The second mixture 428 may include less than or equal to about 99 wt.% of the first solution 408 (e.g., less than or equal to about 98 wt.%, less than or equal to about 97 wt.%, less than or equal to about 96 wt.%, less than or equal to about 95 wt.%, less than or equal to about 94 wt.%, less than or equal to about 93 wt.%, less than or equal to about 92 wt.%, less than or equal to about 91 wt.%, less than or equal to about 90 wt.%, less than or equal to about 89 wt.%, less than or equal to about 88 wt.%, less than or equal to about 87 wt.%, or less than or equal to about 86 wt.%).

In at least some example embodiments, such as when the nicotine-containing formulation 422 includes pure nicotine, the second mixture 428 includes an amount of the nicotine-containing formulation 422 ranging from about 1 wt.% to about 10 wt.%. For example, the second mixture 428 may include greater than or equal to about 1 wt.% of the nicotine-containing formulation 422 (e.g., greater than or equal to about 2 wt.%, greater than or equal to about 3 wt.%, greater than or equal to about 4 wt.%, greater than or equal to about 5 wt.%, greater than or equal to about 6 wt.%, greater than or equal to about 7 wt.%, greater than or equal to about 8 wt.%, or greater than or equal to about 9 wt.%). The second mixture 428 may include less than or equal to about 10 wt.% of the nicotine-containing formulation 422 (e.g., less than or equal to about 9 wt.%, less than or equal to about 8 wt.%, less than or equal to about 7 wt.%, less than or equal to about 6 wt.%, less than or equal to about 5 wt.%, less than or equal to about 4 wt.%, less than or equal to about 3 wt.%, or less than or equal to about 2 wt.%).

The second mixture 428 may have a viscosity such that the second mixture 428 can be readily injected or pumped during subsequent spray drying processes. In at least one example embodiment, the second mixture 428 may have a viscosity at about 22 °C ranging from about 1 centipoise to about 700 centipoise. For example, the second mixture 428 may have a viscosity at about 22 °C greater than or equal to about 1 centipoise (e.g., greater than or equal to about 10 centipoise, greater than or equal to about 20 centipoise, greater than or equal to about 30 centipoise, greater than or equal to about 40 centipoise, greater than or equal to about 50 centipoise, greater than or equal to about 100 centipoise, greater than or equal to about 150 centipoise, greater than or equal to about 200 centipoise, greater than or equal to about 250 centipoise, greater than or equal to about 300 centipoise, greater than or equal to about 350 centipoise, greater than or equal to about 400 centipoise, greater than or equal to about 450 centipoise, greater than or equal to about 500 centipoise, greater than or equal to about 550 centipoise, greater than or equal to about 600 centipoise, or greater than or equal to about 650 centipoise). The second mixture 428 may have a viscosity at about 22 °C less than or equal to about 700 centipoise (e.g., less than or equal to about 690 centipoise, less than or equal to about 680 centipoise, less than or equal to about 670 centipoise, less than or equal to about 660 centipoise, less than or equal to about 650 centipoise, less than or equal to about 600 centipoise, less than or equal to about 550 centipoise, less than or equal to about 500 centipoise, less than or equal to about 450 centipoise, less than or equal to about 400 centipoise, less than or equal to about 350 centipoise, less than or equal to about 300 centipoise, less than or equal to about 250 centipoise, less than or equal to about 200 centipoise, less than or equal to about 150 centipoise, less than or equal to about 100 centipoise, less than or equal to about 50 centipoise, less than or equal to about 40 centipoise, less than or equal to about 30 centipoise, less than or equal to about 20 centipoise, less than or equal to about 10 centipoise). Such viscosities are believed to be desirable to provide suitable rheological properties that allow the slurry to flow under applied pressure, but also permit the mixture 428 to remain stable.

In at least one example embodiment, the method 400 includes spray drying S430 the second mixture 428 to form a plurality of particles that define a nicotine-containing powder 432 (*i.e.*, a dry powder). The plurality of particles defining the nicotine-containing powder 432 may have an average particle size (90% distribution) ranging from about 5 *µ*m to about 200 *µ*m. For example, the plurality of particles defining the nicotine-containing powder 432 may have an average particle size greater than or equal to about 5 *µ*m (e.g., great than or equal to about 10 *µ*m, greater than or equal to about 20 *µ*m, greater than or equal to about 30 *µ*m, greater than or equal to about 40 *µ*m, greater than or equal to about 50 *µ*m, greater than or equal to about 60 *µ*m, greater than or equal to about 70 *µ*m, greater than or equal to about 80 *µ*m, greater than or equal to about 90 *µ*m, greater than or equal to about 100 *µ*m, greater than or equal to about 110 *µ*m, greater than or equal to about 120 *µ*m, greater than or equal to about 130 *µ*m, greater than or equal to about 140 *µ*m, greater than or equal to about 150 *µ*m, greater than or equal to about 160 *µ*m, greater than or equal to about 170 *µ*m, greater than or equal to about 180 *µ*m, or greater than or equal to about 190 *µ*m). The plurality of particles defining the nicotine-containing powder 432 may have an average particle size less than or equal to about 200 *µ*m (e.g., less than or equal to about 190 *µ*m, less than or equal to about 180 *µ*m, less than or equal to about 170 *µ*m, less than or equal to about 160 *µ*m, less than or equal to about 150 *µ*m, less than or equal to about 140 *µ*m, less than or equal to about 130 *µ*m, less than or equal to about 120 *µ*m, less than or equal to about 110 *µ*m, less than or equal to about 100 *µ*m, less than or equal to about 90 *µ*m, less than or equal to about 80 *µ*m, less than or equal to about 70 *µ*m, less than or equal to about 60 *µ*m, less than or equal to about 50 *µ*m, less than or equal to about 40 *µ*m, less than or equal to about 30 *µ*m, less than or equal to about 20 *µ*m, or less than or equal to about 10 *µ*m).

The plurality of particles defining the nicotine-containing powder 432 may have a moisture content less than or equal to about 10 %. For example, the nicotine-containing powder 432 may have a moisture content less than or equal to about 10 %, less than or equal to about 9 %, less than or equal to about 8 %, less than or equal to about 7 %, less than or equal to about 5 %, less than or equal to about 4 %, less than or equal to about 3 %, less than or equal to about 2 %, or less than or equal to about 1 %.

In at least some example embodiments, the nicotine-containing powder 432 has a nicotine content less than or equal to about 30 wt.%. For example, the nicotine-containing powder 432 may have a nicotine content ranging from about 10 wt.% to about 30 wt.%. The nicotine-containing powder 432 may have a nicotine content greater than or equal to about 10 wt.% (e.g., greater than or equal to about 11 wt.%, greater than or equal to about 12 wt.%, greater than or equal to about 13 wt.%, greater than or equal to about 14 wt.%, greater than or equal to about 15 wt.%, greater than or equal to about 16 wt.%, greater than or equal to about 17 wt.%, greater than or equal to about 18 wt.%, greater than or equal to about 19 wt.%, greater than or equal to about 20 wt.%, greater than or equal to about 21 wt.%, greater than or equal to about 22 wt.%, greater than or equal to about 23 wt.%, greater than or equal to about 24 wt.%, greater than or equal to about 25 wt.%, greater than or equal to about 26 wt.%, greater than or equal to about 27 wt.%, greater than or equal to about 28 wt.%, or greater than or equal to about 29 wt.%). The nicotine-containing powder 432 may have a nicotine content less than or equal to about 30 wt.% (e.g., less than or equal to about 29 wt.%, less than or equal to about 28 wt.%, less than or equal to about 27 wt.%, less than or equal to about 26 wt.%, less than or equal to about 25 wt.%, less than or equal to about 24 wt.%, less than or equal to about 23 wt.%, less than or equal to about 22 wt.%, less than or equal to about 21 wt.%, less than or equal to about 20 wt.%, less than or equal to about 19 wt.%, less than or equal to about 18 wt.%, less than or equal to about 17 wt.%, less than or equal to about 16 wt.%, less than or equal to about 15 wt.%, less than or equal to about 14 wt.%, less than or equal to about 13 wt.%, less than or equal to about 12 wt.%, or less than or equal to about 11 wt.%).

Various spray drying techniques and equipment known to those of skill in the art may be employed. Spray drying parameters may be used to tailor the dried end-product (e.g., nicotine-containing powder 432) to precise quality standards and physical characteristics). These standards and characteristics include particle size distribution, residual moisture, bulk density, and particle morphology.

In some example embodiments, a nozzle air pressure for the spray drying process S430 ranges from about 30 psi to about 40 psi. For example, the nozzle air pressure may be greater than or equal to about 30 psi (e.g., greater than or equal to about 31 psi, greater than or equal to about 32 psi, greater than or equal to about 33 psi, greater than or equal to about 34 psi, greater than or equal to about 35 psi, greater than or equal to about 36 psi, greater than or equal to about 37 psi, greater than or equal to about 38 psi, or greater than or equal to about 39 psi). The nozzle air pressure may be less than or equal to about 40 psi (e.g., less than or equal to about 39 psi, less than or equal to about 38 psi, less than or equal to about 37 psi, less than or equal to about 36 psi, less than or equal to about 35 psi, less than or equal to about 34 psi, less than or equal to about 33 psi, less than or equal to about 32 psi, or less than or equal to about 31 psi).

In some example embodiments, a solution pump revolutions per minute for the spray drying process S430 ranges from about 15 rpm to about 35 rpm. For example, the solution pump revolutions may be greater than or equal to about 15 rpm (e.g., greater than or equal to about 16 rpm, greater than or equal to about 17 rpm, greater than or equal to about 18 rpm, greater than or equal to about 19 rpm, greater than or equal to about 20 rpm, greater than or equal to about 21 rpm, greater than or equal to about 22 rpm, greater than or equal to about 23 rpm, greater than or equal to about 24 rpm, greater than or equal to about 25 rpm, greater than or equal to about 26 rpm, greater than or equal to about 27 rpm, greater than or equal to about 28 rpm, greater than or equal to about 29 rpm, greater than or equal to about 30 rpm, greater than or equal to about 31 rpm, greater than or equal to about 32 rpm, greater than or equal to about 33 rpm, or greater than or equal to about 34 rpm). The solution pump revolutions may be less than or equal to about 35 rpm (e.g., less than or equal to about 35 rpm, less than or equal to about 34 rpm, less than or equal to about 33 rpm, less than or equal to about 32 rpm, less than or equal to about 31 rpm, less than or equal to about 30 rpm, less than or equal to about 29 rpm, less than or equal to about 28 rpm, less than or equal to about 27 rpm, less than or equal to about 26 rpm, less than or equal to about 25 rpm, less than or equal to about 24 rpm, less than or equal to about 23 rpm, less than or equal to about 22 rpm, less than or equal to about 21 rpm, less than or equal to about 20 rpm, less than or equal to about 19 rpm, less than or equal to about 18 rpm, less than or equal to about 17 rpm, or less than or equal to about 16 rpm).

In some example embodiments, a solution spray rate for the spray drying process S430 ranges from about 9 g/min to about 15 g/min. For example, the solution spray may be greater than or equal to about 9 g/min (e.g., greater than or equal to about 10 g/min, greater than or equal to about 11 g/min, greater than or equal to about 12 g/min, greater than or equal to about 13 g/min, or greater than or equal to about 14 g/min). The solution spray may be less than or equal to about 15 g/min (e.g., less than or equal to about 14 g/min, less than or equal to about 13 g/min, less than or equal to about 12 g/min, less than or equal to about 11 g/min, or less than or equal to about 10 g/min).

In some example embodiments, a spray time for the spray drying process S430 ranges from about 40 minutes to about 200 minutes. For example, the spray time may be greater than or equal to about 40 minutes (e.g., greater than or equal to about 45 minutes, greater than or equal to about 50 minutes, greater than or equal to about 55 minutes, greater than or equal to about 60 minutes, greater than or equal to about 65 minutes, greater than or equal to about 70 minutes, greater than or equal to about 75 minutes, greater than or equal to about 80 minutes, greater than or equal to about 85 minutes, greater than or equal to about 90 minutes, greater than or equal to about 95 minutes, greater than or equal to about 100 minutes, greater than or equal to about 105 minutes, greater than or equal to about 110 minutes, greater than or equal to about 115 minutes, greater than or equal to about 120 minutes, greater than or equal to about 125 minutes, greater than or equal to about 130 minutes, greater than or equal to about 135 minutes, greater than or equal to about 140 minutes, greater than or equal to about 145 minutes, greater than or equal to about 150 minutes, greater than or equal to about 155 minutes, greater than or equal to about 160 minutes, greater than or equal to about 165 minutes, greater than or equal to about 170 minutes, greater than or equal to about 175 minutes, greater than or equal to about 180 minutes, greater than or equal to about 185 minutes greater than or equal to about 190 minutes, or greater than or equal to about 195 minutes). The spray time may be less than or equal to about 200 minutes (e.g., less than or equal to about 195 minutes, less than or equal to about 190 minutes, less than or equal to about 185 minutes, less than or equal to about 180 minutes, less than or equal to about 175 minutes, less than or equal to about 170 minutes, less than or equal to about 165 minutes, less than or equal to about 160 minutes, less than or equal to about 155 minutes, less than or equal to about 150 minutes, less than or equal to about 145 minutes, less than or equal to about 140 minutes, less than or equal to about 135 minutes, less than or equal to about 130 minutes, less than or equal to about 125 minutes, less than or equal to about 120 minutes, less than or equal to about 115 minutes, less than or equal to about 110 minutes, less than or equal to about 105 minutes, less than or equal to about 100 minutes, less than or equal to about 95 minutes, less than or equal to about 90 minutes, less than or equal to about 85 minutes, less than or equal to about 80 minutes, less than or equal to about 75 minutes, less than or equal to about 70 minutes, less than or equal to about 65 minutes, less than or equal to about 60 minutes, less than or equal to about 55 minutes, less than or equal to about 50 minutes, or less than or equal to about 45 minutes).

In some example embodiments, such as when the solvent 404 includes water, an inlet temperature for the spray drying process S430 ranges from about 120 °C to about 210 °C. For example, the inlet temperature may be greater than or equal to about 120 °C (e.g., greater than or equal to about 125 °C, greater than or equal to about 130 °C, greater than or equal to about 135 °C, greater than or equal to about 140 °C, greater than or equal to about 145 °C, greater than or equal to about 150 °C, greater than or equal to about 155 °C, greater than or equal to about 160 °C, greater than or equal to about 165 °C, greater than or equal to about 170 °C, greater than or equal to about 175 °C, greater than or equal to about 180 °C, greater than or equal to about 185 °C, greater than or equal to about 190 °C, greater than or equal to about 195 °C, greater than or equal to about 200 °C, or greater than or equal to about 205 °C). The inlet temperature may be less than or equal to about 210 °C (e.g*.*, less than or equal to about 205 °C, less than or equal to about 200 °C, less than or equal to about 195 °C, less than or equal to about 190 °C, less than or equal to about 185 °C, less than or equal to about 180 °C, less than or equal to about 175 °C, less than or equal to about 170 °C, less than or equal to about 165 °C, less than or equal to about 160 °C, less than or equal to about 155 °C, less than or equal to about 150 °C, less than or equal to about 145 °C, less than or equal to about 140 °C, less than or equal to about 135 °C, or less than or equal to about 130 °C).

In some example embodiments, such as when the solvent 404 includes ethanol, an inlet temperature for the spray drying process S430 ranges from about 65 °C to about 180 °C. For example, the inlet temperature may be greater than or equal to about 65 °C (e.g., greater than or equal to about 70 °C, greater than or equal to about 75 °C, greater than or equal to about 80 °C, greater than or equal to about 85 °C, greater than or equal to about 90 °C, greater than or equal to about 95 °C, greater than or equal to about 100 °C, greater than or equal to about 105 °C, greater than or equal to about 110 °C, greater than or equal to about 115 °C, greater than or equal to about 120 °C, greater than or equal to about 125 °C, greater than or equal to about 130 °C, greater than or equal to about 135 °C, greater than or equal to about 140 °C, greater than or equal to about 145 °C, greater than or equal to about 150 °C, greater than or equal to about 155 °C, greater than or equal to about 160 °C, greater than or equal to about 165 °C, greater than or equal to about 170 °C, or greater than or equal to about 175 °C). The inlet temperature may be less than or equal to about 180 °C (e.g., less than or equal to about 175 °C, less than or equal to about 170 °C, less than or equal to about 165 °C, less than or equal to about 160 °C, less than or equal to about 155 °C, less than or equal to about 150 °C, less than or equal to about 145 °C, less than or equal to about 140 °C, less than or equal to about 135 °C, less than or equal to about 130 °C, less than or equal to about 125 °C, less than or equal to about 120 °C, less than or equal to about 115 °C, less than or equal to about 110 °C, less than or equal to about 105 °C, less than or equal to about 100 °C, less than or equal to about 95 °C, less than or equal to about 90 °C, less than or equal to about 85 °C, less than or equal to about 80 °C, less than or equal to about 75 °C, or less than or equal to about 70 °C)

In some example embodiments, such as when the solvent 404 includes water, an initial product temperature for the spray drying process S430 ranges from about 25 °C to about 100 °C. For example, the initial product temperature may be greater than or equal to about 25 °C (e.g.*,* greater than or equal to about 30 °C, greater than or equal to about 35 °C, greater than or equal to about 40 °C, greater than or equal to about 45 °C, greater than or equal to about 50 °C, greater than or equal to about 65 °C, greater than or equal to about 75 °C, greater than or equal to about 80 °C, greater than or equal to about 85 °C, greater than or equal to about 90 °C, or greater than or equal to about 95 °C). The initial product temperature may be less than or equal to about 100 °C (e.g.*,* less than or equal to about 95 °C, less than or equal to about 90 °C, less than or equal to about 85 °C, less than or equal to about 80 °C, less than or equal to about 75 °C, less than or equal to about 70 °C, less than or equal to about 65 °C, less than or equal to about 60 °C, less than or equal to about 55 °C, less than or equal to about 50 °C, less than or equal to about 45 °C, less than or equal to about 40 °C, less than or equal to about 35 °C, or less than or equal to about 30 °C).

In some example embodiments, such as when the solvent 404 includes ethanol, an initial product temperature for the spray drying process S430 ranges from about 25 °C to about 79 °C. For example, the initial product temperature may be greater than or equal to about 25 °C (e.g., greater than or equal to about 30 °C, greater than or equal to about 35 °C, greater than or equal to about 40 °C, greater than or equal to about 45 °C, greater than or equal to about 50 °C, greater than or equal to about 55 °C, greater than or equal to about 60 °C, greater than or equal to about 65 °C, or greater than or equal to about 75 °C). The initial product temperature may be less than or equal to about 79 °C (e.g., less than or equal to about 78 °C, less than or equal to about 77 °C, less than or equal to about 76 °C, less than or equal to about 75 °C, less than or equal to about 70 °C, less than or equal to about 65 °C, less than or equal to about 60 °C, less than or equal to about 55 °C, less than or equal to about 50 °C, less than or equal to about 45 °C, less than or equal to about 40 °C, less than or equal to about 35 °C, or less than or equal to about 30 °C).

After spray drying S430, the nicotine-containing powder 432 may be optionally pressed into tablets, grains, pellets, cylinders, or other geometries to produce solid bodies having a controllable release and suitable for inclusion in oral products, such as gums, sprays, lozenges, dissolvable tablets, non-dissolvable chews, films, gels, capsules, and pouches (e.g., containing fibers or granules).

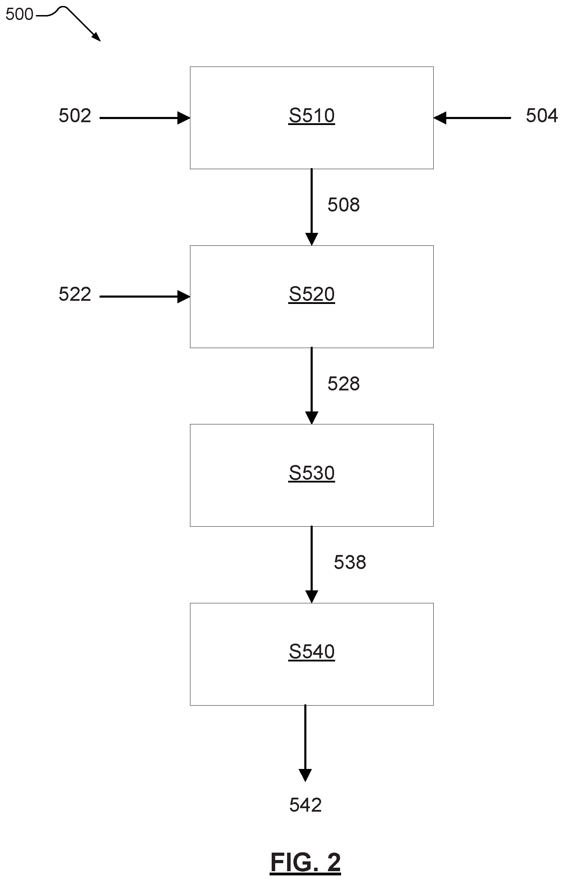

FIG. 2 is flow diagram illustrating a method 500 for forming a nicotine-containing powder 542 for inclusion in oral products such as gums, sprays, lozenges, dissolvable tablets, non-dissolvable chews, films, gels, capsules, and pouches (e.g., containing fibers or granules). The method 500 is like method 400, except that method 500 includes heating S530 a second mixture (i.e., feed solution) 528 to a first temperature prior to spray drying S540.

For example, the method 500 includes contacting S510 a carrier 502 and a solvent 504 to form a first solution 508 (i.e., carrier solution); contacting S520 the first solution 508 with a nicotine-containing formulation 522 to form a second mixture 528 (i.e., feed solution); heating S530 the second mixture 528; and spray drying S540 the heated second mixture 538 to form a plurality of particles that define the nicotine-containing powder 542 (i.e., a dry powder). After spray drying S540, although not illustrated, the method 500 may include pressing the nicotine-containing powder 542 into tablets, grains, pellets, cylinders, or other geometries to produce solid bodies having a controllable release and suitable for inclusion in oral products such as gums, sprays, lozenges, dissolvable tablets, non-dissolvable chews, films, gels, capsules, and pouches (e.g., containing fibers or granules).

In at least some example embodiment, the method 500 may include heating S530 the second mixture to a first temperature.

In at least one example embodiment, such as when the solvent 504 includes water, the first temperature may range from about 120 °C to about 210 °C. For example, the first temperature may be greater than or equal to about 120 °C (e.g., greater than or equal to about 125 °C, greater than or equal to about 130 °C, greater than or equal to about 135 °C, greater than or equal to about 140 °C, greater than or equal to about 145 °C, greater than or equal to about 150 °C, greater than or equal to about 155 °C, greater than or equal to about 160 °C, greater than or equal to about 165 °C, greater than or equal to about 170 °C, greater than or equal to about 175 °C, greater than or equal to about 180 °C, greater than or equal to about 185 °C, greater than or equal to about 190 °C, greater than or equal to about 195 °C, greater than or equal to about 200 °C, or greater than or equal to about 205 °C). The first temperature may be less than or equal to about 210 °C (e.g*.*, less than or equal to about 205 °C, less than or equal to about 200 °C, less than or equal to about 195 °C, less than or equal to about 190 °C, less than or equal to about 185 °C, less than or equal to about 180 °C, less than or equal to about 175 °C, less than or equal to about 170 °C, less than or equal to about 165 °C, less than or equal to about 160 °C, less than or equal to about 155 °C, less than or equal to about 150 °C, less than or equal to about 145 °C, less than or equal to about 140 °C, less than or equal to about 135 °C, or less than or equal to about 130 °C).

In some embodiments, such as when the solvent 504 includes ethanol, the first temperature may range from about 65 °C to about 180 °C. For example, the first temperature may be greater than or equal to about 65 °C (e.g., greater than or equal to about 70 °C, greater than or equal to about 75 °C, greater than or equal to about 80 °C, greater than or equal to about 85 °C, greater than or equal to about 90 °C, greater than or equal to about 95 °C, greater than or equal to about 100 °C, greater than or equal to about 105 °C, greater than or equal to about 110 °C, greater than or equal to about 115 °C, greater than or equal to about 120 °C, greater than or equal to about 125 °C, greater than or equal to about 130 °C, greater than or equal to about 135 °C, greater than or equal to about 140 °C, greater than or equal to about 145 °C, greater than or equal to about 150 °C, greater than or equal to about 155 °C, greater than or equal to about 160 °C, greater than or equal to about 165 °C, greater than or equal to about 170 °C, or greater than or equal to about 175 °C). The first temperature may be less than or equal to about 180 °C (e.g., less than or equal to about 175 °C, less than or equal to about 170 °C, less than or equal to about 165 °C, less than or equal to about 160 °C, less than or equal to about 155 °C, less than or equal to about 150 °C, less than or equal to about 145 °C, less than or equal to about 140 °C, less than or equal to about 135 °C, less than or equal to about 130 °C, less than or equal to about 125 °C, less than or equal to about 120 °C, less than or equal to about 115 °C, less than or equal to about 110 °C, less than or equal to about 105 °C, less than or equal to about 100 °C, less than or equal to about 95 °C, less than or equal to about 90 °C, less than or equal to about 85 °C, less than or equal to about 80 °C, less than or equal to about 75 °C, or less than or equal to about 70 °C).

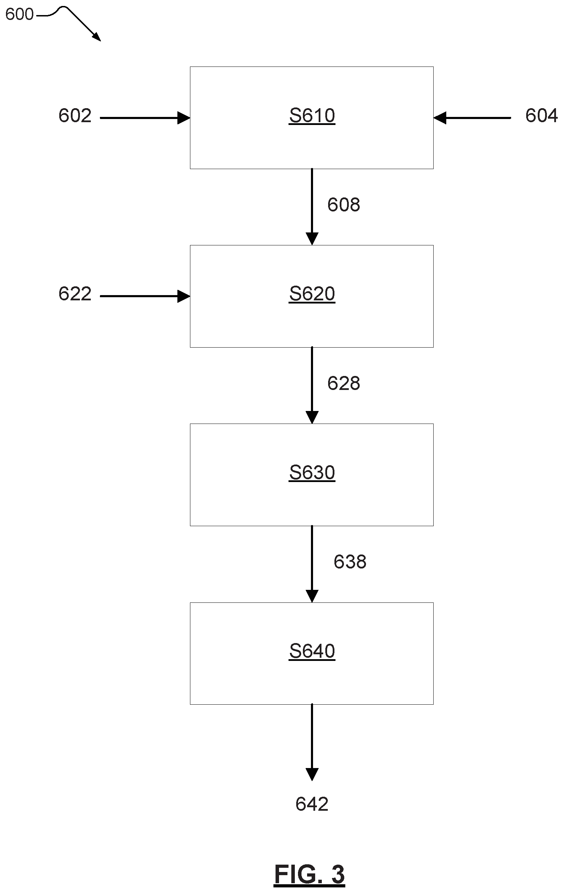

FIG. 3 is a flow diagram illustrating a method for forming a nicotine-containing powder 642 for inclusion in oral products such as gums, sprays, lozenges, dissolvable tablets, non-dissolvable chews, films, gels, capsules, and pouches (e.g., containing fibers or granules). The method 500 is like method 400 and/or method 500, except that method 600 includes homogenizing S630 the second mixture 628 to form a third mixture 638 (i.e., feed solution) that has substantially uniformed distribution. In some example embodiments, the second mixture 628 may be homogenized S630 using a paddle mixture, a high-pressure mixer, a high shear mixture, an ultrasonic homogenizer, or any combination thereof.

For example, the method 600 includes contacting S610 a carrier 602 and a solvent 604 to form a first solution 508 (i.e., carrier solution); contacting S620 the first solution 608 with a nicotine-containing formulation 622 to form a second mixture 628; homogenizing S630 the second mixture 628 to form a third mixture 638 (i.e., feed solution); and spray drying S640 the third mixture 638 to form a plurality of particles that define the nicotine-containing powder 642 (i.e., a dry powder). After spray drying S640, although not illustrated, the method 600 may include pressing the nicotine-containing powder 642 into tablets, grains, pellets, cylinders, or other geometries to produce solid bodies having a controllable release and suitable for inclusion in oral products such as gums, sprays, lozenges, dissolvable tablets, non-dissolvable chews, films, gels, capsules, and pouches (e.g., containing fibers or granules).

Though the homogenization S630 is illustrated as following the contacting S620, in at least one example embodiment, the contacting S620 and the homogenizing S630 may occur simultaneously. Similarly, the method 600 may include in certain embodiments, heating the second mixture 628 and/or third mixture 638, such as illustrated in method 500, and/or adding an additive, such as illustrated in method 700.

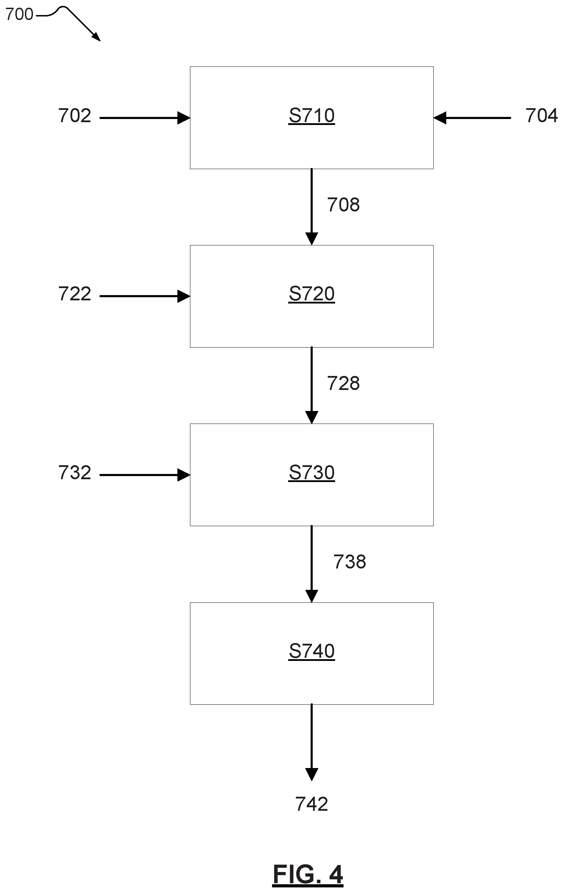

FIG. 4 is a flow diagram illustrating a method 700 for forming nicotine-containing powder 742 for inclusion in oral products such as gums, sprays, lozenges, dissolvable tablets, non-dissolvable chews, films, gels, capsules, and pouches (e.g., containing fibers or granules). The method 700 is like method 400 and/or method 500 and/or method 600, except that method 700 includes adding one or more additives 732 to the feed solution 738.

For example, the method 700 includes contacting S710 a carrier 702 and a solvent 704 to form a first solution 708 (i.e., carrier solution); contacting S720 the first solution 708 with a nicotine-containing formulation 722 to form a second mixture 728; adding S730 the one or more additives 732 to the second mixture 728 to form a third mixture 738 (i.e., feed solution); and spray drying S740 the third mixture 738 to form a plurality of particles that define the nicotine-containing powder 742 (i.e., a dry powder). After spray drying S740, although not illustrated, the method 700 may include pressing the nicotine-containing powder 742 into tablets, grains, pellets, cylinders, or other geometries to produce solid bodies having a controllable release and suitable for inclusion in oral products such as gums, sprays, lozenges, dissolvable tablets, non-dissolvable chews, films, gels, capsules, and pouches (e.g., containing fibers or granules).

In some example embodiments, the one or more additives include a pH modifier, an antioxidant, or a combination of the pH modifier and the antioxidant. The pH modifier may include sodium carbonate/bicarbonate, potassium carbonate/bicarbonate, citric acid, or any combination thereof. The antioxidant may include scorbyl palmitate, butylated hydroxytoluene (BHT), ascorbic acid, sodium ascorbate, monosterol citrate, tocopherols, propyl gallate, tertiary butylhydroquinone (TBHQ), butylated hydroxyanisole (BHA), Vitamin E, and any combination or derivative thereof. The presence of the antioxidant may help to limit the formation of nicotine-N-oxides.

In at least some example embodiments, the third mixture 738 may have amount of the additive 732 ranging from about 0.1 wt.% to about 10 wt.%. For example, the third mixture 738 may include greater than or equal to about 0.1 wt.% (e.g., greater than or equal to about 0.5 wt.%, greater than or equal to about 1 wt.%, greater than or equal to about 1.5 wt.%, greater than or equal to about 2 wt.%, greater than or equal to about 2.5 wt.%, greater than or equal to about 3 wt.%, greater than or equal to about 3.5 wt.%, greater than or equal to about 4 wt.%, greater than or equal to about 4.5 wt.%, greater than or equal to about 5 wt.%, greater than or equal to about 5.5 wt.%, greater than or equal to about 6 wt.%, greater than or equal to about 6.5 wt.%, greater than or equal to about 7 wt.%, greater than or equal to about 7.5 wt.%, greater than or equal to about 8 wt.%, greater than or equal to about 8.5 wt.%, greater than or equal to about 9 wt.%, or greater than or equal to about 9.5 wt.%. The third mixture 728 may include less than or equal to about 10 wt.% (e.g., less than or equal to about 9.5 wt.%, less than or equal to about 9 wt.%, less than or equal to about 8.5 wt.%, less than or equal to about 8 wt.%, less than or equal to about 7.5 wt.%, less than or equal to about 7 wt.%, less than or equal to about 6.5 wt.%, less than or equal to about 6 wt.%, less than or equal to about 5.5 wt.%, less than or equal to about 5 wt.%, less than or equal to about 4.5 wt.%, less than or equal to about 4 wt.%, less than or equal to about 3.5 wt.%, less than or equal to about 3 wt.%, less than or equal to 2.5 wt.%, less than or equal to about 2 wt.%, less than or equal to about 1 wt.%, or less than or equal to about 0.5 wt.%).

Though the adding S730 the additive 731 is illustrated as following the contacting S720, in at least one example embodiment, the contacting S720 and the addition of the additive S730 may occur simultaneously. Similarly, the method 700 may include in certain embodiments, heating the second mixture 728 and/or third mixture 738, such as illustrated in method 500, and/or homogenizing the second mixture 728 and/or third mixture 738, such as illustrated in method 600.

In at least one example embodiment, an example feed solution for use in a spray dried process, like that illustrated in FIGS. 2-4, may be prepared that includes pectin (which is a natural binder for nicotine) and nicotine oil in water*.* The example feed solution may include about 6 wt.% of the pectin (i.e., carrier) and 1.5 wt.% of the nicotine oil in the water solvent. The feed solution may be spray dried using, for example, a VSD-200 spray dryer having a spray rate of ranging from about 9 g/minute to about 15 g/minute and a spray time of about 150 minutes. The feed solution may have an initial product temperature ranging from about 86 °C to about 90 °C. The inlet temperature for the spray dryer may range from about 170 °C to about 200 °C. The example feed solution may be spray dried in accordance with these listed parameters to produce a nicotine-containing powder with the properties listed in Table 1.

**TABLE 1. Example Nicotine-Containing Powder Prepared using Spray Dried Process**

| **Particle Size Distribution** | | | **% Moisture** | **% Yield** |
|---|---|---|---|---|
| **D10 (*µ*m)** | **D50 (*µ*m)** | **D90 (*µ*m)** | | |
| 14.73 | 54.33 | 1131.03 | 7.0 | 54.8 |

In at least one example embodiment, an example feed solution for use in a spray dried process, like that illustrated in FIGS. 2-4, may be prepared that includes pectin (which is a natural binder for nicotine) and nicotine oil in water*.* The example feed solution may include about 6 wt.% of the pectin (i.e., carrier) and 1.5 wt.% of the nicotine oil in the water solvent. The feed solution may be spray dried using, for example, a VSD-200 spray dryer having a spray rate of ranging from about 9 g/minute to about 10 g/minute and a spray time of about 200 minutes. The feed solution may have an initial product temperature ranging from about 96 °C to about 98 °C. The inlet temperature for the spray dryer may be about 200 °C. The example feed solution may be spray dried in accordance with these listed parameters to produce a nicotine-containing powder with the properties listed in Table 2.

**TABLE 2. Example Nicotine-Containing Powder Prepared using Spray Dried Process**

| **Particle Size Distribution** | | | **% Moisture** | **% Yield** |
|---|---|---|---|---|
| **D10 (*µ*m)** | **D50 (*µ*m)** | **D90 (*µ*m)** | | |
| 11.26 | 29.37 | 58.68 | 6.79 | 48.5 |

In at least one example embodiment, an example feed solution for use in a spray dried process, such as illustrated in FIGS. 2-4, may be prepared that includes a mixture of gum Arabic and maltodextrin and nicotine oil in water*.* The example feed solution may include about 30 wt.% of the mixture of gum Arabic and maltodextrin (i.e., carrier) and 3.3 wt.% of the nicotine oil in the water solvent. The ratio of the gum Arabic to maltodextrin may be about 2:3 w/w. The feed solution may be spray dried using, for example, a VSD-200 spray dryer having a spray rate of ranging from about 10 g/minute to about 12 g/minute and a spray time of about 100 minutes. The feed solution may have an initial product temperature ranging from about 92 °C to about 96 °C. The inlet temperature for the spray dryer may range from about 170 °C to about 180 °C. The example feed solution may be spray dried in accordance with these listed parameters to produce a nicotine-containing powder with the properties listed in Table 3.

**TABLE 3. Example Nicotine-Containing Powder Prepared using Spray Dried Process**

| **Particle Size Distribution** | | | **% Moisture** | **% Yield** |
|---|---|---|---|---|
| **D10 (*µ*m)** | **D50 (*µ*m)** | **D90 (*µ*m)** | | |
| 14 | 34.75 | 73.52 | 3.81 | 63.7 |

In at least one example embodiment, an example feed solution for use in a spray dried process, such as illustrated in FIGS. 2-4, may be prepared that includes a mixture of gum Arabic and maltodextrin and nicotine oil in water*.* The example feed solution may include about 30 wt.% of the mixture of gum Arabic and maltodextrin (i.e., carrier) and 7.5 wt.% of the nicotine oil in the water solvent. The ratio of the gum Arabic to maltodextrin may be about 2:3 w/w. The feed solution may be spray dried using, for example, a VSD-200 spray dryer having a spray rate of ranging from about 9 g/minute to about 12 g/minute and a spray time of about 47.7 minutes. The feed solution may have an initial product temperature ranging from about 95 °C to about 102 °C. The inlet temperature for the spray dryer may be about 190 °C. The example feed solution may be spray dried in accordance with these listed parameters to produce a nicotine-containing powder with the properties listed in Table 4.

**TABLE 4. Example Nicotine-Containing Powder Prepared using Spray Dried Process**

| **Particle Size Distribution** | | | **% Moisture** | **% Yield** |
|---|---|---|---|---|
| **D10 (*µ*m)** | **D50 (*µ*m)** | **D90 (*µ*m)** | | |
| 11**.**49 | 30.98 | 68.94 | 2.57 | 47.8 |

### ENCAPSULATED NICOTINE GRANULES

In at least one example embodiment, encapsulated nicotine granules (ENGs) suitable for inclusion in oral products are provided.

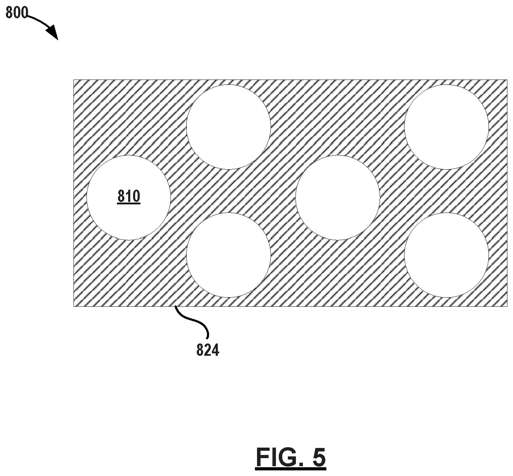

FIG. 5 is a cross-sectional illustration of an example encapsulated nicotine granule 800 for inclusion in oral products such as gums, sprays, lozenges, dissolvable tablets, non-dissolvable chews, films, gels, capsules, and pouches (e.g., containing fibers or granules). In a least one example embodiment, the encapsulated nicotine granule 800 includes a nicotine-containing material 810 that is surrounded or encapsulated by a matrix 824. Though nicotine-containing material 810 dispersed within the matrix 824 is illustrated, the skilled artisan will recognize that in various other instances the matrix 824 may take various other shapes or configurations. For example, in some example embodiments, the matrix 824 may have a nicotine material 810 dispersed homogeneously throughout the encapsulated nicotine granule 800.

The encapsulated nicotine granule 800 may have an average particle size (D50) ranging from about 100 µm to about 5 mm. For example, the encapsulated nicotine granule 800 may have an average particle size greater than or equal to about 100 µm (e.g., greater than or equal to about 200 µm, greater than or equal to about 300 µm, greater than or equal to about 400 µm, greater than or equal to about 500 µm, greater than or equal to about 600 µm, greater than or equal to about 700 µm, greater than or equal to about 800 µm, greater than or equal to about 900 µm, greater than or equal to about 1 mm, greater than or equal to about 1.5 mm, greater than or equal to about 2.0 mm, greater than or equal to about 2.5 mm, greater than or equal to about 3.0 mm, greater than or equal to about 3.5 mm, greater than or equal to about 4.0 mm, greater than or equal to about 4.5 mm, or greater than or equal to about 5.0 mm). The encapsulated nicotine granulate 800 may have an average particle size less than or equal to about 5 mm (e.g., less than or equal to about 4.5 mm, less than or equal to about 4.0 mm, less than or equal to about 3.5 mm, less than or equal to about 3.0 mm, less than or equal to about 2.5 mm, less than or equal to about 2.0 mm, less than or equal to about 1.5 mm, less than or equal to about 1 mm, less than or equal to about 900 µm, less than or equal to about 800 µm, less than or equal to about 700 µm, less than or equal to about 600 µm, less than or equal to about 500 µm, less than or equal to about 400 µm, less than or equal to about 300 µm, less than or equal to about 200 µm, or less than or equal to about 150 µm).

In some example embodiment, the nicotine-containing material 810 includes nicotine, a nicotine complex (such as, nicotine polacrilex), a nicotine salt, or any combination thereof. The nicotine salt may include, for example, nitrate, monotartrate, bitartrate, bitartrate dihydrate, salicylate, sulfate or bisulfate, phosphate or acid phosphate, acetate, lactate, succinate, maleate, fumarate, gluconate, saccharate, benzoate, methanesulfonate, hydrochloride, hydrobromide, hydroiodide, or any combination thereof.

In at least one example embodiment, the matrix 824 includes sugar alcohols, humectants/oils, or a combination of sugar alcohols and humectants/oils. The sugar alcohol may include sorbitol, mannitol, xylitol, maltitol, lactitol, isomalt, hydrogenated isomaltulose, hydrogenated starch hydrolyzates, or any combination thereof. The humectant/oil may help to maintain the moisture levels of the matrix 824. In some example embodiments, the humectant includes glycerol, propylene glycol, or a combination of glycerol and propylene glycol.

In at least one example embodiment, the matrix 824 may include an amount of the sugar alcohols ranging from about 50 wt.% to about 99.5 wt.%. For example, the matrix 824 may include greater than or equal to about 50 wt.% of the sugar alcohols (e.g., greater than or equal to about 55 wt.%, greater than or equal to about 60 wt.%, greater than or equal to about 65 wt.%, greater than or equal to about 70 wt.%, greater than or equal to about 75 wt.%, greater than or equal to about 80 wt.%, greater than or equal to about 85 wt.%, greater than or equal to about 90 wt.%, or greater than or equal to about 95 wt.%). The matrix 824 may include less than or equal to about 99.5 wt.% of the sugar alcohols (e.g., less than or equal to about 95 wt.%, less than or equal to about 90 wt.%, less than or equal to about 85 wt.%, less than or equal to about 80 wt.%, less than or equal to about 75 wt.%, less than or equal to about 70 wt.%, less than or equal to about 65 wt.%, less than or equal to about 60 wt.%, or less than or equal to about 55 wt.%).

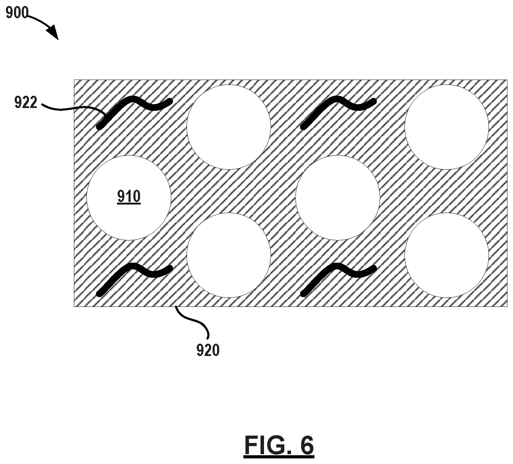

FIG. 6 is a cross-sectional illustration of an example encapsulated nicotine granule 900 for inclusion in oral products such as gums, sprays, lozenges, dissolvable tablets, non-dissolvable chews, films, gels, capsules, and pouches (e.g., containing fibers or granules). The encapsulated nicotine granule 900 is the same as encapsulated nicotine granule 800 except that encapsulated nicotine granule 900 includes one or more additives 922.

In a least one example embodiment, the encapsulated nicotine granule 900 includes a nicotine-containing material 910 that is surrounded or encapsulated by a matrix 920. The matrix 920 may include one or more additives 922. For example, the matrix 920 may include one or more additives 922 dispersed throughout the matrix 920. The one or more additives 922 may be substantially uniformly dispersed within the matrix 920 and around the nicotine-containing material 910. The encapsulated nicotine granule 900 may include an amount of the one or more additives 922 ranging from greater than 0 wt.% to less than or equal to about 10 wt.%. For example, the encapsulated nicotine granule 900 may include greater than 0 wt.% of the one or more additives 922 (e.g., greater than or equal to about 0.1 wt.%, greater than or equal to about 0.5 wt.%, greater than or equal to about 1.0 wt.%, greater than or equal to about 1.5 wt.%, greater than or equal to about 2.0 wt.%, greater than or equal to about 2.5 wt.%, greater than or equal to about 3.0 wt.%, greater than or equal to about 3.5 wt.%, greater than or equal to about 4.0 wt.%, greater than or equal to about 4.5 wt.%, greater than or equal to about 5.0 wt.%, greater than or equal to about 5.5 wt.%, greater than or equal to about 6.0 wt.%, greater than or equal to about 6.5 wt.%, greater than or equal to about 7.0 wt.%, greater than or equal to about 7.5 wt.%, greater than or equal to about 8.0 wt.%, greater than or equal to about 8.5 wt.%, greater than or equal to about 9.0 wt.%, or greater than or equal to about 9.5 wt.%). The encapsulated nicotine granule 900 may include less than or equal to about 10 wt.% of the one or more 922 (e.g., less than or equal to about 9.5 wt.%, less than or equal to about 9.0 wt.%, less than or equal to about 8.5 wt.%, less than or equal to about 8.0 wt.%, less than or equal to about 7.5 wt.%, less than or equal to about 7.0 wt.%, less than or equal to about 6.5 wt.%, less than or equal to about 6.0 wt.%, less than or equal to about 5.5 wt.%, less than or equal to about 5.0 wt.%, less than or equal to about 4.5 wt.%, less than or equal to about 4.0 wt.%, less than or equal to about 3.5 wt.%, less than or equal to about 3.0 wt.%, less than or equal to about 2.5 wt.%, less than or equal to about 2.0 wt.%, less than or equal to about 1.5 wt.%, less than or equal to about 1.0 wt.%, or less than or equal to about 0.5 wt.%).

The one or more additives 922 may include, for example, an antioxidant, sweetener, pH adjuster, polysaccharide, flavorant, or any combination thereof. In at least one example embodiment, the antioxidant additive includes ascorbyl palmitate, tertiary butylhydroquinone (TBHQ), sodium ascorbate, or any combination thereof. In at least one example embodiment, the sweetener includes acesulfame potassium, aspartame, sodium saccharin, sucralose, or any combination thereof. In at least one example embodiment, the pH adjuster includes sodium carbonate/bicarbonate, potassium carbonate/bicarbonate, citric acid, or any combination thereof. In at least one example embodiment, the polysaccharide includes guar gum, xanthum gum, gum arabic, or any combination thereof.

The flavorant may include spray dried, powdered, and/or liquid flavors. In at least one example embodiment, the flavorant includes peppermint, spearmint, wintergreen, menthol, cinnamon, chocolate, vanillin, licorice, clove, anise, sandalwood, geranium, rose oil, vanilla, lemon oil, cassia, fennel, ginger, ethylacetate, isoamylacetate, propylisobutyrate, isobutylbutyrate, ethylbutyrate, ethylvalerate, benzylformate, limonene, cymene, pinene, linalool, geraniol, citronellol, citral, orange oil, coriander oil, borneol, fruit extract, coffee, tea, cacao, mint, pomegranate, acai, raspberry, blueberry, strawberry, boysenberry, cranberry, bourbon, scotch, whiskey, cognac, hydrangea, lavender, apple, peach, pear, cherry, plum, orange, lime, lichy, grape, grapefruit, butter, rum, coconut, almond, pecan, walnut, hazelnut, french vanilla, macadamia, sugar cane, maple, cassis, caramel, banana, malt, espresso, kahlua, white chocolate, spice flavors such as cinnamon, clove, cilantro, basil, oregano, garlic, mustard, nutmeg, rosemary, thyme, tarragon, dill, sage, anise, and fennel, methyl salicylate, linalool, jasmine, coffee, olive oil, sesame oil, sunflower oil, bergamot oil, geranium oil, lemon oil, ginger oil, balsamic vinegar, rice wine vinegar, and red wine vinegar, all spice, pimento, mango, soursop, sweetsop, naseberry, sorrel, or any combination thereof.

Methods for preparing encapsulated nicotine granules (ENGs), like those illustrated in FIG. 5 and/or FIG. 6, that are suitable for inclusion in oral products such as gums, sprays, lozenges, dissolvable tablets, non-dissolvable chews, films, gels, capsules, and pouches (e.g., containing fibers or granules) are provided. In some example embodiments, a method for forming such encapsulated nicotine granules includes heating and cooling a first mixture, adding a nicotine-containing material to the cooled first mixture to form a second mixture, and cooling the second mixture to a further temperature to form solidified structures, such as one or more sheets. The one or more solidified structures can be fragmented to form the encapsulated nicotine granules for inclusion in oral products and having desired sensory characteristics.

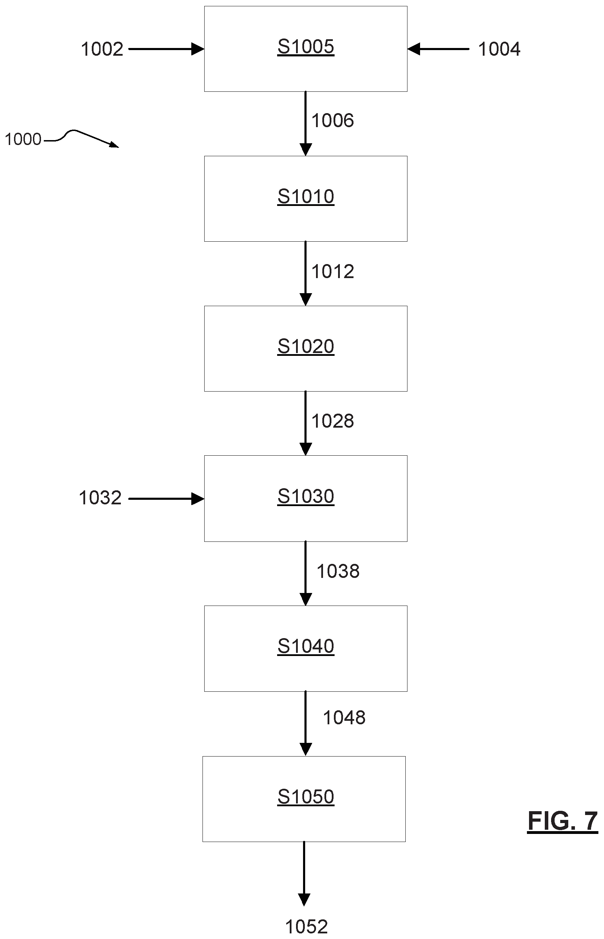

FIG. 7 is a flow diagram illustrating a method 1000 for preparing a nicotine-containing powder 1052 for inclusion in oral products such as gums, sprays, lozenges, dissolvable tablets, non-dissolvable chews, films, gels, capsules, and pouches (e.g., containing fibers or granules). The nicotine-containing powder 1052 may include a plurality of encapsulated nicotine granules, such as illustrated in FIG. 5 and/or FIG. 6. The method 1000 can be performed using a batch process, a continuous process, or both a batch process and a continuous process (e.g., compounding extrusion).

In as least one example embodiment, the method 1000 for preparing a nicotine-containing powder 1052 includes preparing S1010 a molten mixture 1012. In some example embodiments, preparing S1010 the molten mixture 1012 includes heating a first mixture 1006. For example, the first mixture 1006 may be heated to a first temperature.

In at least one example embodiment, the first temperature ranges from about 120 °C to about 200 °C. For example, the first temperature may be greater than or equal to about 120 °C (e.g., greater than or equal to about 125 °C, greater than or equal to about 130 °C, greater than or equal to about 135 °C, greater than or equal to about 140 °C, greater than or equal to about 145 °C, greater than or equal to about 150 °C, greater than or equal to about 155 °C, greater than or equal to about 160 °C, greater than or equal to about 165 °C, greater than or equal to about 170 °C, greater than or equal to about 175 °C, greater than or equal to about 180 °C, greater than or equal to about 185 °C, greater than or equal to about 190 °C, or greater than or equal to about 195 °C). The first temperature may be less than or equal to about 200 °C (e.g., less than or equal to about 195 °C, less than or equal to about 190 °C, less than or equal to about 185 °C, less than or equal to about 180 °C, less than or equal to about 175 °C, less than or equal to about 170 °C, less than or equal to about 165 °C, less than or equal to about 160 °C, less than or equal to about 155 °C, less than or equal to about 150 °C, less than or equal to about 145 °C, less than or equal to about 140 °C, less than or equal to about 135 °C, less than or equal to about 130 °C, or less than or equal to about 125 °C).

The first mixture 1006 may include one or more polyols (e.g., sugars and/or sugar alcohols) 1002. In at least one example embodiment, the method 1000 includes preparing S1005 the first mixture 1006. In some example embodiments, preparing S1005 the first mixture 1006 includes contacting one or more polyols 1002 and an aqueous solvent 1004. For example, the one or more polyols 1002 may be mixed with the aqueous solvent 1004 to form the first mixture 1006. Although not illustrated, in at least one example embodiment, the first mixture 1006 may further include one or more additives. The one or more additives may include, for example only, an antioxidant, sweetener, pH adjuster, polysaccharide, flavorant, or any combination thereof.

In at least one example embodiment, the method 1000 includes forming a cooled molten mixture 1028. In some example embodiments, forming the cooled molten mixture 1028 includes cooling S1020 the molten mixture 1012. For example, the molten mixture 1012 may be cooled to a second temperature to form the cooled molten mixture 1028.

In at least one example embodiment, the second temperature ranges from about 65 °C to about 200 °C. For example, the second temperature may be greater than or equal to 65 °C (e.g., greater than or equal to about 70 °C, greater than or equal to about 75 °C, greater than or equal to about 80 °C, greater than or equal to about 85 °C, greater than or equal to about 90 °C, greater than or equal to about 95 °C, greater than or equal to about 100 °C, greater than or equal to about 105 °C, greater than or equal to about 110 °C, greater than or equal to about 115 °C, greater than or equal to about 120 °C, greater than or equal to about 125 °C, greater than or equal to about 130 °C, greater than or equal to about 135 °C, greater than or equal to about 140 °C, greater than or equal to about 145 °C, greater than or equal to about 150 °C, greater than or equal to about 155 °C, greater than or equal to about 160 °C, greater than or equal to about 165 °C, greater than or equal to about 170 °C, greater than or equal to about 175 °C, greater than or equal to about 180 °C, greater than or equal to about 185 °C, greater than or equal to about 190 °C, or greater than or equal to about 195 °C). The second temperature may be less than or equal to about 200 °C (e.g., less than or equal to about 195 °C, less than or equal to about 190 °C, less than or equal to about 185 °C, less than or equal to about 180 °C, less than or equal to about 175 °C, less than or equal to about 170 °C, less than or equal to about 165 °C, less than or equal to about 160 °C, less than or equal to about 155 °C, less than or equal to about 150 °C, less than or equal to about 145 °C, less than or equal to about 140 °C, less than or equal to about 135 °C, less than or equal to about 130 °C, less than or equal to about 125 °C, less than or equal to about 120 °C, less than or equal to about 115 °C, less than or equal to 110 °C, less than or equal to about 105 °C, less than or equal to about 100 °C, less than or equal to about 95 °C, less than or equal to about 90 °C, less than or equal to about 85 °C, less than or equal to about 80 °C, less than or equal to about 75 °C, or less than or equal to about 70 °C).

In at least one example embodiment, the method 1000 includes forming a second mixture 1038. In some example embodiments, forming the second mixture 1038 includes adding S1030 one or more nicotine-containing materials 1032 to the cooled molten mixture 1028.

The second mixture 1038 may include an amount of the cooled molten mixture 1028 ranging from about 0.1 wt.% to about 50 wt.%. For example, the second mixture 1038 may include greater than or equal to about 0.1 wt.% of the cooled molten mixture 1028 (e.g., greater than or equal to about 0.5 wt.%, greater than or equal to about 1 wt.%, greater than or equal to about 5 wt.%, greater than or equal to about 10 wt.%, greater than or equal to about 15 wt.%, greater than or equal to about 20 wt.%, greater than or equal to about 25 wt.%, greater than or equal to about 30 wt.%, greater than or equal to about 35 wt.%, greater than or equal to about 40 wt.%, or greater than or equal to about 45 wt.%). The second mixture 1038 may include less than or equal to about 50 wt.% of the cooled molten mixture 1028 (e.g., less than or equal to about 45 wt.%, less than or equal to about 40 wt.%, less than or equal to about 35 wt.%, less than or equal to about 30 wt.%, less than or equal to about 25 wt.%, less than or equal to about 20 wt.%, less than or equal to about 15 wt.%, less than or equal to about 10 wt.%, less than or equal to about 5 wt.%, less than or equal to about 1 wt.%, or less than or equal to about 0.5 wt.%.

In at least one example embodiment, the second mixture 1038 may include an amount of the cooled molten mixture 1028 ranging from about 60 wt.% to about 80 wt.%. For example, the second mixture 1038 may include greater than or equal to about 60 wt.% of the cooled molten mixture 1028 (e.g., greater than or equal to about 62 wt.%, greater than or equal to about 64 wt.%, greater than or equal to about 66 wt.%, greater than or equal to about 68 wt.%, greater than or equal to about 70 wt.%, greater than or equal to about 72 wt.%, greater than or equal to about 74 wt.%, greater than or equal to about 76 wt.%, or greater than or equal to about 78 wt.%). The second mixture 1038 may include less than or equal to about 80 wt.% (e.g., less than or equal to about 78 wt.%, less than or equal to about 76 wt.%, less than or equal to about 74 wt.%, less than or equal to about 72 wt.%, less than or equal to about 70 wt.%, less than or equal to about 68 wt.%, less than or equal to about 66 wt.%, less than or equal to about 64 wt.%, or less than or equal to about 62 wt.%).

The second mixture 1038 may include an amount of the one or more nicotine-containing materials 1032 ranging from about 0.1 wt.% to about 50 wt.%. For example, the second mixture 1038 may include greater than or equal to about 0.1 wt.% of the nicotine-containing material 1032 (e.g., greater than or equal to about 0.5 wt.%, greater than or equal to about 1 wt.%, greater than or equal to about 5 wt.%, greater than or equal to about 10 wt.%, greater than or equal to about 15 wt.%, greater than or equal to about 20 wt.%, greater than or equal to about 25 wt.%, greater than or equal to about 30 wt.%, greater than or equal to about 35 wt.%, greater than or equal to about 40 wt.%, or greater than or equal to about 45 wt.%). The second mixture 1038 may include less than or equal to about 50 wt.% of the nicotine-containing material 1032 (e.g., less than or equal to about 45 wt.%, less than or equal to about 40 wt.%, less than or equal to about 35 wt.%, less than or equal to about 30 wt.%, less than or equal to about 25 wt.%, less than or equal to about 20 wt.%, less than or equal to about 15 wt.%, less than or equal to about 10 wt.%, less than or equal to about 5 wt.%, less than or equal to about 1 wt.%, or less than or equal to about 0.5 wt.%.

In at least one example embodiment, the second mixture 1038 may include an amount of the nicotine-containing materials 1032 ranging from about 20 wt.% to about 40 wt.%. For example, the second mixture 1038 may include greater than or equal to about 20 wt.% of the nicotine-containing materials 1032 (e.g., greater than or equal to about 22 wt.%, greater than or equal to about 24 wt.%, greater than or equal to about 26 wt.%, greater than or equal to about 28 wt.%, greater than or equal to about 30 wt.%, greater than or equal to about 32 wt.%, greater than or equal to about 34 wt.%, greater than or equal to about 36 wt.%, or greater than or equal to about 38 wt.%). The second mixture may include less than or equal to about 40 wt.% of the nicotine-containing materials 1032 (e.g., less than or equal to about 38 wt.%, less than or equal to about 36 wt.%, less than or equal to about 34 wt.%, less than or equal to about 32 wt.%, less than or equal to about 30 wt.%, less than or equal to about 28 wt.%, less than or equal to about 26 wt.%, less than or equal to about 24 wt.%, or less than or equal to about 22 wt.%).

Although not illustrated, in at least one example embodiment, the second mixture 1038 may include one or more additives and forming the second mixture may include adding the one or more additives to the cooled molten mixture 1028. The one or more additives may include, for example only, an antioxidant, sweetener, pH adjuster, polysaccharide, flavorant, or any combination thereof.

In some example embodiments, the one or more nicotine-containing materials 1032 include nicotine, a nicotine complex (such as, nicotine polacrilex), a nicotine salt, or any combination thereof. The nicotine salt may include, for example, nitrate, monotartrate, bitartrate, bitartrate dihydrate, salicylate, sulfate or bisulfate, phosphate or acid phosphate, acetate, lactate, succinate, maleate, fumarate, gluconate, saccharate, benzoate, methanesulfonate, hydrochloride, hydrobromide, hydroiodide, or any combination thereof.

In at least one example embodiment, such as when the one or more nicotine-containing materials 1032 include solid nicotine salts, the second temperature ranges from about 120 °C to about 130 °C. For example, the second temperature may be greater than or equal to about 120 °C (e.g., greater than or equal to about 121 °C, greater than or equal to about 122 °C, greater than or equal to about 123 °C, greater than or equal to about 124 °C, greater than or equal to about 125 °C, greater than or equal to about 126 °C, greater than or equal to about 127 °C, greater than or equal to about 128 °C, or greater than or equal to about 129 °C). The second temperature may be less than or equal to about 130 °C (e.g., less than or equal to about 129 °C, less than or equal to about 128 °C, less than or equal to about 127 °C, less than or equal to about 126 °C, less than or equal to about 125 °C, less than or equal to about 124 °C, less than or equal to about 123 °C, less than or equal to about 122 °C, or less than or equal to about 121 °C).

In at least one example embodiment, such as when the one or more nicotine-containing materials 1032 include a liquid nicotine solution or nicotine salt, the second temperature ranges from about 65 °C to about 100 °C. For example, the second temperature may be greater than or equal to about 65 °C (e.g., greater than or equal to about 70 °C, greater than or equal to about 75 °C, greater than or equal to about 80 °C, greater than or equal to about 85 °C, greater than or equal to about 90 °C, or greater than or equal to about 95 °C). The second temperature may be less than or equal to about 100 °C (e.g., less than or equal to about 95 °C, less than or equal to about 90 °C, less than or equal to about 85 °C, less than or equal to about 80 °C, less than or equal to about 75 °C, or less than or equal to about 70 °C).

In at least one example embodiment, the method 1000 includes forming one or more solidified structures 1048. In some example embodiments, forming the one or more solidified structures 1048 includes cooling S1040 the second mixture 1038. For example, the second mixture 1038 may be cooled to a third temperature to form the cooled molten mixture 1048. In some example embodiments, forming one or more solidified structures 1048 includes glassifying the second mixture 1038. For example, the third temperature may be below the glass transition temperature (T_{g}) of the one or more polyols 1002.

In at least one example embodiment, the third temperature ranges from about 40 °C to about 45 °C. For example, the third temperature may be greater than or equal to about 40 °C (e.g., greater than or equal to about 40.5 °C, greater than or equal to about 41 °C, greater than or equal to about 41.5 °C, greater than or equal to about 42 °C, greater than or equal to about 42.5 °C, greater than or equal to about 43 °C, greater than or equal to about 43.5 °C, greater than or equal to about 44 °C, or greater than or equal to about 44.5 °C). The third temperature maybe less than or equal to about 45 °C (e.g., less than or equal to about 44.5 °C, less than or equal to about 44 °C, less than or equal to about 43.5 °C, less than or equal to about 43 °C, less than or equal to about 42.5 °C, less than or equal to about 42 °C, less than or equal to about 41.5 °C, less than or equal to about 41 °C, or less than or equal to about 40.5 °C).

The one or more solidified structures 1048 may include one or more sheets, or one of a variety of other configurations such as would be recognized by one of ordinary skill in the art. For example, in at least one example embodiment, the one or more solidified structures 1048 may include one or more brittle sheets at room temperature.

In at least one example embodiment, the method 1000 includes fragmenting S1050 the one or more solidified structures 1048 to form the nicotine-containing powder 1052.

Nicotine-containing materials, such as nicotine-containing powders, under ambient conditions, are often sensitive to light and air and because of various chemical and physical properties can present various difficulties during the preparation, processing, and storage of oral product including the nicotine-containing materials.

Example encapsulated nicotine granules prepared in accordance with at least one example embodiment and included in oral products (such as, gums, sprays, lozenges, dissolvable tablets, non-dissolvable chews, films, gels, capsules, and pouches (e.g., containing fibers or granules), by way of example) may be less irritating to the consumer.

### ENCAPSULATED SWEETENER GRANULES

In at least one example embodiment, encapsulated sweetener granules (ESGs) suitable for inclusion in oral products are provided.

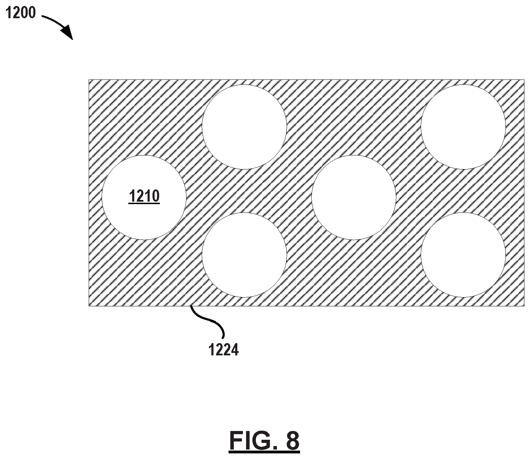

FIG. 8 is a cross-sectional illustration of an example encapsulated sweetener granule 1200 for inclusion in oral products such as gums, sprays, lozenges, dissolvable tablets, non-dissolvable chews, films, gels, capsules, and pouches (e.g., containing fibers or granules). In a least one example embodiment, the encapsulated sweetener granule 1200 includes a sweetener-containing material 1210 that is surrounded or encapsulated by a matrix 1224. Though nicotine-containing material 1210 dispersed within the matrix 1224 is illustrated, the skilled artisan will recognize that in various other instances the matrix 1224 may take various other shapes or configurations. For example, in some example embodiments, the matrix 1224 has the sweetener-containing material 1210 dispersed homogenously throughout the encapsulated sweetener granule 1200. In some other example embodiments, the matrix 112 may define a continuous coating that surrounds or encapsulates the sweetener-containing material 1210.

The encapsulated sweetener granule 1200 may have an average particle size (D50) ranging from about 100 µm to about 5 mm. For example, the encapsulated sweetener granule 1200 may have an average particle size greater than or equal to about 100 µm (e.g., greater than or equal to about 200 µm, greater than or equal to about 300 µm, greater than or equal to about 400 µm, greater than or equal to about 500 µm, greater than or equal to about 600 µm, greater than or equal to about 700 µm, greater than or equal to about 800 µm, greater than or equal to about 900 µm, greater than or equal to about 1 mm, greater than or equal to about 1.5 mm, greater than or equal to about 2.0 mm, greater than or equal to about 2.5 mm, greater than or equal to about 3.0 mm, greater than or equal to about 3.5 mm, greater than or equal to about 4.0 mm, greater than or equal to about 4.5 mm, or greater than or equal to about 5.0 mm). The encapsulated sweetener granulate 1200 may have an average particle size less than or equal to about 5 mm (e.g., less than or equal to about 4.5 mm, less than or equal to about 4.0 mm, less than or equal to about 3.5 mm, less than or equal to about 3.0 mm, less than or equal to about 2.5 mm, less than or equal to about 2.0 mm, less than or equal to about 1.5 mm, less than or equal to about 1 mm, less than or equal to about 900 µm, less than or equal to about 800 µm, less than or equal to about 700 µm, less than or equal to about 600 µm, less than or equal to about 500 µm, less than or equal to about 400 µm, less than or equal to about 300 µm, less than or equal to about 200 µm, or less than or equal to about 150 µm).

In some example embodiment, the sweetener-containing material 1210 includes one or more high-intensity sweeteners. The one or more high-intensity sweeteners may include a synthetic sweetener and/or a natural sweetener. In at least one example embodiment, the natural sweetener includes a sugar (such as a monosaccharide, a disaccharide, and/or a polysaccharide). In at least one example embodiment, the natural sweetener includes sucrose (e.g., table sugar), honey, a mixture of low-molecular-weight sugars excluding sucrose, glucose (e.g., grape sugar, corn sugar, dextrose), molasses, corn sweetener, glucose syrup (e.g., corn syrup), fructose (e.g., fruit sugar), lactose (e.g., milk sugar), maltose (e.g., malt sugar, maltobiose), sorghum syrup, fruit juice concentrate, or any combination thereof. In at one example embodiment, the sweetener-containing material 1210 includes a sugar alcohol. The sugar alcohol may include mannitol, sorbitol, xylitol, maltitol, or any combination thereof. In at least one example embodiment, the sweetener-containing material 1210 includes a non-nutritive sweetener including stevia, saccharin, aspartame, sucralose, acesulfame potassium, or any combination thereof.

In at least one example embodiment, the matrix 1224 includes sugar alcohols, humectants/oils, or a combination of sugar alcohols and humectants/oils. The sugar alcohol may include sorbitol, mannitol, xylitol, maltitol, lactitol, isomalt, hydrogenated isomaltulose, hydrogenated starch hydrolyzates, or any combination thereof. The humectant/oil may help to maintain the moisture levels of the matrix 1224. In some example embodiments, the humectant includes glycerol, propylene glycol, or a combination of glycerol and propylene glycol.

In at least one example embodiment, the matrix 1224 may include an amount of the sugar alcohols ranging from about 50 wt.% to about 99.5 wt.%. For example, the matrix 824 may include greater than or equal to about 50 wt.% of the sugar alcohols (e.g., greater than or equal to about 55 wt.%, greater than or equal to about 60 wt.%, greater than or equal to about 65 wt.%, greater than or equal to about 70 wt.%, greater than or equal to about 75 wt.%, greater than or equal to about 80 wt.%, greater than or equal to about 85 wt.%, greater than or equal to about 90 wt.%, or greater than or equal to about 95 wt.%). The matrix 824 may include less than or equal to about 99.5 wt.% of the sugar alcohols (e.g., less than or equal to about 95 wt.%, less than or equal to about 90 wt.%, less than or equal to about 85 wt.%, less than or equal to about 80 wt.%, less than or equal to about 75 wt.%, less than or equal to about 70 wt.%, less than or equal to about 65 wt.%, less than or equal to about 60 wt.%, or less than or equal to about 55 wt.%).

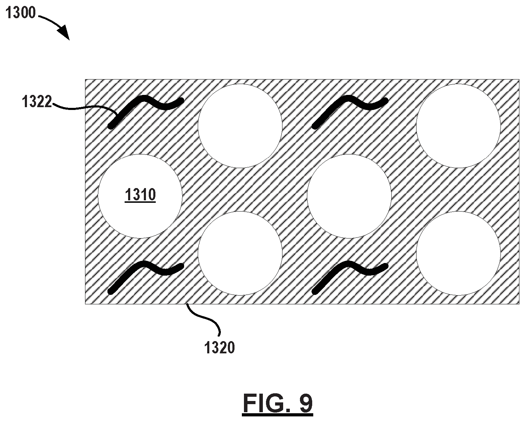

FIG. 9 is a cross-sectional illustration of an example encapsulated sweetener granule 1300 for inclusion in oral products such as gums, sprays, lozenge, dissolvable tablets, non-dissolvable chews, films, gels, capsules, and pouches (e.g., containing fibers or granules). The encapsulated sweetener granule 1300 is the same as encapsulated sweetener granule 1200 except that encapsulated sweetener granule 1300 includes one or more additives 1322.

In a least one example embodiment, the encapsulated sweetener granule 1300 includes a sweetener-containing material 1310 that is surrounded or encapsulated by a matrix 1320. The matrix 1320 may include one or more additives 1322. For example, the matrix 1320 may include one or more additives 1322 dispersed throughout the matrix 1320. The one or more additives 1322 may be substantially uniformly dispersed within the matrix 1320 and around the sweetener-containing material 1310.

The encapsulated sweetener granule 1300 may include an amount of the one or more additives 1322 ranging from about 0.01 wt.% to about 45 wt.%. For example, the encapsulated sweetener granule 1300 may include greater than or equal to about 0.01 wt.% of the one or more additives 1322 (e.g., greater than or equal to about 5 wt.%, greater than or equal to about 10 wt.%, greater than or equal to about 15 wt.%, greater than or equal to about 20 wt.%, greater than or equal to about 25 wt.%, greater than or equal to about 30 wt.%, greater than or equal to about 35 wt.%, or greater than or equal to about 40 wt.%). The encapsulated sweetener granule 1300 may include less than or equal to about 45 wt.% of the one or more additives 1322 (e.g., less than or equal to about 40 wt.%, less than or equal to about 35 wt.%, less than or equal to about 30 wt.%, less than or equal to about 25 wt.%, less than or equal to about 20 wt.%, less than or equal to about 15 wt.%, less than or equal to about 10 wt.%, or less than or equal to about 5 wt.%).

The one or more additives 1322 may include, for example, an antioxidant, sweetener, pH adjuster, polysaccharide, flavorant, or any combination thereof. In at least one example embodiment, the antioxidant additive includes ascorbyl palmitate, tertiary butylhydroquinone (TBHQ), sodium ascorbate, or any combination thereof. In at least one example embodiment, the sweetener includes acesulfame potassium, aspartame, sodium saccharin, sucralose, or any combination thereof. In at least one example embodiment, the pH adjuster includes sodium carbonate/bicarbonate, potassium carbonate/bicarbonate, citric acid, or any combination thereof. In at least one example embodiment, the polysaccharide includes guar gum, xanthum gum, gum arabic, or any combination thereof.

The flavorant may include spray dried, powdered, and/or liquid flavors. In at least one example embodiment, the flavorant includes peppermint, spearmint, wintergreen, menthol, cinnamon, chocolate, vanillin, licorice, clove, anise, sandalwood, geranium, rose oil, vanilla, lemon oil, cassia, fennel, ginger, ethylacetate, isoamylacetate, propylisobutyrate, isobutylbutyrate, ethylbutyrate, ethylvalerate, benzylformate, limonene, cymene, pinene, linalool, geraniol, citronellol, citral, orange oil, coriander oil, borneol, fruit extract, coffee, tea, cacao, mint, pomegranate, acai, raspberry, blueberry, strawberry, boysenberry, cranberry, bourbon, scotch, whiskey, cognac, hydrangea, lavender, apple, peach, pear, cherry, plum, orange, lime, lichy, grape, grapefruit, butter, rum, coconut, almond, pecan, walnut, hazelnut, french vanilla, macadamia, sugar cane, maple, cassis, caramel, banana, malt, espresso, kahlua, white chocolate, spice flavors such as cinnamon, clove, cilantro, basil, oregano, garlic, mustard, nutmeg, rosemary, thyme, tarragon, dill, sage, anise, and fennel, methyl salicylate, linalool, jasmine, coffee, olive oil, sesame oil, sunflower oil, bergamot oil, geranium oil, lemon oil, ginger oil, balsamic vinegar, rice wine vinegar, and red wine vinegar, all spice, pimento, mango, soursop, sweetsop, naseberry, sorrel, or any combination thereof.

Methods for preparing encapsulated sweetener granules (ESGs), like those illustrated in FIG. 8 and/or FIG. 9, that are suitable for inclusion in oral products such as gums, sprays, lozenges, dissolvable tablets, non-dissolvable chews, films, gels, capsules, and pouches (e.g., containing fibers or granules) are provided. In some example embodiments, the method for forming such encapsulated sweetener granules includes heating and cooling a first mixture, adding a sweetener-containing material to the cooled first mixture to form a second mixture, and cooling the second mixture to a further temperature to form solidified structures, such as one or more sheets. The one or more solidified structures can be fragmented to form the encapsulated sweetener granules for inclusion in oral products and having desired sensory characteristics.

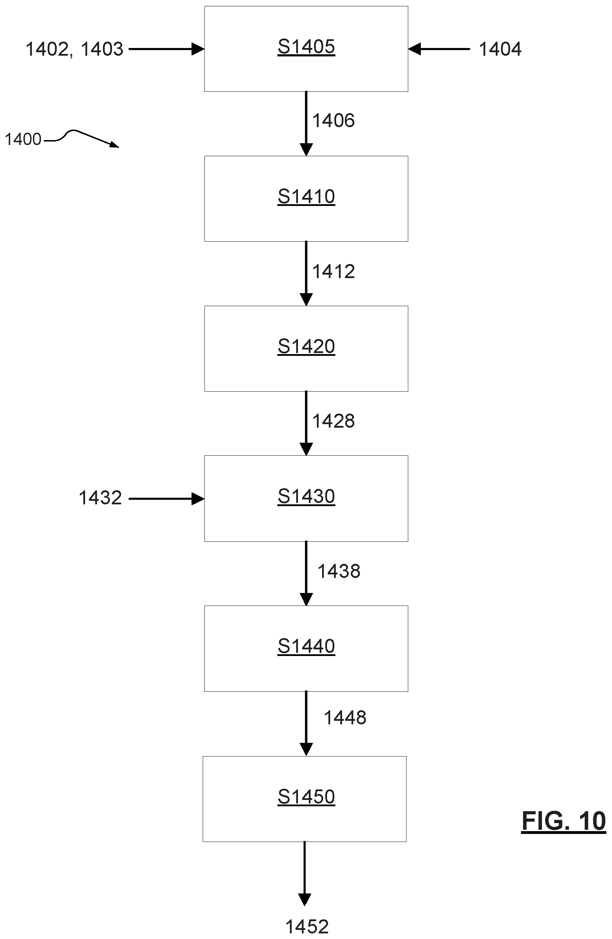

FIG. 10 is a flow diagram illustrating a method 1400 for preparing a sweetener-containing powder 1452 for inclusion in oral products such as gums, sprays, lozenges, dissolvable tablets, non-dissolvable chews, films, gels, capsules, and pouches (e.g., containing fibers or granules). The sweetener-containing powder 1452 may include a plurality of encapsulated nicotine granules, such as illustrated in FIG. 8 and/or FIG. 9. The method 1400 can be performed using a batch process, a continuous process, or both a batch process and a continuous process (e.g., compounding extrusion).

In as least one example embodiment, the method 1400 for preparing a sweetener-containing powder 1442 includes preparing S1410 a molten mixture 1412. In some example embodiments, preparing S1410 the molten mixture 1412 includes heating a first mixture 1406. For example, the first mixture 1406 may be heated to a first temperature.

In at least one example embodiment, the first temperature range from about 120 °C to about 200 °C. For example, the first temperature may be greater than or equal to about 120 °C (e.g., greater than or equal to about 125 °C, greater than or equal to about 130 °C, greater than or equal to about 135 °C, greater than or equal to about 140 °C, greater than or equal to about 145 °C, greater than or equal to about 150 °C, greater than or equal to about 155 °C, greater than or equal to about 160 °C, greater than or equal to about 165 °C, greater than or equal to about 170 °C, greater than or equal to about 175 °C, greater than or equal to about 180 °C, greater than or equal to about 185 °C, greater than or equal to about 190 °C, or greater than or equal to about 195 °C). The first temperature may be less than or equal to about 200 °C (e.g., less than or equal to about 195 °C, less than or equal to about 190 °C, less than or equal to about 185 °C, less than or equal to about 180 °C, less than or equal to about 175 °C, less than or equal to about 170 °C, less than or equal to about 165 °C, less than or equal to about 160 °C, less than or equal to about 155 °C, less than or equal to about 150 °C, less than or equal to about 145 °C, less than or equal to about 140 °C, less than or equal to about 135 °C, less than or equal to about 130 °C, or less than or equal to about 125 °C).

The first mixture 1406 may include one or more polyols 1402 and/or one or more hydrocolloids 1403. In at least one example embodiment, the one or more polyols 1402 may include isomalt, maltitol, mannitol, sorbitol, xylitol, or any combination thereof. In at least one example embodiment, the one or more hydrocolloids 1403 may include guar gum, gum arabic, or any combination thereof.

The first mixture 1406 may include an amount of the one or more polyols 1402 ranging from about 50 wt.% to about 99.5 wt.%. For example, the first mixture 1406 may include greater than or equal to about 50 wt.% of the one or more polyols 1402 (e.g., greater than or equal to about 55 wt.%, greater than or equal to about 60 wt.%, greater than or equal to about 65 wt.%, greater than or equal to about 70 wt.%, greater than or equal to about 75 wt.%, greater than or equal to about 80 wt.%, greater than or equal to about 85 wt.%, greater than or equal to about 90 wt.%, or greater than or equal to about 95 wt.%). The first mixture 1406 may include less than or equal to about 99.5 wt.% of the one or more polyols 1402 (e.g., less than or equal to about 95 wt.%, less than or equal to about 90 wt.%, less than or equal to about 85 wt.%, less than or equal to about 80 wt.%, less than or equal to about 75 wt.%, less than or equal to about 70 wt.%, less than or equal to about 65 wt.%, less than or equal to about 60 wt.%, or less than or equal to about 50 wt.%).

The first mixture 1406 may include an amount of the one or more hydrocolloids 1403 ranging from about 0.01 wt.% to about 5 wt.%. For example, the first mixture 1406 may include greater than or equal to about 0.01 wt.% of the one or more hydrocolloids (e.g., greater than or equal to about 0.02 wt.%, greater than or equal to about 0.05 wt.%, greater than or equal to about 1.0 wt.%, greater than or equal to about 1.5 wt.%, greater than or equal to about 2.0 wt.%, greater than or equal to about 2.5 wt.%, greater than or equal to about 3.0 wt.%, greater than or equal to about 3.5 wt.%, greater than or equal to about 4.0 wt.%, greater than or equal to about 4.5 wt.%, or greater than or equal to about 4.9 wt.%). The first mixture 1406 may include less than or equal to about 5 wt.% of the one or more hydrocolloids (e.g., less than or equal to about 4.9 wt.%, less than or equal to about 4.5 wt.%, less than or equal to about 4.0 wt.%, less than or equal to about 3.5 wt.%, less than or equal to about 3.0 wt.%, less than or equal to about 2.5 wt.%, less than or equal to about 2.0 wt.%, less than or equal to about 1.5 wt.%, less than or equal to about 1.0 wt.%, or less than or equal to about 0.5 wt.%)

In at least one example embodiment, the method 1400 includes preparing S1405 the first mixture 1406. In some example embodiments, preparing S1405 the first mixture 1406 includes contacting one or more polyols 1402 and/or one or more hydrocolloids 1403 and an aqueous solvent 1404. For example, the one or more polyols 1402 and/or one or more hydrocolloids 1403 may be mixed with the aqueous solvent 1404 to form the first mixture 1406. Although not illustrated, in at least one example embodiment, the first mixture 1406 may further include one or more additives. The one or more additives may include, for example only, an antioxidant, sweetener, pH adjuster, polysaccharide, flavorant, or any combination thereof.

In at least one example embodiment, the method 1400 includes forming a cooled molten mixture 1428. In some example embodiments, forming the cooled molten mixture 1428 includes cooling S1420 the molten mixture 1412. For example, the molten mixture 1412 may be cooled to a second temperature to form the cooled molten mixture 1428.

In at least one example embodiment, the second temperature ranges from about 65 °C to about 200 °C. For example, the second temperature may be greater than or equal to about 65 °C (e.g., greater than or equal to about 70 °C, greater than or equal to about 75 °C, greater than or equal to about 80 °C, greater than or equal to about 85 °C, greater than or equal to about 90 °C, greater than or equal to about 95 °C, greater than or equal to about 100 °C, greater than or equal to about 105 °C, greater than or equal to about 110 °C, greater than or equal to about 115 °C, greater than or equal to about 120 °C, greater than or equal to about 125 °C, greater than or equal to about 130 °C, greater than or equal to about 135 °C, greater than or equal to about 140 °C, greater than or equal to about 145 °C, greater than or equal to about 150 °C, greater than or equal to about 155 °C, greater than or equal to about 160 °C, greater than or equal to about 165 °C, greater than or equal to about 170 °C, greater than or equal to about 175 °C, greater than or equal to about 180 °C, greater than or equal to about 185 °C, greater than or equal to about 190 °C, or greater than or equal to about 195 °C). The second temperature may be less than or equal to about 200 °C (e.g., less than or equal to about 195 °C, less than or equal to about 190 °C, less than or equal to about 185 °C, less than or equal to about 180 °C, less than or equal to about 175 °C, less than or equal to about 170 °C, less than or equal to about 165 °C, less than or equal to about 160 °C, less than or equal to about 155 °C, less than or equal to about 150 °C, less than or equal to about 145 °C, less than or equal to about 140 °C, less than or equal to about 135 °C, less than or equal to about 130 °C, less than or equal to about 125 °C, less than or equal to about 120 °C, less than or equal to about 115 °C, less than or equal to about 110 °C, less than or equal to about 105 °C, less than or equal to about 100 °C, less than or equal to about 95 °C, less than or equal to about 90 °C, less than or equal to about 85 °C, less than or equal to about 80 °C, less than or equal to about 75 °C, or less than or equal to about 70 °C).

In at least one example embodiment, the method 1400 includes forming a second mixture 1438. In some example embodiments, forming the second mixture 1438 includes adding S1430 one or more sweetener-containing materials 1432 to the cooled molten mixture 1428. The second mixture 1438 may include an amount ranging from about 0.01 wt.% to about 45 wt.% of the one or more sweetener-containing materials 1432. For example, the second mixture 1438 may include greater than or equal to about 0.01 wt.% of the one or more sweetener-containing materials 1432 (e.g., greater than or equal to about 5 wt.%, greater than or equal to about 10 wt.%, greater than or equal to about 15 wt.%, greater than or equal to about 20 wt.%, greater than or equal to about 25 wt.%, greater than or equal to about 30 wt.%, greater than or equal to about 35 wt.%, or greater than or equal to about 40 wt.%). The second mixture 1438 may include less than or equal to about 45 wt.% of the one or more sweetener-containing materials 1432 (e.g., less than or equal to about 40 wt.%, less than or equal to about 35 wt.%, less than or equal to about 30 wt.%, less than or equal to about 25 wt.%, less than or equal to about 20 wt.%, less than or equal to about 15 wt.%, less than or equal to about 10 wt.%, or less than or equal to about 5 wt.%).

Although not illustrated, in at least one embodiment, the second mixture 1438 may include one or more additives and forming the second mixture may include adding the one or more additives to the cooled molten mixture 1428. The one or more additives may include, for example only, an antioxidant, sweetener, pH adjuster, polysaccharide, flavorant, or any combination thereof.

In some example embodiments, the sweetener-containing materials 1432 include one or more high-intensity sweeteners. The one or more high-intensity sweeteners include may include a synthetic sweetener and/or a natural sweetener. In at least one example embodiment, the natural sweetener includes a sugar (such as a monosaccharide, a disaccharide, and/or a polysaccharide). In at least one example embodiment, the natural sweetener includes sucrose (e.g., table sugar), honey, a mixture of low-molecular-weight sugars excluding sucrose, glucose (e.g., grape sugar, corn sugar, dextrose), molasses, corn sweetener, glucose syrup (e.g., corn syrup), fructose (e.g., fruit sugar), lactose (e.g., milk sugar), maltose (e.g., malt sugar, maltobiose), sorghum syrup, fruit juice concentrate, or any combination thereof. In at one example embodiment, the sweetener-containing materials 1432 includes a sugar alcohol. The sugar alcohol may include mannitol, sorbitol, xylitol, maltitol, or any combination thereof. In at least one example embodiment, the sweetener-containing materials 1432 includes a non-nutritive sweetener including stevia, saccharin, aspartame, sucralose, acesulfame potassium, or any combination thereof.

In at least one example embodiment, the method 1400 includes forming one or more solidified structures 1448. In some example embodiments, forming the one or more solidified structures 1448 includes cooling S1440 the second mixture 1438. For example, the second mixture 1438 may be cooled to a third temperature to form the cooled molten mixture 1428. In some example embodiments, forming one or more solidified structures 1448 may include glassifying the second mixture 1438. For example, the third temperature may be below the glass transition temperature (T_{g}) of the one or more polyols 1402 and/or 1403.

In at least one example embodiment, the third temperature ranges from about 40 °C to about 45 °C. For example, the third temperature may be greater than or equal to about 40 °C (e.g., greater than or equal to about 40.5 °C, greater than or equal to about 41 °C, greater than or equal to about 41.5 °C, greater than or equal to about 42 °C, greater than or equal to about 42.5 °C, greater than or equal to about 43 °C, greater than or equal to about 43.5 °C, greater than or equal to about 44 °C, or greater than or equal to about 44.5 °C). The third temperature maybe less than or equal to about 45 °C (e.g., less than or equal to about 44.5 °C, less than or equal to about 44 °C, less than or equal to about 43.5 °C, less than or equal to about 43 °C, less than or equal to about 42.5 °C, less than or equal to about 42 °C, less than or equal to about 41.5 °C, less than or equal to about 41 °C, or less than or equal to about 40.5 °C).

The one or more solidified structures 1448 may include one or more sheets, or one of a variety of other configurations such as would be recognized by of ordinary skill in the art. For example, in at least one example embodiment, the one or more solidified structures 1448 may include one or more brittle sheets at room temperature.

In at least one example embodiment, the method 1400 includes fragmenting S1450 the one or more solidified structures 1448 to form the sweetener-containing powder 1452.

### LIQUID MIXTURES OF A TRIGLYCERIDE AND LIQUID NICOTINE

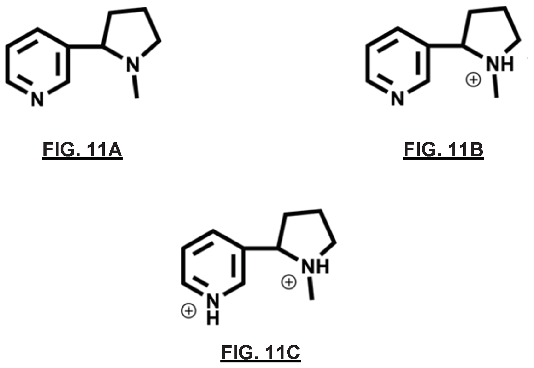

FIGS. 11A-11C depict chemical structures of nicotine in different forms.

Nicotine, or 3-(l-methyl-2-pyrrolidinyl) pyridine, is a tertiary amine. Under ambient conditions, nicotine is an oily, volatile, hygroscopic liquid. In this state, nicotine is a free base (non-protonated) and has the structure as shown in FIG. 11A. Free-base nicotine has a pKₐ value of about 8.

Nicotine may also be in a form of a complex or a salt. Nicotine complexes and salts may be provided in solid form, such as a powder. One example of a nicotine complex that is used in oral products is nicotine polacrilex. In a salt, nicotine is mono-protonated, as shown in FIG. 11B, or di-protonated, as shown in FIG. 11C. Mono-protonated and di-protonated nicotine have lower pKₐ values than free-base nicotine. Nicotine salts may include nitrate, monotartrate, bitartrate, bitartrate dihydrate, salicylate, sulfate or bisulfate, phosphate or acid phosphate, acetate, lactate, succinate, maleate, fumarate, gluconate, saccharate, benzoate, methanesulfonate, hydrochloride, hydrobromide, hydroiodide, or any combination thereof.

Oral products often include nicotine in the form of a complex or salt for reasons related to manufacturing, handling, and stability. However, nicotine is believed to more readily absorb in the buccal mucosa at higher pKₐ values. Accordingly, oral products including nicotine in a protonated state may also include a pH adjuster so as to create a more basic environment in the oral product.

In at least one example embodiment, an oral product includes a liquid mixture of nicotine and a triglyceride. An oral product including the liquid mixture may be configured to have increased buccal nicotine absorption compared to oral products including aqueous nicotine, nicotine complexes, and/or nicotine salts. The nicotine may be liquid nicotine. At least a portion of the liquid nicotine may be dissolved in the triglyceride to form a solution of the triglyceride and the liquid nicotine. In at least one example embodiment, all of the liquid nicotine is dissolved in the triglyceride. In at least one example embodiment, the liquid mixture may consist essentially of liquid nicotine and a triglyceride, such as a medium-chain triglyceride (MCT).

A weight ratio of the triglyceride to the nicotine in the liquid mixture may be greater than or equal to about 1:1 (e.g., greater than or equal to about 3:2, greater than or equal to about 2:1, greater than or equal to about 3:1, greater than or equal to about 4:1, greater than or equal to about 5:1, greater than or equal to about 6:1, greater than or equal to about 7:1, greater than or equal to about 8:1, greater than or equal to about 9:1, or greater than or equal to about 10:1). The weight ratio may be less than or equal to about 95:5 (e.g., less than or equal to about 9:1, less than or equal to about 8:1, less than or equal to about 7:1, less than or equal to about 6:1, less than or equal to about 5:1, less than or equal to about 4:1, less than or equal to about 3:1, or less than or equal to about 3:2). In at least one example embodiment, the weight ratio ranges from about 1:1 to about 9:1 (e.g., from about 3:2 to about 4:1, from about 3:1 to about 5:1, or about 4:1).

In at least one example embodiment, the oral product may include additional triglyceride beyond what is present in the liquid mixture. The additional triglyceride may, in at least one example embodiment, be used as a plasticizer. A ratio of the triglyceride to the liquid nicotine in the oral product (i.e., both the liquid mixture and any additional triglyceride) may be greater than or equal to about 1:1 (e.g., greater than or equal to about 3:2, greater than or equal to about 2:1, greater than or equal to about 3:1, greater than or equal to about 4:1, greater than or equal to about 5:1, greater than or equal to about 6:1, greater than or equal to about 7:1, greater than or equal to about 8:1, greater than or equal to about 9:1, greater than or equal to about 10:1, greater than or equal to about 15:1, greater than or equal to about 20:1, greater than or equal to about 25:1, greater than or equal to about 30:1, greater than or equal to about 40:1, greater than or equal to about 50:1, greater than or equal to about 60:1, greater than or equal to about 70:1, greater than or equal to about 75:1, or greater than or equal to about 80:1). The weight ratio of the triglyceride to the liquid nicotine may be less than or equal to about 100:1 (e.g., less than or equal to about 90:1, less than or equal to about 80:1, less than or equal to about 75:1, less than or equal to about 70:1, less than or equal to about 60:1, less than or equal to about 50:1, less than or equal to about 40:1, less than or equal to about 30:1, less than or equal to about 25:1, less than or equal to about 20:1, less than or equal to about 15:1, less than or equal to about 10:1, less than or equal to about 9:1, less than or equal to about 8:1, less than or equal to about 7:1, less than or equal to about 6:1, less than or equal to about 5:1, less than or equal to about 4:1, less than or equal to about 3:1, less than or equal to about 2:1, or less than or equal to about 3:2).

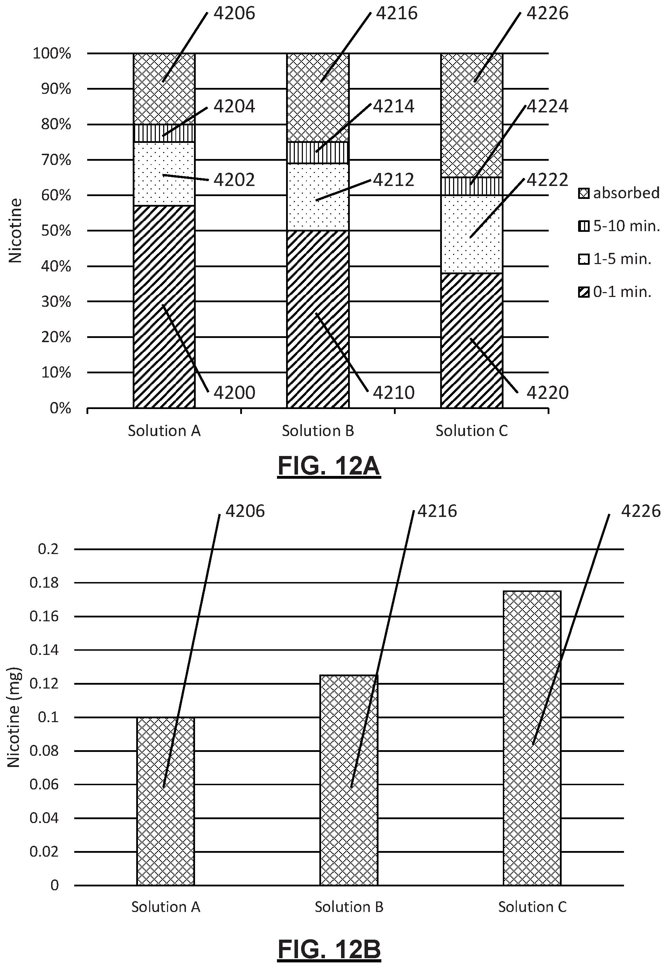

FIGS. 12A-12B are a graphs depicting buccal nicotine disposition for different nicotine solutions.

In at least one example embodiment, providing the nicotine dissolved in a triglyceride may facilitate increased buccal absorption compared to nicotine in an aqueous phase. It is believed that providing the nicotine dissolved in the triglyceride facilitates retention of at least a portion of the nicotine in its free-base state, regardless of the presence of a pH adjuster.

As shown in FIGS. 12A-12B, three solutions are prepared according to Table 5, below. Amounts in Table 5 are by weight percent. Solution A includes 0.5 mg liquid nicotine in water having a pH of 7. Solution B includes 0.5 mg nicotine in water having a pH of 10. Solution C includes 0.5 mg nicotine in MCT oil.

**TABLE 5. Solution Compositions by Weight Percent**

| | **Solution A** | **Solution B** | **Solution C** |
|---|---|---|---|
| Nicotine | 0.050% | 0.050% | 0.050% |
| Propylene Glycol | 0.200% | 0.200% | -- |
| MCT | -- | -- | 99.950% |
| Water | 99.732% | 99.746% | --- |
| Citric Acid | 0.018% | --- | --- |
| Sodium Carbonate | --- | 0.004% | --- |

To determine the amount of nicotine absorbed, versus the amount not absorbed, each of Solutions A, B, and C is held in an oral cavity of an adult tobacco consumer. Five adult tobacco consumers participate. Expectorant samples are collected for each of the five adult tobacco consumers for each of the three solutions at three time intervals: 0-1 minute, 1-5 minutes, and 5-10 minutes. Each expectorant sample is analyzed to measure a weight of nicotine in the expectorant sample. The nicotine measured in the expectorant samples is necessarily not absorbed in the buccal mucosa. An amount of absorbed nicotine is calculated for each solution based on a difference between a known weight of nicotine in each solution and the measured amounts of nicotine in each of the expectorant samples for each solution, as described in greater detail below.

Solution A includes 0.5 mg of nicotine. A first solution A expectorant sample 4200 is collected at 0-1 minute, and includes 57 weight percent of the 0.5 mg of nicotine. A second solution A expectorant sample 4202 is collected at 1-5 minutes, and includes 18 weight percent of the 0.5 mg nicotine. A third solution A expectorant sample 4204 is collected at 5-10 minutes, and includes 5 weight percent of the 0.5 mg of nicotine. Accordingly, a solution A absorbed nicotine 4206 is calculated to be 20 weight percent (100%-57%-18%-5%=20%).

Solution B includes 0.5 mg of nicotine. A first solution B expectorant sample 4210 is collected at 0-1 minute, and includes 50 weight percent of the 0.5 mg of nicotine. A second solution B expectorant sample 4212 is collected at 1-5 minutes, and includes 19 weight percent of the 0.5 mg nicotine. A third solution B expectorant sample 4214 is collected at 5-10 minutes, and includes 6 weight percent of the 0.5 mg of nicotine. Accordingly, a solution B absorbed nicotine 4216 is calculated to be 25 weight percent (100%-50%-19%-6%=25%).

Solution C includes 0.5 mg of nicotine. A first solution C expectorant sample 4220 is collected at 0-1 minute, and includes 38 weight percent of the 0.5 mg of nicotine. A second solution C expectorant sample 4222 is collected at 1-5 minutes, and includes 22 weight percent of the 0.5 mg nicotine. A third solution C expectorant sample 4224 is collected at 5-10 minutes, and includes 5 weight percent of the 0.5 mg of nicotine. Accordingly, a solution C absorbed nicotine 4226 is calculated to be 35 weight percent (100%-38%-22%-5%=35%).

As shown by the differences in behavior of Solution A and Solution B, increasing a pH of an aqueous solution, as in Solution B, facilitates increased buccal nicotine absorption. For example, the increased pH of the aqueous environment may facilitate retention of nicotine in the free-base phase. As shown by the differences in behavior of Solutions A/B and Solution C, providing the nicotine in the MCT facilitates, as in Solution C, increased buccal nicotine absorption compared to providing nicotine in an aqueous phase. Moreover, as shown by the differences in behavior of Solution B and Solution C, providing the nicotine in the MCT facilitates, as in Solution C, increased buccal absorption compared to an aqueous phase including a pH adjuster. As shown in FIG. 12B, the amount of absorbed nicotine for Solution C is higher than the amount absorbed for each of Solutions A and B.

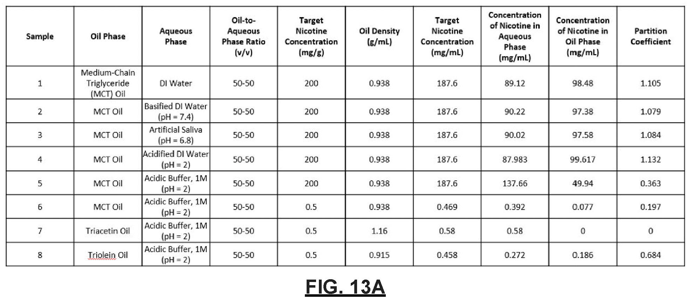

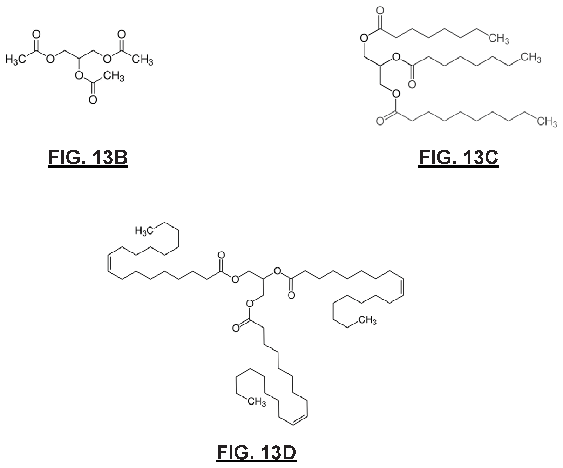

FIG. 13A is a table depicting partition coefficient data for nicotine in different oil and water phase combinations. FIG. 13B depicts of a chemical structure of triacetin (C2). FIG. 13C depicts a chemical structure of MCT (C8-C10). FIG. 13D depicts a chemical structure of triolein (C18).

As shown in FIG. 13A, nicotine has solubility in both oil and water. Each of Samples 1-8 is prepared by and measured according to the following method. Liquid nicotine is added at the target nicotine concentration to a vessel containing an oil phase. The oil phase containing nicotine is mixed with an aqueous phase at a one-to-one volume ratio (v:v) using an orbital shaker at about 100 rotations per minute (rpm) for about 5 minutes to prepare a nicotine oil : aqueous phase mix. The nicotine oil : aqueous phase mix is allowed to sit at room temperature (about 25°C) for about 24 hours. A concentration of nicotine in each of the oil phase and the nicotine phase is measured using a liquid chromatography method. A partition coefficient, which is a ratio concentrations of a compound (i.e., nicotine) in two immiscible solvents (i.e., an oil phase and an aqueous phase) at equilibrium, is calculated for each sample. The partition coefficient is a comparison of the solubilities of the nicotine in the two liquid phases.

Samples 1-6 include MCT oil (CAS No. 73398-61-5) as an oil phase. Sample 7 includes triacetin oil as an oil phase. Sample 8 includes triolein oil as an oil phase. Sample 1 includes deionized (DI) water as an aqueous phase. Sample 2 includes basified DI water having a pH of 7.4 as an aqueous phase. Sample 3 includes artificial saliva having a pH of 6.8 as an aqueous phase. Artificial saliva simulates mucus conditions. Sample 4 includes acidified DI water having a pH of 2 as an aqueous phase. Samples 5-8 include 1M acetic acid pH adjuster having a pH of 2 as an aqueous phase, which simulates stomach conditions.

In each of Samples 5-8, a partition coefficient is less than 1, indicating a higher solubility of nicotine in the acetic acid pH adjuster than the respective oil phase. This is believed to be caused by protonation of the nicotine and higher electrostatic interactions. In each of Samples 1-4, a partition coefficient is greater than 1, indicating a higher solubility in the MCT oil than the respective aqueous phase. However, in every sample, at least a portion of the nicotine is dissolved in the aqueous phase. Accordingly, if water is present in a liquid mixture, at least a portion of the nicotine will be dissolved in the water phase.

In at least one example embodiment, the oral product includes water. The oral product may include water in an amount less than or equal to about 8 weight percent (e.g., less than or equal to about 5 weight percent, less than or equal to about 3 weight percent, less than or equal to about 2 weight percent, less than or equal to about 1 weight percent, less than or equal to about 0.5 weight percent. In at least one example embodiment, the oral product is free of water.

In at least one example embodiment, the liquid mixture includes water in amount less than or equal to about 5 weight percent (e.g., less than or equal to about 4 weight percent, less than or equal to about 3 weight percent, less than or equal to about 2 weight percent, less than or equal to about 1 weight percent, less than or equal to about 0.5 weight percent, or less than or equal to about 0.25 weight percent). In at least one example embodiment, no water is intentionally added to the liquid mixture. However, a small amount of water may come into contact with the liquid mixture via other elements in the oral product. In at least one example embodiment, the liquid mixture is free of water. Limiting an amount of water and/or omitting water may facilitate increasing an amount of nicotine dissolved in the triglyceride. Accordingly, an oral product having the liquid mixture may be configured to facilitate increased buccal absorption of nicotine compared to an oral product having all or a portion of the nicotine in a water phase.

As noted above, in at least one example embodiment, the nicotine may be liquid nicotine. Greater than or equal to about 50 weight percent of the nicotine may be free base nicotine (e.g., greater than or equal to about 55 weight percent, greater than or equal to about 60 weight percent, greater than or equal to about 65 weight percent, greater than or equal to about 70 weight percent, greater than or equal to about 75 weight percent, greater than or equal to about 80 weight percent, greater than or equal to about 85 weight percent, greater than or equal to about 90 weight percent, greater than or equal to about 95 weight percent, greater than or equal to about 98 weight percent, greater than or equal to about 99 weight percent). In at least one example embodiment, greater than 80 weight percent of the nicotine is free-base nicotine. In at least one example embodiment, all of the nicotine is free-base nicotine.

In at least one example embodiment, the nicotine is tobacco-derived nicotine, synthetic nicotine, or both tobacco-derived nicotine and synthetic nicotine. In at least one example embodiment, the oral product includes the nicotine in an amount greater than or equal to about 0.1 mg (e.g., greater than or equal to about 1 mg, greater than or equal to about 2 mg, greater than or equal to about 4 mg, greater than or equal to about 6 mg, greater than or equal to about 8 mg, greater than or equal to about 10 mg, greater than or equal to about 12 mg). The oral product may include the nicotine in an amount less than or equal to about 14 mg (e.g., less than or equal to about 12 mg, less than or equal to about 10 mg, less than or equal to about 8 mg, less than or equal to about 6 mg, less than or equal to about 4 mg, less than or equal to about 2 mg, or less than or equal to about 1 mg). In at least one example embodiment, the oral product includes the liquid nicotine in an amount ranging from 0.1 mg to about 14 mg (e.g., ranging from about 4 mg to about 8 mg, ranging from about 8 mg to about 6 mg).

The triglyceride may include a long-chain triglyceride (LCT), MCT, a short-chain triglyceride (STC), or any combination thereof. In at least one example embodiment, the triglyceride includes MCT. In at least one example embodiment, the liquid mixture further include triacetin, triolein, trilinolein, vegetable oil, a partially-hydrogenated oil, or any combination thereof. In at least one example embodiment, the liquid mixture includes triacetin, triolein, trilinolein, vegetable oil, a partially-hydrogenated oil, or any combination thereof as an alternative to the triglyceride at the same weight ratios and percentages described herein with respect to the triglyceride.

In at least one example embodiment, the oral product includes the triglyceride in an amount greater than or equal to about 1 weight percent (e.g., greater than or equal to about 2 weight percent, greater than or equal to about 3 weight percent, greater than or equal to about 4 weight percent, greater than or equal to about 5 weight percent, greater than or equal to about 10 weight percent, greater than or equal to about 15 weight percent, greater than or equal to about 20 weight percent, greater than or equal to about 25 weight percent, greater than or equal to about 30 weight percent, greater than or equal to about 40 weight percent, greater than or equal to about 50 weight percent, greater than or equal to about 60 weight percent, greater than or equal to about 70 weight percent, greater than or equal to about 80 weight percent, or greater than or equal to about 90 weight percent). In at least one example embodiment, the oral product may include the triglyceride in an amount less than or equal to about 95 weight percent (e.g., less than or equal to about 90 weight percent, less than or equal to about 80 weight percent, less than or equal to about 70 weight percent, less than or equal to about 60 weight percent, less than or equal to about 50 weight percent, less than or equal to about 40 weight percent, less than or equal to about 30 weight percent, less than or equal to about 20 weight percent, less than or equal to about 15 weight percent, less than or equal to about 10 weight percent, less than or equal to about 7 weight percent, or less than or equal to about 5 weight percent). In at least one example embodiment, the oral product is a solid format including the triglyceride at less than or equal to about 50 weight percent. In at least one example embodiment, the oral product is a gel, paste, or liquid format including the triglyceride at greater than or equal to about 75 weight percent (e.g., greater than or equal to about 80 weight percent of the triglyceride, greater than or equal to about 85 weight percent of the triglyceride, greater than or equal to about 90 weight percent of the triglyceride, greater than or equal to about 95 weight percent of the triglyceride).

In at least one example embodiment, the oral product further includes a carrier. At least a portion of the liquid mixture may be absorbed in the carrier. In at least one example embodiment, all of the liquid mixture is absorbed in the carrier. In at least one example embodiment, a weight ratio of the carrier to the liquid mixture may be in a range of about greater than or equal to about 75:25 (e.g., greater than or equal to about 80:20, greater than or equal to about 85:15, greater than or equal to about 90:10, or greater than or equal to about 95:5). In at least one other example embodiment, the oral product is free of a carrier.

In at least one example embodiment, the carrier may be a mouth-insoluble material. In at least one example embodiment, the carrier is a cellulosic material. The cellulosic material may include or be derived from sugar beets, wood pulp, cotton, bran, citrus pulp, grass (e.g., switch grass), willow, poplar, or any combination thereof. The insoluble cellulosic material may be a treated cellulosic material, such as microcrystalline cellulose (MCC), carboxymethyl cellulose (CMC), hydroxypropyl methylcellulose (HPMC), hydroxypropyl cellulose (HPC), or any combination thereof. In at least one example embodiment, the cellulosic material includes mouth-insoluble cellulosic fibers.

In at least one example embodiment, the oral product is free of a pH adjuster. In at least one other example embodiment, the oral product includes a pH adjuster. The pH adjuster may include ammonium carbonate, ammonium bicarbonate, ammonium hydroxide, calcium carbonate, potassium carbonate, potassium bicarbonate, potassium hydroxide, sodium carbonate, sodium bicarbonate, sodium hydroxide, or any combination thereof. The pH adjuster may be included in an amount greater than or equal to about 0.01 weight percent (e.g., greater than or equal to about 0.05 weight percent, greater than or equal to about 0.1 weight percent, greater than or equal to about 0.5 weight percent, or greater than or equal to about 1 weight percent). The pH adjuster may be included in an amount less than or equal to about 2 weight percent (e.g., less than or equal to about 1 weight percent, less than or equal to about 0.5 weight percent, less than or equal to about 0.1 weight percent, or less than or equal to about 0.05 weight percent). In at least one example embodiment, the pH adjuster is present in an amount ranging from about 0.01 weight percent to 2 weight percent.

In at least one example embodiment, the oral product includes the liquid mixture, optionally absorbed in a carrier, and additional elements. The additional elements may include a mouth-soluble polymer, a mouth-stable polymer, a plasticizer, a flavorant, a sweetener, mouth-soluble fibers, an antioxidant, an energizing agent, a soothing agent (e.g., theanine and/or melatonin), a focusing agent (e.g., gingko biloba), an alkaloid, a mineral, a vitamin, a dietary supplement, a nutraceutical, a coloring agent, an amino acid, a chemesthetic agent, a food-grade emulsifier, a botanical (e.g., green tea), a tooth-whitening agent (e.g., sodium hexametaphosphate (SHMP)), a therapeutic agent, a processing aid, a stearate (e.g., magnesium and/or potassium), a wax (e.g., glycerol monostearate, propylene glycol monostearate, and/or an acetylated monoglyceride), a stabilizer (e.g., ascorbic acid and monosterol citrate, butylated hydroxytoluene (BHT), or butylated hydroxyanisole (BHA)), a lubricant (e.g., sodium lauryl sulfate (SLS)), a preservative (e.g., sodium benzoate), a filler, or any combination thereof. The oral product may include multiple additional elements. Additionally, a single element may belong to more than one of the categories above.

In at least one example embodiment, the additional elements may be included in an amount greater than or equal to about 0.5 weight percent (e.g., greater than or equal to about 1 weight percent, greater than or equal to about 2 weight percent, greater than or equal to about 5 weight percent, greater than or equal to about 10 weight percent, greater than or equal to about 15 weight percent, greater than or equal to about 20 weight percent, greater than or equal to about 25 weight percent, greater than or equal to about 30 weight percent, greater than or equal to about 35 weight percent, greater than or equal to about 40 weight percent, greater than or equal to about 45 weight percent, greater than or equal to about 50 weight percent, greater than or equal to about 55 weight percent, greater than or equal to about 60 weight percent, greater than or equal to about 65 weight percent, greater than or equal to about 70 weight percent, greater than or equal to about 75 weight percent, greater than or equal to about 80 weight percent, greater than or equal to about 85 weight percent, or greater than or equal to about 90 weight percent). The additional elements may be present in an amount less than or equal to about 95 weight percent (e.g., less than or equal to about 90 weight percent, less than or equal to about 85 weight percent, less than or equal to about 80 weight percent, less than or equal to about 75 weight percent, less than or equal to about 70 weight percent, less than or equal to about 65 weight percent, less than or equal to about 60 weight percent, less than or equal to about 55 weight percent, less than or equal to about 50 weight percent, less than or equal to about 45 weight percent, less than or equal to about 40 weight percent, less than or equal to about 35 weight percent, less than or equal to about 30 weight percent, less than or equal to about 25 weight percent, less than or equal to about 20 weight percent, less than or equal to about 15 weight percent, less than or equal to about 10 weight percent, or less than or equal to about 5 weight percent).

In at least one example embodiment, the oral product includes a mouth-soluble polymer. As used herein, "mouth-soluble" means that the polymer experiences significant degradation when exposed to saliva within an oral cavity over a period of about four hours. In at least one example embodiment, the mouth-soluble polymer disintegrates when exposed to saliva at the normal human body temperature for a period of less than or equal to about an hour (e.g., less than or equal to about 30 minutes, less than or equal to about 15 minutes, less than or equal to about 10 minutes, or less than or equal to about 5 minutes). The mouth-soluble polymer may be biocompatible.

In at least one example embodiment, the mouth-soluble polymer may include a cellulosic polymer, such as carboxymethyl cellulose (CMC), hydroxypropyl cellulose (HPC), hydroxyethyl cellulose (HEC), hydroxypropyl methyl cellulose (HPMC), and/or methyl cellulose (MC); a natural polymer, such as a starch, a modified starch, konjac, collagen, inulin, soy protein, whey protein, casein, and/or wheat gluten; a seaweed-derived polymer, such as a carrageenan (e.g., kappa carrageenan, iota carrageenan, lambda carrageenan), an alginate (e.g., propylene glycol alginate); a microbial-derived polymer, such as xanthan, dextran, pullulan, curdlan, and/or gellan; an extract, such as locust bean gum, guar gum, tara gum, gum tragacanth, pectin (e.g., low methoxy and amidated), agar, zein, karaya, gelatin, psyllium seed, chitin, and/or chitosan, an exudates, such as gum acacia (arabic) and/or shellac; a synthetic polymer, such as polyvinyl pyrrolidone, polyethylene oxide, and/or polyvinyl alcohol, or any combination thereof. Other useful mouth-soluble polymers are known in the art, for example, see Krochta et al. Food Technology, 1997, 51:61-74, Glicksman Food Hydrocolloids CRC 1982, Krochta Edible Coatings and Films to Improve Food Quality Technomic 1994, Industrial Gums Academic 1993, Nussinovitch Water-Soluble Polymer Applications in Foods Blackwell Science 2003, the entire contents of which are incorporated herein by reference.

In at least one example, the oral product includes a mouth-stable polymer. As used herein, "mouth-stable" means that the polymer does not appreciably dissolve or disintegrate when exposed to saliva at the normal human body temperature (i.e., 98.6°F) over a period of about one hour. In at least one example embodiment, the mouth-stable polymer is a biodegradable polymer that is configured to break down over a period of days, weeks, months, or years, but does not appreciably break down when held in an oral cavity and exposed to saliva for a period of about one hour. In at least one example embodiment, the mouth-stable polymer is stable within an oral cavity and exposed to saliva at the normal human body temperature for a period of greater than or equal to about 2 hours (e.g., greater than or equal to about 6 hours, greater than or equal to about 12 hours, greater than or equal to about 1 day, or greater than or equal to about 2 days). Accordingly, an oral product including a mouth-stable polymer according to at least one example embodiment is configured to remain intact when placed in an adult consumer's mouth. After a period of time, the mouth-stable polymer and any other mouth-stable elements may be removed from the adult consumer's mouth and discarded.

The mouth-stable polymer may be biocompatible and biostable. The mouth-stable polymer may generally be recognized as safe and in compliance with applicable food-contact regulations by an appropriate regulatory agency (e.g., the U.S. Food and Drug Administration (FDA)). In at least one example embodiment, the mouth-stable polymer has a flexural modulus of greater than or equal to 5 MPa when tested according to ASTM Testing Method D790 or ISO 178 at 23°C, or optionally greater than or equal to 10 MPa.

In at least one example embodiment, the mouth-stable polymer includes a polyurethane, a silicone, a polyester, a polyacrylate, a polyethylene, a polypropylene, a polyetheramide, a polystyrene, a polyvinyl alcohol, a polyvinyl acetate, a polyvinyl chloride, a polybutyl acetate, a butyl rubber, poly(styrene-ethylene-butylene-styrene) (SEBS), poly(styrene-butadiene-styrene) (SBS), poly(styrene-isoprene-styrene) (SIS), any copolymer thereof, or any combination thereof. In at least one example embodiment, the mouth-stable polymer includes polyurethane. In at least one example embodiment, the mouth-stable polymer includes a food-grade or medical-grade polymer, such as medical-grade polyurethane.

In at least one example embodiment, the mouth-stable polymer is a thermoplastic polymer. The thermoplastic polymer may be a thermoplastic elastomer. In at least one example embodiment, the mouth-stable polymer includes a thermoplastic elastomer meeting the requirements of the FDA-modified ISO 10993, Part 1 "Biological Evaluation of Medical Devices" tests with human tissue contact time of 30 days or less. The mouth-stable polymer may have a shore Hardness of 50D or softer, a melt flow index of 3g/10 min at 200°C/10 kg, a tensile strength of greater than or equal to 10 MPa (using ISO 37), and/or a ultimate elongation of less than 100% (using ISO 37).

In at least one example embodiment, the oral product includes a plasticizer. The plasticizer may include a triglyceride (e.g., long, medium, and/or short chain), triacetin, propylene glycol, glycerin, vegetable oil, a phthalate, an ester of a polycarboxylic acid with a linear or branched aliphatic alcohol of moderate chain length, or any combination thereof. The plasticizer may be present in addition to triglycerides and/or other oils in the liquid mixture.

In at least one example embodiment, the oral product includes a flavorant. The flavorant may be natural or artificial. In at least one example embodiment, the flavorant includes a fruit flavorant (e.g., bergamot, berry, cherry, lemon, and/or orange), a liquor or liqueur flavorant (e.g., bourbon, cognac, scotch, whiskey, and/or DRAMBUIE brand liqueur), a mint flavorant (e.g., Japanese mint, menthol, peppermint, spearmint, wintergreen, and/or mint oils from a species of the genus Mentha), a floral flavorant (e.g., geranium, lavender, and/or rose), a spice, an herb, or another botanical or botanical-derived flavorant (e.g., anise, apium graveolens, caraway, cardamom, cascarilla, cassia, cinnamon, chamomile, clove, cocoa, coffee, coriander, fennel, ginger, jasmine, licorice, nutmeg, pimenta, sage, sandalwood vanilla, and/or ylang-ylang), honey essence, or any combination thereof. In at least one example embodiment, the flavorant includes bergamot, berry, cherry, lemon, orange, bourbon, cognac, scotch, whiskey, DRAMBUIE brand liqueur, Japanese mint, menthol, peppermint, spearmint, wintergreen, mint oils from a species of the genus Mentha, geranium, lavender, rose, anise, apium graveolens, caraway, cardamom, cascarilla, cassia, cinnamon, chamomile, clove, coffee, coriander, fennel, ginger, jasmine, licorice, nutmeg, pimenta, sage, sandalwood vanilla, ylang-ylang, honey essence, or any combination thereof. In at least one example embodiment, the oral product includes an encapsulated flavorant.

In at least one example embodiment, the oral product includes a sweetener. The sweetener may include a synthetic sweetener and/or a natural sweetener. The natural sweetener may include a sugar, such as a monosaccharide, a disaccharide, and/or a polysaccharide. In at least one example embodiment, the sweetener includes a natural sweetener including sucrose (i.e., table sugar), honey, a mixture of low-molecular-weight sugars excluding sucrose, glucose (i.e., grape sugar, corn sugar, dextrose), molasses, corn sweetener, glucose syrup (i.e., corn syrup), fructose (i.e., fruit sugar), lactose (i.e., milk sugar), maltose (i.e., malt sugar, maltobiose), sorghum syrup, fruit juice concentrate, or any combination thereof. In at one example embodiment, the sweetener includes a sugar alcohol. The sugar alcohol may include ethylene glycol, glycerol, erythritol, threitol, arabitol, xylitol, ribitol, mannitol, sorbitol, galactitol, fucitol, iditol, inositol, volemitol, isomalt, maltitol, lactitol, maltotriitol, maltotetraitol, polyglycitol, or any combination thereof. In at least one example embodiment, the sweetener includes a non-nutritive sweetener including stevia, saccharin, aspartame, sucralose, acesulfame potassium, or any combination thereof.

In at least one example embodiment, the oral product includes mouth-soluble fibers. The mouth-soluble fibers may be configured to dissolve when exposed to saliva in an adult tobacco consumer's mouth at the normal human body temperature. In at least one example embodiment, the mouth-soluble fibers include maltodextrin, psyllium, starch, or any combination thereof. In at least one example embodiment, the mouth-soluble fibers include soluble dietary fibers. In at least one example embodiment oral product includes partially-soluble fibers, such as sugar beet fibers.

In at least one example embodiment, the oral product may include the energizing agent. In at least one example embodiment, the energizing agent includes caffeine, taurine, glucaronalactone, or any combination thereof. Caffeine may include synthetic caffeine and/or natural caffeine, such as coffee-bean-extracted caffeine. In at least one example embodiment, the oral product includes caffeine in an amount greater than or equal to about 10 mg (e.g., greater than or equal to about 25 mg, greater than or equal to about 50 mg, greater than or equal to about 75 mg, greater than or equal to about 100 mg, greater than or equal to about 150 mg) The caffeine may be included in an amount less than or equal to about 200 mg (e.g., less than or equal to about 150 mg, less than or equal to about 100 mg, less than or equal to about 75 mg, less than or equal to about 50 mg, or less than or equal to about 25 mg).

In at least one example embodiment, the oral product may include the coloring agent. The coloring agent may be a natural colorant and/or an artificial colorant. In at least one example embodiment, the oral product is free of a colorant.

In at least one example embodiment, the oral product includes a filler. The filler may be configured to alter a texture or pliability of the oral product compared to an oral without the filler. The filler may include mouth-soluble elements, mouth-insoluble elements, or both mouth-soluble and mouth-insoluble elements. Mouth-soluble elements may be configured to dissolve or disintegrate when in an adult consumer's mouth so as to render the oral product more pliable. Fillers may include dicalcium phosphate, calcium sulfate, a clay, silica, glass particles, glyceryl palmitostearate, sodium stearyl fumarate, talc, or any combination thereof. Additionally, certain elements described above may also be classified as fillers, such as mouth-soluble fibers, sweeteners, minerals, or any combination thereof. In at least one example embodiment, cellulosic materials may be present in the oral product as fillers in addition to or as an alternative to being carriers for the liquid mixture.

In at least one example embodiment, the oral product is a chewing gum, a spray, a lozenge, a dissolvable tablet, a non-dissolvable chew, a film, a gel, a capsule, or a pouch (e.g., containing fibers or granules). In at least one example embodiment, the oral product directly includes the liquid mixture. In at least one example embodiment, the oral product includes the liquid mixture absorbed on a carrier (e.g., a cellulosic material such as MCC).

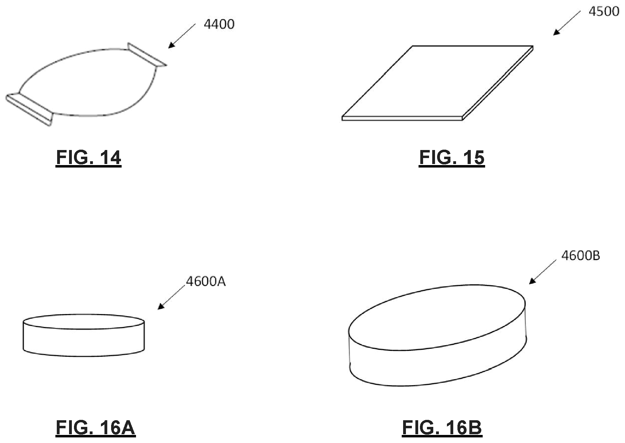

FIG. 14 is a perspective view of a pouched product according to at least one example embodiment.

In at least one example embodiment, as shown in FIG. 14, the oral product may be a pouched product 4400. The pouched product may include the liquid mixture absorbed on a carrier, such as MCC, and contained in a pouch. The pouch may be formed from a permeable fabric. The pouched product 4400 may be a pouched product as described in any of U.S. Patent No. 9,066,540, issued June 30, 2015; U.S. Patent No. 8,978,661, issued March 17, 2015; U.S. Patent No. 10,039,309, issued August 7, 2018; U.S. Patent No. 9,414,624, issued August 16, 2016; U.S. Patent No. 9,693,582, issued July 4, 2017; or U.S. Patent No. 9,462,827, issued October 11, 2016, the entire contents of each of which are incorporated herein by this reference hereto, and further including a liquid mixture as described above as an alternative or in addition to tobacco and/or nicotine.

In at least one example embodiment, the oral product is a mouth spray. The mouth spray includes the liquid mixture. The mouth spray may further include a liquid carrier. The liquid carrier may include water, propylene glycol, glycerin, ethanol, or any combination thereof.

FIG. 15 is a perspective view of a dissolvable film according to at least one example embodiment.

In at least one example embodiment, the oral product may be a dissolvable film 4500. The dissolvable film 4500 may be a film as described in U.S. Patent No. 8,469,036, issued June 25, 2013, the entire contents of which is incorporated herein by this reference hereto, and further including a liquid mixture as described above as an alternative or in addition to tobacco.

In at least one example embodiment, the oral product includes a chewing gum. The chewing gum includes a gum base and the liquid mixture. In at least one example embodiment, at least a portion of the liquid mixture is absorbed in a carrier, such as MCC.

In at least one example embodiment, the gum base includes an elastomer, a resin, a wax, a fat, an emulsifier, a filler, an antioxidant, or any combination thereof. The elastomer may include couma macrocarpa, loquat, tunu, jelutong, chicle, styrene-butadiene rubber, butyl rubber, polyisobutylene, or any combination thereof, by way of example. The resin may include glycerol esters of gum, terpene resins, polyvinyl acetate, or any combination thereof, by way of example. The wax may include paraffin, microcrystalline wax, or both paraffin and microcrystalline wax, by way of example. The fat may include a hydrogenated vegetable oil, by way of example. The emulsifier may include lecithin, glycerol monosterate, or a combination of lecithin and glycerol monostearate, by way of example. The filler may include calcium carbonate, talc, or both calcium carbonate and talc, by way of example. The antioxidant may include butylated hydroxytoluene (BHT), butylated hydroxyanisole (BHA), tocopherol, ascorbyl palmitate, or any combination thereof, by way of example. In at least one example embodiment, the gum base includes a natural latex, a vegetable gum (e.g., chicle), a spruce gum, a mastic gum, or any combination thereof.

In at least one example embodiment, the oral product is a lozenge. The lozenge may include a solid solution and the liquid mixture. The solid solution may include soluble fibers and a sugar alcohol, such as those described above. In at least one example embodiment, the solid solution includes isomalt. In at least one example embodiment, the liquid mixture is absorbed in a carrier (e.g., MCC). In at least one example embodiment, the lozenge directly includes the liquid mixture without a carrier. In at least one example embodiment, the lozenge may be a lozenge as described in U.S. Patent No. 9,351,936, issued May 31, 2016, the entire contents of which is incorporated herein by this reference thereto, and further including the liquid mixture as described above in addition to or as an alternative in addition to nicotine.

In at least one example embodiment, the oral product is a dissolvable tablet. The dissolvable tablet may include a mouth-soluble polymer, such as those described above, and the liquid mixture optionally absorbed in a carrier. In at least one example embodiment, the dissolvable tablet may be a tablet (or tab) as described in U.S. Application Serial No. 14/505,814, filed October 3, 2014; U.S. Patent No. 9,930,909, issued April 3, 2018; or U.S. Patent No. 8,469,036, issued June 25, 2013, the entire contents of each of which are incorporated herein by this reference thereto, and further including the liquid mixture as described above as an alternative or in addition to tobacco.

In at least one example embodiment, the oral product is non-dissolvable chew. The non-dissolvable chew may include a mouth-stable polymer, such as those described above, and the liquid mixture optionally absorbed in a carrier. The non-dissolvable chew may be a chew as described in U.S. Patent No. 9,854,831, issued January 2, 2018; U.S. Patent No. 10,098,376, issued October 16, 2018; U.S. Patent 9,420,827, issued August 23, 2016; or U.S. Patent No. 9,185,931, issued November 17, 2015, the entire contents of each of which are incorporated herein by this reference hereto, and further including the liquid mixture as described above as an alternative or in addition to tobacco.

In at least one example embodiment, the oral product may be a gel. The gel may be a gel as described in U.S. Patent No. 8,469,036, issued June 25, 2013, the entire contents of which is incorporated herein by this reference hereto, and further including the liquid mixture as described above as an alternative or in addition to tobacco.

Oral products according to at least one example embodiment may be manufactured to have a variety of different sizes and shapes (as described in greater detail below and shown in FIGS. 16A-17). In at least one example embodiment, a size and/or shape of the oral product promotes desired positioning of the oral product within an oral cavity and/or a package. In at least one example embodiment, the oral product may have dimensions ranging from about 0.25 mm to about 30 mm (e.g., about 0.25 to about 1 mm, about 1 mm to about 10 mm, about 1 mm to about 5 mm, about 5 mm to about 25 mm, about 5 mm to about 10 mm, about 10 mm to about 15 mm, about 15 mm to about 20 mm, about 20 mm to about 30 mm, about 20 mm to about 25 mm, or about 25 mm to 30 mm). In at least one example embodiment, the oral product has a first dimension (e.g., smallest dimension or thickness) ranging from about 0.25 mm to about 10 mm (e.g., about 2.5 mm). In at least one example embodiment, the oral product has a largest dimension (e.g., diameter, height, or width) ranging from about 5 mm to about 25 mm (e.g., about 12 mm). The oral product may have a weight ranging from about 1 g to about 10 g (e.g., about 1 g to about 5 g, about 2 g to about 4 g, or about 5 g to about 10 g).

In at least one example embodiment, an oral product, such as a chewing gum, a dissolvable tablet, a chewable tablet, and/or a gel, may define a thickness and a cross-sectional shape perpendicular to a thickness. As used herein, "thickness" refers to the smallest dimension of an oral product. In at least one example embodiment, an oral product may have a substantially uniform thickness. FIGS. 16A-16G depict oral products having different cross-sectional shapes according to at least one example embodiment.

FIG. 16A is a perspective view of an oral product having a circular cross section according to at least one example embodiment.

In at least one example embodiment, as shown in FIG. 16A, an oral product 4600A is provided. The oral product 4600A may have a circular cross section. While not shown, the oral product 4600A may have rounded edges.

FIG. 16B is a perspective view of an oral product having an oval-shaped cross section according to at least one example embodiment.

In at least one example embodiment, as shown in FIG. 16B, an oral product 4600B is provided. The oral product 4600B may have a substantially oval-shaped cross section. In at least one example embodiment, the substantially oval-shaped cross section is a substantially elliptical cross section. While not shown, the oral product 4600B may have rounded edges.

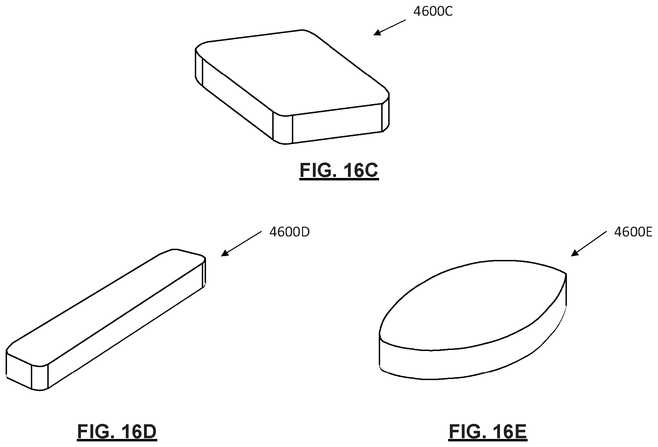

FIG. 16C is a perspective view of an oral product having a rectangular cross section according to at least one example embodiment.

In at least one example embodiment, as shown in FIG. 16C, an oral product 4600C is provided. The oral product 4600C may have a substantially rectangular cross section. In at least one example embodiment, the substantially rectangular cross section is a substantially square cross section. In at least one example embodiment, the rectangular cross section may have rounded corners. While not shown, the oral product 4600C may have rounded edges.

FIG. 16D is a perspective view of an oral product having an elongated rectangular cross section according to at least one example embodiment.

In at least one example embodiment, as shown in FIG. 16D, an oral product 4600D is provided. The oral product 4600D may have an elongated rectangular cross section. The elongated rectangular cross section may have rounded corners. While not shown, the oral product 4600D may have rounded edges.

FIG. 16E is a perspective view of an oral product having a lens or football shaped cross section according to at least one example embodiment.

In at least one example embodiment, as shown in FIG. 16E, an oral product 4600E is provided. The oral product 4600E may have a lens-shaped cross section. In at least one example embodiment, the lens-shaped cross section may have rounded corners. While not shown, the oral product 4600E may have rounded edges.

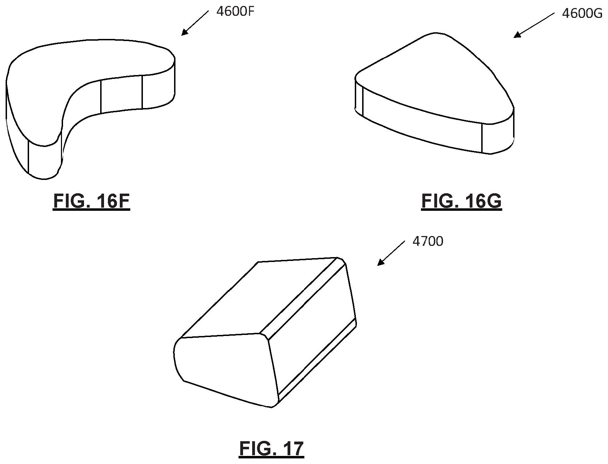

FIG. 16F is a perspective view of an oral product having a boomerang-shaped cross section according to at least one example embodiment.

In at least one example embodiment, as shown in FIG. 16F, an oral product 4600F is provided. The oral product 4600F may have a boomerang-shaped cross section. The boomerang-shaped cross section may have rounded corners. While not shown, the oral product 4600F may have rounded edges.

FIG. 16G is a perspective view of an oral product having a shield-shaped cross section according to at least one example embodiment.

In at least one example embodiment, as shown in FIG. 16G, an oral product 4600G is provided. The oral product 4600G may have a shield-shaped cross section. The cross section may have reflection symmetry about a center plane. In at least one example embodiment, the oral product 4600G includes three rounded corners. In at least one example embodiment, an oral product includes a triangular cross section. While not shown, the oral product 4600G may have rounded edges.

In at least one other example embodiment, an oral product defines another shape. For example, an oral product may have a semi-circular cross-sectional shape, a comma cross-sectional shape, a bowtie cross-sectional shape, or a bean/kidney cross-sectional shape.

In at least one example embodiment, an oral product may have a nonuniform thickness (e.g., a pillow shape). In at least one example embodiment, an oral product does not have a single smallest dimension, such as when the oral product has a spherical shape or a cube shape. In at least one example embodiment, an oral product defines another three-dimensional shape.

FIG. 17 is a perspective view of an oral product according to at least one example embodiment.

In at least one example embodiment, as shown in FIG. 17, an oral product 4700 is provided. The oral product 4700 may have a wedge shape with rounded edges. Rounded edges may be included so as to avoid sharp corners that may cause discomfort when placed in an adult consumer's mouth. In at least one other example embodiment, an oral product may have a hemispherical shape, a semi-cylindrical shape, a twist shape, a spiral shape, a cushion/pillow shape, a rod shape, or a pebble shape.

At least one example embodiment relates to a method of increasing buccal absorption of nicotine in an oral product. The method may include preparing a liquid mixture, such as those described above, and incorporating the liquid mixture into an oral product. In at least one example embodiment, the method includes preparing the liquid mixture prior to incorporation in an oral product. In at least one example embodiment, the method further includes absorbing the liquid mixture on a carrier prior to incorporation in the oral product. In at least one example embodiment, the method further includes forming the oral product.

In at least one example embodiment, preparing the liquid mixture includes dissolving liquid nicotine in a triglyceride, such as MCT. At least a portion of the liquid nicotine is in the free-base state. In at least one example embodiment, a weight ratio of the triglyceride to the liquid nicotine ranges from about 1:1 to about 9:1.

In at least one example embodiment, absorbing the liquid mixture on a carrier may include admixing the liquid mixture and the carrier. The method may include allowing the liquid mixture and the carrier to equilibrate for a desired (or alternative, predetermined) period of time prior to incorporation in an oral product.

Incorporating may include incorporating the liquid mixture into any of the oral products described herein, such as a chewing gum, a spray, a lozenge, a dissolvable tablet, a non-dissolvable chew, a film, a gel, a capsule, or a pouch (e.g., containing fibers or granules). In at least one example embodiment, incorporating includes combining the liquid mixture and optional carrier with one or more of the additional elements described herein. In at least one example embodiment, the oral product may be formed concurrently with incorporating the liquid mixture. In at least one example embodiment, the oral product may be formed prior to incorporation of the liquid mixture, such as by forming a tablet and absorbing the liquid mixture into the formed tablet.

In at least one example embodiment, incorporating includes adding the carrier, such as a cellulosic material, with the liquid mixture absorbed therein, to a gum base to form a chewing gum. In at least one example embodiment, incorporating includes combining the liquid mixture with a soluble fiber and a sugar alcohol to form a lozenge. In at least one example embodiment, incorporating includes admixing the liquid mixture with a carrier liquid to form an oral spray product. In at least one example embodiment, incorporating includes combining the liquid mixture with a mouth-soluble polymer to form a dissolvable oral product. In at least one example embodiment, incorporating includes combining the liquid mixture with a mouth-stable polymer to form a chewable oral product.

In at least one example embodiment, at least a portion of the nicotine remains dissolved in the triglyceride during and after the incorporating. All of the nicotine may remain dissolved in the triglyceride during and after the incorporating. In at least one example embodiment, at least a portion of the nicotine remains in the free-base state during and after the incorporating.

### CONTROLLED-RELEASE CHEWING GUMS

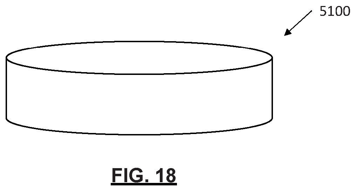

FIG. 18 is a perspective view of a chewing gum according to at least one example embodiment.

In at least one example embodiment, the present disclosure provides a controlled-release nicotine chewing gum 5100. The chewing gum 5100 includes a gum base polymer, an oil, and nicotine or a nicotine derivative. In at least one example embodiment, the gum base polymer includes polyvinyl acetate (PVA) and the oil includes a triglyceride.

In at least one example embodiment, the gum base polymer, such as the PVA, is present a higher amount compared to a typical gum base (e.g., a gum base including a polymer, a sugar alcohol, and a filler, such as calcium carbonate and/or talc, in amounts greater than 10 weight percent), as described in greater detail below. The increased amount of gum base polymer may facilitate controlled release of nicotine. For example, a release rate of nicotine may be decreased as amount of gum base polymer is increased.

In at least one example embodiment, after being chewed by an adult tobacco consumer for about 10 minutes, less than 95 weight percent of the nicotine is released from the chewing gum (e.g., less than 90 weight percent of the nicotine, less than 85 weight percent of the nicotine, or less than 80 weight percent of the nicotine). In at least one example embodiment, after being chewed by an adult tobacco consumer for about 10 minutes, nicotine in an amount ranging from about 75 weight percent to about 90 weight percent is released from the oral product.

In at least one example embodiment, the oil, which may be an admixture of oils, may be present in a higher amount compared to a typical chewing gum. An amount of oil may be selected to achieve a desired chewing gum texture and/or softness. More particularly, softness of the chewing gum may be increased as amount of oil is increased. In at least one example embodiment, the oil includes a triglyceride, triacetin, and a flavor oil, as will be described in greater detail below.

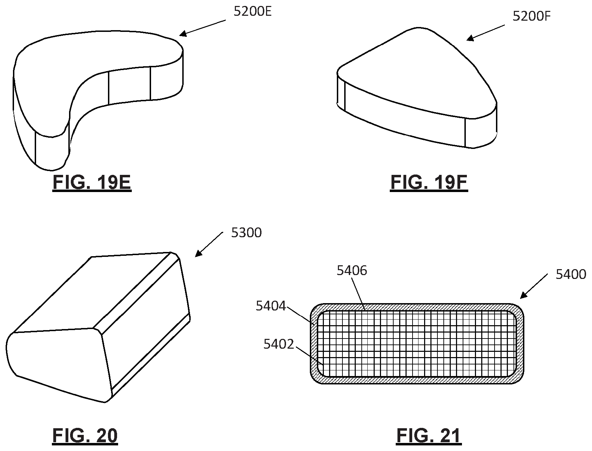

In at least one example embodiment, the chewing gum 5100 is sized and shaped to be wholly received in an oral cavity of an adult tobacco consumer. The chewing gum 5100 may be uncoated, as shown, or coated (shown in FIG. 21). A coated chewing gum may include a body and a coating that partially or fully surrounds the body. Chewing gum weight percentages described herein refer to an uncoated chewing gum or a body of a coated chewing gum.

In at least one example embodiment, the gum base polymer includes a resin, an elastomer, or any combination thereof. The resin may include polyvinyl acetate (PVA), glycerol esters of gum, terpene resins, or any combination thereof, by way of example. The elastomer may include couma macrocarpa, loquat, tunu, jelutong, chicle, styrene-butadiene rubber, butyl rubber, polyisobutylene, or any combination thereof, by way of example. In at least one example embodiment, the gum base polymer includes a natural latex, a vegetable gum (e.g., chicle), a spruce gum, a mastic gum, or any combination thereof. The gum base polymer may include a single polymer or a combination of polymers. In at least one example embodiment, the gum base polymer consists essentially of the PVA.

In at least one example embodiment, the gum base polymer is present in the chewing gum 5100 (i.e., an uncoated chewing gum or the body of a coated chewing gum) in an amount greater than or equal to about 30 weight percent of the chewing gum 5100. For example, the chewing gum 5100 may include the gum base polymer in an amount greater than or equal to about 35 weight percent (e.g., greater than or equal to about 40 weight percent, greater than or equal to about 45 weight percent, greater than or equal to about 50 weight percent, or greater than or equal to about 55 weight percent). The chewing gum 5100 may include the chewing gum polymer in an amount less than or equal to about 60 weight percent (e.g., less than or equal to about 55 weight percent, less than or equal to about 50 weight percent, less than or equal to about 45 weight percent, or less than or equal to about 40 weight percent). In at least one example embodiment, the gum base polymer is present in an amount ranging from about 35 weight percent to about 55 weight percent of the chewing gum 5100 (e.g., about 40 weight percent to about 50 weight percent, about 43 weight percent to about 47 weight percent, about 44 weight percent to about 46 weight percent, or about 45 weight percent).

In at least one example embodiment, the gum base polymer includes PVA. The chewing gum 5100 (i.e., an uncoated chewing gum or the body of a coated chewing gum) may include the PVA in an amount greater than or equal to about 30 weight percent of the chewing gum 5100. For example, the chewing gum 5100 may include the PVA in an amount greater than or equal to about 35 weight percent (e.g., greater than or equal to about 40 weight percent, greater than or equal to about 45 weight percent, greater than or equal to about 50 weight percent, or greater than or equal to about 55 weight percent). The chewing gum 5100 may include the PVA in an amount less than or equal to about 60 weight percent (e.g., less than or equal to about 55 weight percent, less than or equal to about 50 weight percent, less than or equal to about 45 weight percent, or less than or equal to about 40 weight percent). In at least one example embodiment, the PVA is present in an amount ranging from about 35 weight percent to about 55 weight percent of the chewing gum 5100 (e.g., about 40 weight percent to about 50 weight percent, about 43 weight percent to about 47 weight percent, about 44 weight percent to about 46 weight percent, or about 45 weight percent).

In at least one example embodiment, the chewing gum 5100 further includes a polysaccharide to facilitate controlled release of the nicotine or nicotine derivative. The polysaccharide facilitates controlled release of the nicotine by reducing a rate of dissolution and corresponding nicotine release. The polysaccharide may include xanthan gum, guar gum, pullulan, locust bean gum, or any combination thereof. The chewing gum 5100 may include the polysaccharide in an amount greater than or equal to about 0.01 weight percent (e.g., greater than or equal to about 0.05 weight percent, greater than or equal to about 0.1 weight percent, greater than or equal to about 0.2 weight percent, greater than or equal to about 0.5 weight percent, greater than or equal to about 1 weight percent, greater than or equal to about 2 weight percent, greater than or equal to about 3 weight percent, or greater than or equal to about 4 weight percent). The chewing gum 5100 may include the polysaccharide in an amount less than or equal to about 5 weight percent (e.g., less than or equal to about 4 weight percent, less than or equal to about 3 weight percent, less than or equal to about 2 weight percent, less than or equal to about 1 weight percent, less than or equal to about 0.5 weight percent, less than or equal to about 0.5 weight percent, less than or equal to about 0.2 weight percent, or less than or equal to about 0.05 weight percent). The chewing gum 5100 may include the polysaccharide in an amount ranging from 0.2 weight percent to 1 weight percent.

As noted above, in at least one example embodiment, the chewing gum 5100 further includes an oil. The oil may be an admixture of oils. In at least one example embodiment, the oil includes a plasticizer, a flavor oil, or both the plasticizer and the flavor oil.

In at least one example embodiment, the chewing gum 5100 (i.e., an uncoated chewing gum or a body of a coated chewing gum) includes oil in an amount greater than or equal to about 5 weight percent (e.g., greater than or equal to about 8 weight percent, greater than or equal to about 9 weight percent, greater than or equal to about 10 weight percent, greater than or equal to about 11 weight percent, greater than or equal to about 12 weight percent, greater than or equal to about 13 weight percent, or greater than or equal to about 15 weight percent). The chewing gum 5100 may include the oil in an amount less than or equal to about 20 weight percent (e.g., less than or equal to about 15 weight percent, less than or equal to about 14 weight percent, less than or equal to about 13 weight percent, less than or equal to about 12 weight percent, less than or equal to about 11 weight percent, less than or equal to about 10 weight percent, or less than or equal to about 8 weight percent). In at least one example embodiment, the chewing gum 5100 includes the oil in an amount ranging from about 8 weight percent to about 16 weight percent (e.g., about 10 weight percent to about 14 weight percent, about 11 weight percent to about 13 weight percent, or about 12 weight percent).

The plasticizer may include a triglyceride (e.g., long, medium, and/or short chain), triacetin, propylene glycol, glycerin, vegetable oil, a phthalate, an ester of a polycarboxylic acid with a linear or branched aliphatic alcohol of moderate chain length, or any combination thereof. In at least one example embodiment, the chewing gum 5100 includes a triglyceride including a long-chain triglyceride (LCT), a medium-chain triglyceride (MCT), or both LCT and MCT. In at least one example embodiment, the plasticizer includes a triglyceride and another different plasticizer. In at least one example embodiment, the plasticizer includes a triglyceride and triacetin. In at least one example embodiment, the plasticizer includes MCT and triacetin.

In at least one example embodiment, the chewing gum 5100 (i.e., an uncoated chewing gum or a body of a coated chewing gum) includes a triglyceride (e.g., MCT) in an amount greater than or equal to about 4 weight percent (e.g., greater than or equal to about 5 weight percent, greater than or equal to about 6 weight percent, greater than or equal to about 7 weight percent, or greater than or equal to about 8 weight percent). The chewing gum 5100 may include the triglyceride in an amount less than or equal to about 12 weight percent (e.g., less than or equal to about 10 weight percent, less than or equal to about 8 weight percent, less than or equal to about 7 weight percent, less than or equal to about 6 weight percent, or less than or equal to about 5 weight percent). In at least one example embodiment, the chewing gum 5100 includes the triglyceride in an amount ranging from about 4 weight percent to about 8 weight percent (e.g., about 4 weight percent to about 7 weight percent, about 4 weight percent to about 6 weight percent, about 5 weight percent to about 7 weight percent, about 5 weight percent to about 6 weight percent).

In at least one example embodiment, the chewing gum 5100 (i.e., an uncoated chewing gum or a body of a coated chewing gum) includes triacetin in an amount greater than or equal to about 1 weight percent (e.g., greater than or equal to about 2 weight percent, greater than or equal to about 3 weight percent, greater than or equal to about 4 weight percent, or greater than or equal to about 5 weight percent). The chewing gum 5100 may include the triacetin in an amount less than or equal to about 7 weight percent (e.g., less than or equal to about 5 weight percent, less than or equal to about 4 weight percent, less than or equal to about 3 weight percent, or less than or equal to about 2 weight percent). In at least one example embodiment, the chewing gum 5100 includes triacetin in an amount ranging from about 1 weight percent to about 4 weight percent (e.g., about 1 weight percent to about 3 weight percent, about 2 weight percent to about 4 weight percent, or about 2 weight percent to about 3 weight percent).

As noted above, in at least one example embodiment, the oil further includes a flavor oil. The flavor oil may be or include a flavorant. The flavorant may be natural or artificial. In at least one example embodiment, the flavorant includes a fruit flavorant (e.g., bergamot, berry, cherry, lemon, and/or orange), a liquor or liqueur flavorant (e.g., bourbon, cognac, scotch, whiskey, and/or DRAMBUIE brand liqueur), a mint flavorant (e.g., Japanese mint, menthol, peppermint, spearmint, wintergreen, and/or mint oils from a species of the genus Mentha), a floral flavorant (e.g., geranium, lavender, and/or rose), a spice, an herb, or another botanical or botanical-derived flavorant (e.g., anise, apium graveolens, caraway, cardamom, cascarilla, cassia, cinnamon, chamomile, clove, cocoa, coffee, coriander, fennel, ginger, jasmine, licorice, nutmeg, pimenta, sage, sandalwood vanilla, and/or ylang-ylang), honey essence, or any combination thereof. In at least one example embodiment, the flavorant includes bergamot, berry, cherry, lemon, orange, bourbon, cognac, scotch, whiskey, DRAMBUIE brand liqueur, Japanese mint, menthol, peppermint, spearmint, wintergreen, mint oils from a species of the genus *Mentha*, geranium, lavender, rose, anise, *apium graveolens*, caraway, cardamom, cascarilla, cassia, cinnamon, chamomile, clove, coffee, coriander, fennel, ginger, jasmine, licorice, nutmeg, *pimenta*, sage, sandalwood vanilla, ylang-ylang, honey essence, or any combination thereof.

In at least one example embodiment, the flavorant (e.g., spearmint oil and/or peppermint oil) or another component of a flavor oil (e.g., propylene glycol, MCT, and/or triacetin in a complex flavor compound) may act as a plasticizer.

In at least one example embodiment, in addition to the flavorant being present as a flavor oil or alternatively to the flavorant being present as a flavor oil, the chewing gum 5100 may include a non-oil-based flavorant. In at least one example embodiment, the chewing gum 5100 includes an encapsulated flavorant oil and/or an encapsulated non-oil-based flavorant. Non-oil-based flavorants and encapsulated flavorants and flavor oils are not considered to be part of the oil including the nicotine and the triglyceride.

In at least one example embodiment, the chewing gum 5100 includes the flavor oil in an amount greater than or equal to about 0.001 weight percent (e.g., greater than or equal to about 0.01 weight percent, greater than or equal to about 0.1 weight percent, greater than or equal to about 1 weight percent, greater than or equal to about 2 weight percent, greater than or equal to about 3 weight percent, greater than or equal to about 4 weight percent, or greater than or equal to about 5 weight percent). The chewing gum 5100 may include the flavor oil in an amount less than or equal to about 10 weight percent (e.g., less than or equal to about 7 weight percent, less than or equal to about 5 weight percent, less than or equal to about 4 weight percent, less than or equal to about 3 weight percent, less than or equal to about 2 weight percent, less than or equal to about 1 weight percent, less than or equal to about 0.1 weight percent, or less than or equal to about 0.01 weight percent). In at least one example embodiment, the chewing gum 5100 includes the flavor oil in an amount ranging from about 0.001 weight percent to about 6 weight percent (e.g., about 1 weight percent to about 6 weight percent, about 1 weight percent to about 4 weight percent, about 1 weight percent to about 3 weight percent, about 2 weight percent to about 5 weight percent, about 2 weight percent to about 4 weight percent, about 3 weight percent to about 6 weight percent, or about 3 weight percent to about 4 weight percent). In at least one example embodiment, the oil is free of a flavor oil. In at least one example embodiment, the chewing gum is free of a flavorant.

As noted above, in at least one example embodiment, the chewing gum 5100 includes nicotine or a nicotine derivative, such as a complex of nicotine (e.g., nicotine polacrilex) or a salt of nicotine (e.g., nitrate, monotartrate, bitartrate, bitartrate dihydrate, salicylate, sulfate or bisulfate, phosphate or acid phosphate, acetate, lactate, succinate, maleate, fumarate, gluconate, saccharate, benzoate, methanesulfonate, hydrochloride, hydrobromide, hydroiodide, or any combination thereof). The nicotine or nicotine derivative may include tobacco-derived nicotine, synthetic nicotine, or both tobacco-derived nicotine and synthetic nicotine. In at least one example embodiment, the chewing gum 5100 includes liquid nicotine.

In at least one example embodiment, tobacco-derived nicotine may include nicotine and one or more additional organoleptic elements. The tobacco-derived nicotine may, in at least one example embodiment, be extracted from raw (e.g., green leaf) tobacco and/or processed tobacco. Processed tobaccos may include fermented and unfermented tobaccos, dark air-cured, dark fire cured, burley, flue cured, and cigar filler or wrapper, as well as the products from whole leaf stemming operations. The tobacco may also be conditioned by heating, sweating and/or pasteurizing steps. For example. Fermenting typically is characterized by high initial moisture content, heat generation, and a loss of dry weight in a range of about 10 weight percent to about 20 weight percent. By processing the tobacco prior to extracting nicotine and other organoleptic elements, the tobacco-derived nicotine may include ingredients that provide a favorable experience. In at least one example embodiment, the tobacco-derived nicotine may be obtained by mixing cured and fermented tobacco with water and/or another solvent (e.g., ethanol) followed by removing the insoluble tobacco material. The tobacco extract may be further concentrated or purified. In at least one example embodiment, select tobacco materials may be removed from the tobacco extract.

In at least one example embodiment, the chewing gum 5100 includes the nicotine or nicotine derivative in an amount greater than or equal to about 0.1 mg (e.g., greater than or equal to about 1 mg, greater than or equal to about 2 mg, greater than or equal to about 4 mg, or greater than or equal to about 6 mg, greater than or equal to about 8 mg,, greater than or equal to about 10 mg, greater than or equal to about 12 mg). The chewing gum 5100 may include the nicotine or nicotine derivative in an amount less than or equal to about 14 mg (e.g., less than or equal to about 12 mg, less than or equal to about 10 mg, less than or equal to about 8 mg, less than or equal to about 6 mg, less than or equal to about 4 mg, less than or equal to about 2 mg, or less than or equal to about 1 mg). In at least one example embodiment, the chewing gum 5100 includes the nicotine or nicotine derivative in an amount ranging from about 3 mg to about 8 mg.

In at least one example embodiment, the chewing gum 5100 further includes an additive. The additive may include a sweetener, mouth-soluble fibers, an insoluble cellulosic material, an antioxidant, an energizing agent, a soothing agent, a focusing agent, an alkaloid, a mineral, a vitamin, a dietary supplement, a nutraceutical, a coloring agent, an amino acid, a chemesthetic agent, a food-grade emulsifier, a pH-adjuster, a botanical (e.g., green tea), a tooth-whitening agent (e.g., sodium hexametaphosphate (SHMP)), a therapeutic agent, a processing aid, a stearate (e.g., magnesium and/or potassium), a wax (e.g., glycerol monostearate, propylene glycol monostearate, and/or an acetylated monoglyceride), a stabilizer (e.g., ascorbic acid and monosterol citrate, butylated hydroxytoluene (BHT), or butylated hydroxyanisole (BHA)), a lubricant (e.g., sodium lauryl sulfate (SLS)), a preservative (e.g., sodium benzoate), or any combination thereof. The additive may include multiple additives. Additionally, a single additive may belong to more than one of the categories above.

In at least one example embodiment, the additive may be included in an amount greater than or equal to about 0.001 weight percent (e.g., greater than or equal to about 0.005 weight percent, greater than or equal to about 0.01 weight percent, greater than or equal to about 0.05 weight percent, greater than or equal to about 0.1 weight percent, greater than or equal to about 0.5 weight percent, greater than or equal to about 1 weight percent, greater than or equal to about 2 weight percent, greater than or equal to about 5 weight percent, greater than or equal to about 10 weight percent, greater than or equal to about 15 weight percent, greater than or equal to about 20 weight percent, greater than or equal to about 25 weight percent, greater than or equal to about 30 weight percent, greater than or equal to about 35 weight percent, greater than or equal to about 40 weight percent). The additive may be present in an amount less than or equal to about 65 weight percent (e.g., less than or equal to about 60 weight percent, less than or equal to about 55 weight percent, less than or equal to about 50 weight percent, less than or equal to about 45 weight percent, less than or equal to about 40 weight percent, less than or equal to about 35 weight percent, less than or equal to about 30 weight percent, less than or equal to about 25 weight percent, less than or equal to about 20 weight percent, less than or equal to about 15 weight percent, less than or equal to about 10 weight percent, less than or equal to about 5 weight percent, less than or equal to about 1 weight percent, less than or equal to about 0.5 weight percent, less than or equal to about 0.1 weight percent, or less than or equal to about 0.01 weight percent).

In at least one example embodiment, the additive includes a sweetener. The sweetener may include a synthetic sweetener and/or a natural sweetener. The natural sweetener may include a sugar, such as a monosaccharide, a disaccharide, and/or a polysaccharide. In at least one example embodiment, the sweetener includes a natural sweetener including sucrose (i.e., table sugar), honey, a mixture of low-molecular-weight sugars excluding sucrose, glucose (i.e., grape sugar, corn sugar, dextrose), molasses, corn sweetener, glucose syrup (i.e., corn syrup), fructose (i.e., fruit sugar), lactose (i.e., milk sugar), maltose (i.e., malt sugar, maltobiose), sorghum syrup, fruit juice concentrate, or any combination thereof. In at one example embodiment, the sweetener includes a sugar alcohol. The sugar alcohol may include ethylene glycol, glycerol, erythritol, threitol, arabitol, xylitol, ribitol, mannitol, sorbitol, galactitol, fucitol, iditol, inositol, volemitol, isomalt, maltitol, lactitol, maltotriitol, maltotetraitol, polyglycitol, or any combination thereof. In at least one example embodiment, the sweetener includes a non-nutritive sweetener including stevia, saccharin, aspartame, sucralose, acesulfame potassium, or any combination thereof.

In at least one example embodiment, the additive includes mouth-soluble fibers. The mouth-soluble fibers may be configured to dissolve when exposed to saliva in an adult tobacco consumer's mouth at the normal human body temperature. In at least one example embodiment, the mouth-soluble fibers include maltodextrin, psyllium, starch, or any combination thereof. In at least one example embodiment, the mouth-soluble fibers include soluble dietary fibers. In at least one example embodiment additive includes partially-soluble fibers, such as sugar beet fibers.

In at least one example embodiment, the additive includes a mouth-insoluble cellulosic material. The mouth-insoluble cellulosic material may include or be derived from sugar beets, wood pulp, cotton, bran, citrus pulp, grass (e.g., switch grass), willow, poplar, or any combination thereof. The insoluble cellulosic material may be a treated cellulosic material, such as microcrystalline cellulose (MCC), carboxymethyl cellulose (CMC), hydroxypropyl methylcellulose (HPMC), hydroxypropyl cellulose (HPC), or any combination thereof. In at least one example embodiment, the mouth-insoluble cellulosic material includes mouth-insoluble cellulosic fibers.

In at least one example embodiment, the additive may include the energizing agent. In at least one example embodiment, the energizing agent includes caffeine, taurine, glucaronalactone, or any combination thereof. Caffeine may include synthetic caffeine and/or natural caffeine, such as coffee-bean-extracted caffeine. In at least one example embodiment, the chewing gum 5100 includes caffeine in an amount greater than or equal to about 10 mg (e.g., greater than or equal to about 25 mg, greater than or equal to about 50 mg, greater than or equal to about 75 mg, greater than or equal to about 100 mg, greater than or equal to about 150 mg) The caffeine may be included in an amount less than or equal to about 200 mg (e.g., less than or equal to about 150 mg, less than or equal to about 100 mg, less than or equal to about 75 mg, less than or equal to about 50 mg, or less than or equal to about 25 mg).

In at least one example embodiment, the additive may include the soothing agent. In at least one example embodiment, the soothing agent includes theanine, melatonin, or both theanine and melatonin.

In at least one example embodiment, the additive may include the focusing agent. In at least one example embodiment, the focusing agent includes ginkgo biloba.

In at least one example embodiment, the additive may include the coloring agent. The coloring agent may be a natural colorant and/or an artificial colorant. In at least one example embodiment, the chewing gum 5100 is free of a colorant. In at least one example embodiment, the chewing gum 5100 has a white or an off-white color.

In at least one example embodiment, the additive may include the pH adjuster. The pH adjuster may include ammonium carbonate, ammonium bicarbonate, ammonium hydroxide, calcium carbonate, potassium carbonate, potassium bicarbonate, potassium hydroxide, sodium carbonate, sodium bicarbonate, sodium hydroxide, or any combination thereof. The pH adjuster may be included in an amount greater than or equal to about 0.01 weight percent (e.g., greater than or equal to about 0.05 weight percent, greater than or equal to about 0.1 weight percent, greater than or equal to about 0.5 weight percent, or greater than or equal to about 1 weight percent). The pH adjuster may be included in an amount less than or equal to about 2 weight percent (e.g., less than or equal to about 1 weight percent, less than or equal to about 0.5 weight percent, less than or equal to about 0.1 weight percent, or less than or equal to about 0.05 weight percent). In at least one example embodiment, the pH adjuster is present in an amount ranging from about 0.01 weight percent to 2 weight percent. In at least one example embodiment, the chewing gum 5100 is free of a pH adjuster.

In at least one example embodiment, the chewing gum 5100 includes a filler. Fillers may be generally described as elements that do not undergo a physical change before or after mixing. Certain additives described above, such as mouth-insoluble fibers, may be classified as fillers. Filler may additionally or alternatively include dicalcium phosphate, calcium sulfate, a clay, silica, glass particles, glyceryl palmitostearate, sodium stearyl fumarate, talc, or any combination thereof. In at least one example embodiment, the filler may include mouth-insoluble fibers, dicalcium phosphate, calcium sulfate, a clay, silica, glass particles, glyceryl palmitostearate, sodium stearyl fumarate, talc, or any combination thereof.

The chewing gum 5100 may include the filler in an amount less than or equal to 20 weight percent (e.g., less than or equal to 15 weight percent, less than or equal to 10 weight percent, less than or equal to 9 weight percent, less than or equal to 8 weight percent, less than or equal to 7 weight percent, less than or equal to 6 weight percent, less than or equal to 5 weight percent, less than or equal to 4 weight percent, less than or equal to 3 weight percent, less than or equal to 2 weight percent, or less than or equal to 1 weight percent). The chewing gum 5100 may include the filler in an amount greater than or equal to about 0 weight percent (e.g., greater than or equal to about 1 weight percent, greater than or equal to about 2 weight percent, greater than or equal to about 3 weight percent, greater than or equal to about 4 weight percent, or greater than or equal to about 5 weight percent). In at least one example embodiment, the filler may be may be included in an amount ranging from about 0 weight percent to about 8 weight percent.

In the example embodiment shown, the chewing gum 5100 includes a uniform unitary structure. In at least one example embodiment, a chewing gum includes two or more layers. Layers may be separable or inseparable. Layers may have different compositions. For example, layers may have different flavors, amounts of nicotine or nicotine derivative (including no nicotine or nicotine derivative), textures and/or softnesses, and/or be configured to have different nicotine release rates. In at least one example embodiment, to achieve layers having different textures or release rates, the layers may have different amounts of chewing gum polymer (e.g., PVA), polysaccharide, and/or oil (e.g., MCT and/or triacetin).

Chewing gums according to at least one example embodiment may be manufactured to have a variety of different sizes and shapes (as described in greater detail below and shown in FIGS. 18-21). In at least one example embodiment, a size and/or shape of the chewing gum product promotes desired positioning of the chewing gum within an oral cavity and/or a package. In at least one example embodiment, the chewing gum 5100 may have dimensions ranging from about 1 mm to about 25 mm (e.g., about 1 mm to about 10 mm, about 1 mm to about 5 mm, about 5 mm to about 25 mm, about 5 mm to about 10 mm, about 10 mm to about 15 mm, about 15 mm to about 20 mm, or about 20 mm to about 25 mm). In at least one example embodiment, the chewing gum 5100 has a first dimension (e.g., smallest dimension or thickness) ranging from about 1 mm to about 10 mm (e.g., about 2.5 mm). In at least one example embodiment, the chewing gum 5100 has a largest dimension (e.g., diameter, height, or width) ranging from about 5 mm to about 25 mm (e.g., about 12 mm). The chewing gum 5100 may have a weight ranging from about 1 g to about 10 g (e.g., about 1 g to about 5 g, about 2 g to about 4 g, or about 5 g to about 10 g).

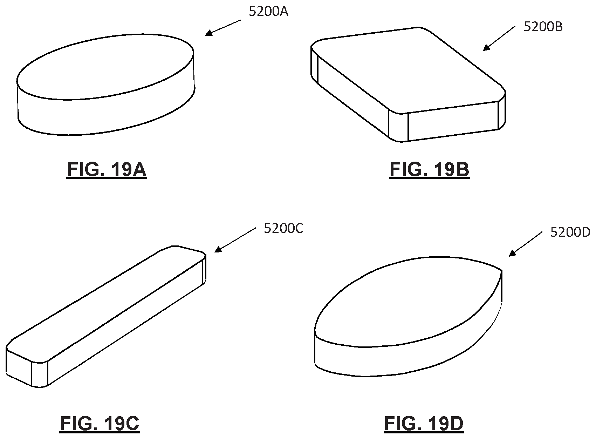

In at least one example embodiment, a chewing gum may define a thickness and a cross-sectional shape perpendicular to a thickness. As used herein, "thickness" refers to the smallest dimension of a chewing gum. In at least one example embodiment, a chewing gum may have a substantially uniform thickness. While not shown, any of the chewing gums described herein may include rounded edges. In at least one example embodiment, a chewing gum is substantially disk-shaped (e.g., the chewing gum 5100 of FIG. 18, which has a substantially circular cross section perpendicular to a thickness). FIGS. 19A-19F depict chewing gums having other cross-sectional shapes according to at least one example embodiment.

FIG. 19A is a perspective view of a chewing gum having an oval-shaped cross section according to at least one example embodiment.

In at least one example embodiment, a chewing gum 5200A is provided. The chewing gum 5200A may be the same as the chewing gum 5100 except that the chewing gum 5200A has a substantially oval-shaped cross section. In at least one example embodiment, the substantially oval-shaped cross section is a substantially elliptical cross section.

FIG. 19B is a perspective view of a chewing gum having a rectangular cross section according to at least one example embodiment.

In at least one example embodiment, a chewing gum 5200B is provided. The chewing gum 5200B may be the same as the chewing gum 5100 except that the chewing gum 5200B has a substantially rectangular cross section. In at least one example embodiment, the substantially rectangular cross section is a substantially square cross section. In at least one example embodiment, the rectangular cross section may have rounded corners.

FIG. 19C is a perspective view of a chewing gum having an elongated rectangular cross section according to at least one example embodiment.

In at least one example embodiment, a chewing gum 5200C is provided. The chewing gum 5200C may be the same as the chewing gum 5100 except that the chewing gum 5200C has an elongated rectangular cross section. The elongated rectangular cross section may have rounded corners.

FIG. 19D is a perspective view of a chewing gum having a lens or football shaped cross section according to at least one example embodiment.

In at least one example embodiment, a chewing gum 5200D is provided. The chewing gum 5200D may be the same as the chewing gum 5100 except that the chewing gum 5200D has a lens-shaped cross section. In at least one example embodiment, the lens-shaped cross section may have rounded corners.

FIG. 19E is a perspective view of a chewing gum having a boomerang-shaped cross section according to at least one example embodiment.

In at least one example embodiment, a chewing gum 5200E is provided. The chewing gum 5200E may be the same as the chewing gum 5100 except that the chewing gum 5200E has a boomerang-shaped cross section. The boomerang-shaped cross section may have rounded corners.

FIG. 19F is a perspective view of a chewing gum having a shield-shaped cross section according to at least one example embodiment.

In at least one example embodiment, a chewing gum 5200F is provided. The chewing gum 5200F may be the same as the chewing gum 5100 except that the chewing gum 5200F has a shield-shaped cross section. The cross section may have reflection symmetry about a center plane. In at least one example embodiment, the chewing gum 5200F includes three rounded corners. In at least one example embodiment, a chewing gum includes a triangular cross section.

In at least one other example embodiment, a chewing gum defines another shape. For example, a chewing gum may have a semi-circular cross-sectional shape, a comma cross-sectional shape, a bowtie cross-sectional shape, or a bean/kidney cross-sectional shape.

In at least one example embodiment, a chewing gum may have a nonuniform thickness (e.g., a pillow shape). In at least one example embodiment, a chewing gum does not have a single smallest dimension, such as when the chewing gum has a spherical shape or a cube shape. In at least one example embodiment, a chewing gum defines another three-dimensional shape.

FIG. 20 is a perspective view of a chewing gum according to at least one example embodiment.

In at least one example embodiment, a chewing gum 5300 is provided. The chewing gum 5300 may be the same as the chewing gum 5100 except that the chewing gum 5300 has a wedge shape with rounded edges. Rounded edges may be included so as to avoid sharp corners that may cause discomfort when placed in the mouth. In at least one other example embodiment, a chewing gum may have a hemispherical shape, a semi-cylindrical shape, a twist shape, a spiral shape, a cushion/pillow shape, a rod shape, or a pebble shape.

FIG. 21 is a cross-sectional view of a chewing gum with a coating according to at least one example embodiment.

In at least one example embodiment, a chewing gum 5400 is provided. The chewing gum 5400 may have any shape, such as those described above and shown in FIGS. 18-20. The chewing gum 5400 includes a body 5402 and a coating 5404 on an outer surface 5406 of the body 5402. The coating 5404 may cover the entire outer surface 5406, as shown, or a portion of the outer surface 5406.

In at least one example embodiment, the coating 5404 may include any of the additives described above. The coating 5404 may include, in at least one example embodiment a mouth-soluble polymer and one or more additional additives. In at least one example embodiment, the coating 5404 may be configured to provide nicotine or a nicotine derivative and/or an additive (e.g., flavorant and/or sweetener) over a different time period than the body 5402. For example, the coating 5404 may be configured to provide an initial burst of nicotine or the additive. The flavorant and/or sweetener may be the same or different than a flavorant and/or sweetener in the body 5402.

In at least one example embodiment, a coating is in the form of flavor strips patterned uniformly or non-uniformly on the body. In at least one example embodiment, a coating includes a flavorant and/or sweetener on the exterior surface of the body, which may be in addition to or as an alternative to a flavorant (e.g., flavor oil) and/or sweetener within the body.

In at least one example embodiment, chewing gums may include functional and/or decorative indicia. In at least one example embodiment, the indicia include a trademark, a product name, an image, a flavor indicator, a decoration, a date, a product identifier, a manufacturer identifier, a lot number, or any combination thereof. In at least one example embodiment, the indicia are embossed or debossed on an exterior surface of a chewing gum. In at least one example embodiment, the indicia are in the form of a dissolvable film on the exterior surface of chewing gum.

At least one example embodiment relates to a method of preparing a controlled-release nicotine chewing gum. The method includes providing a gum base polymer, an oil, and a nicotine or nicotine derivative. In at least one example embodiment, the gum base polymer includes PVA and the oil includes a triglyceride. In at least one example embodiment, the triglyceride includes a medium-chain triglyceride. In at least one example embodiment, the method further includes providing a flavor oil, a polysaccharide, an additive, and/or a filler, such as those described above.

In at least one example embodiment, providing the gum base polymer includes providing the gum base polymer in an amount sufficient to achieve a desired nicotine release rate. In at least one example embodiment, the method further includes determining an amount of gum base polymer based on the desired nicotine release rate. In at least one example embodiment, the method further includes adjusting an amount of gum base polymer based on the desired nicotine release rate.

The gum base polymer may be configured to reduce a rate of nicotine release, such that increasing an amount of the gum base polymer facilitates a reduction in nicotine release rate. In at least one example embodiment, the method includes providing the gum base polymer in an amount ranging from about 35 weight percent to about 55 weight percent of a total chewing gum weight (i.e., a total weight of an uncoated chewing gum or a weight of a body of a coated chewing gum).

In at least one example embodiment, providing the oil includes providing the oil in an amount sufficient to achieve a desired texture and/or softness. In at least one example embodiment, the method may further include determining an amount of oil based on the desired softness. In at least one example embodiment, the method may further include adjusting an amount of oil based on the desired softness.

The oil may be configured to increase a softness of the chewing gum, such that increasing an amount of oil facilitates an increase in chewing gum softness. In at least one example embodiment, the method includes providing the oil in an amount greater than or equal to about 8 weight percent of the total chewing gum weight.

In at least one example embodiment, the method further includes forming the chewing gum from the gum base polymer, the oil, the nicotine or nicotine derivative, and optionally the flavor oil, the polysaccharide, the additive, and/or the filler.

In at least one example embodiment, the chewing gum is formed in a batch process. The batch process may include bringing a heated mixer with to a temperature ranging from about 30°C to about 90°C (e.g., about 60°C). While mixing, a gum base is added to the heated mixture, together with plasticizers, and allowed to melt. Additional elements such as nicotine, oils, sugar alcohols, fillers, flavors, sweeteners, and/or other elements described above are added to the heated mixer. The elements may be added together or in multiple steps, in the order listed or in different orders. A dough is formed and the heated mixer distributes the ingredients evenly throughout the dough by kneading. The dough is discharged and cooled before being sheeted and cut or otherwise separated into portions. In at least one example embodiments, an alternative technique for forming the chewing gum includes extrusion with a rotary cutter and/or sheet extrusion and rolling and scoring operation.

In at least one example embodiment, the method may further include coating the chewing gum. Coating the chewing gum may include pan-coating individual pieces with sugar, sugar alcohols, and/or other elements as described above.

### Example

In at least one example embodiment, an uncoated, controlled-release nicotine chewing gum includes a gum base polymer including PVA at 45 weight percent of the chewing gum. The chewing gum includes an oil at 12 weight percent. The oil includes MCT at 5.8 weight percent, triacetin at 2.5 weight percent, and a balance flavor oil. The chewing gum further includes a filler at 6 weight percent. The filler includes mouth-insoluble fibers. The chewing gum further includes a pH adjuster, an antioxidant, and a bulking agent including a sugar alcohol.

The present disclosure provides methods of enhancing flavor and/or sensory effects of nicotine in oral products, such as gums, sprays, lozenges, dissolvable tablets, non-dissolvable chews, films, gels, capsules, and pouches (e.g., containing fibers or granules). In at least one example embodiment, a nicotine powder is formed that includes spray-drying process. In at least one example embodiment, the nicotine may be encapsulated. In at least one example embodiment, the nicotine powder formed using a spray drying process may be encapsulated. In at least one example embodiment, the oral product may include an encapsulated sweetener. The encapsulated sweetener may be included with the spray dried nicotine powder and/or the encapsulated nicotine.

### ORAL POUCHED PRODUCT

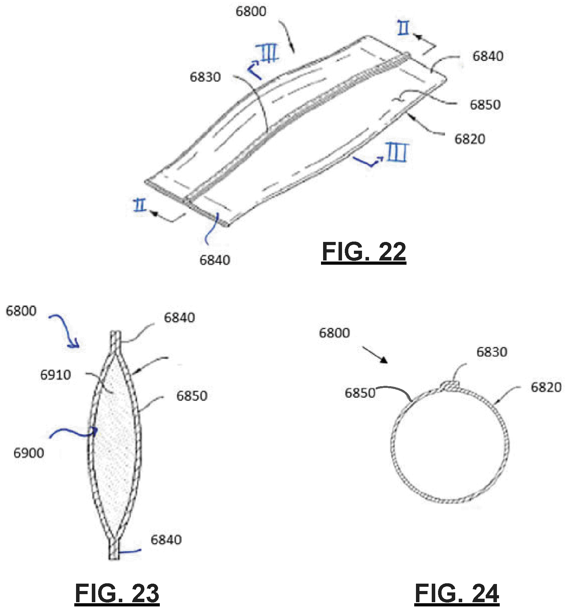

FIG. 22 is a perspective view of an oral pouch product according to at least one example embodiment.

In at least one example embodiment, as shown in FIG. 22, an oral pouch product 6800 is configured to fit in an adult consumer's mouth, and includes a filling material including a dry mixture and the liquid mixture absorbed in the dry mixture as further described with respect to FIG. 23. For example, an adult consumer can suck, chew, or otherwise orally manipulate the oral pouch product 6800 so as to release flavor and/or functional ingredients contained therein. The oral pouch product 6800 may be an oral pouch product having, for example only, a generally rectangular shape. In still other embodiments, the oral pouch product 6800 may have a pouch-shape that is similar to a ravioli or pillow shape, an oblong shape, or any other suitable shape.

Various shapes may be utilized so long as the shapes fit comfortably and discreetly in an adult consumer's mouth. In at least one example embodiment, the oral pouch product 6800 is substantially free of oral cavity irritant, which, as used herein, means that the shape, configuration, and position of the oral pouch product 6800 do not irritate oral tissues (*e.g.*, gums) via sharp edges and the like. Furthermore, "substantial" and "substantially free" as used in connection with oral cavity irritant means that the shape, configuration, and position of the oral pouch product 6800 does not irritate oral tissues (*e.g*., gums) in a time frame or period having the same order of magnitude as a typical length of time during which the oral pouch product 6800 may be enjoyed by an adult consumer. Generally, sharp corners are avoided as sharp corners may lead to oral discomfort.

The oral pouch product 6800 may be sized and configured to fit comfortably in an adult consumer's mouth, such as between the cheek and gum. In at least one example embodiment, the oral pouch product 6800 has a major dimension in the range of about 0.20 inch to about 2.0 inches (*e.g.*, about 0.25 inch to 1.75 inches, about 0.75 inch to about 1.5 inch) and a transverse dimension in the range of about 0.25 to about 1.5 inches (*e.g*., about 0.50 inch to 1.25 inches, about 0.75 inch to about 1.0 inch). The oral pouch product 6800 may weigh about 0.25 gram (g) to about 2.0 grams (*e.g*., about 0.3 gram to about 1.8 grams, about 0.4 gram to about 1.5 grams, about 0.5 gram to about 1.25 grams, or about 0.75 gram to about 1.0 gram).

The oral pouch product 6800 may be placed in an adult consumer's mouth for about 1 minute to about 3 hours (*e.g.*, about 1 minute to about 2 hours, about 5 minutes to about 90 minutes, about 10 minutes to about 60 minutes, about 20 minutes to about 40 minutes). The size of the oral pouch product 6800 may be selected based on desired length of placement in an adult consumer's mouth. For example, a larger pouch including a larger amount of filling material may provide for longer placement. In at least one example embodiment, the oral pouch product 6800 is discarded after a single placement in an adult consumer's mouth.

In at least one example embodiment, as shown in FIG. 22, the oral pouch product 6800 may include a pouch wrapper 6820. The pouch wrapper 6820 can be sealed around one or more edges (*e.g.*, at least one seam) so as to define an inner cavity 6900 that is configured to carry a filling material 6910 (such as discussed below and shown in FIG. 23). For example, the pouch wrapper 6820 can include at least one longitudinal seal 6830 and one or more fin seals 6840. The longitudinal seal 6830 extends between the fin seals 6840. In at least one example embodiment, the oral pouch product 6800 is formed on a high speed, vertical fill and seal machine.

In at least one example embodiment, the pouch wrapper 6820 includes an outer web 6850. The outer web 6850 may have a thickness of about 0.05 mm to about 0.25 mm (*e.g*., about 0.075 mm to about 0.100 mm or about 0.1 mm to about 0.20 mm). The outer web 6850 can be formed of a permeable material or a semi-permeable material, such that, for example, saliva, water, or both saliva and water can pass through the outer web 6850 and into the inner cavity 6900 defined by the pouch wrapper 6820. Flavors and juices formed by mixing of the saliva and/or water with the filling material 6910 contained within the oral pouch product 6800 can be drawn out of the oral pouch product 6800 through the outer web 6850.

In at least one example embodiment, the outer web 6850 is formed from a material that is generally recognized as safe ("GRAS") for use and/or contact with food. The material may be stain resistant, water permeable, and/or porous. For example, in at least one example embodiment, the outer web 6850 comprises a paper. For example, the web can be formed of a cellulose fiber material, such as tea bag material or other materials typically used to form snus pouches. In at least one example embodiment, the outer web 6850 has a desired (or alternatively, predetermined) level for basis weight and/or wet strength so as to reduce occurrence of breakage of the pouch wrapper 6820 during manufacturing operations, storage, and placement in an adult consumer's mouth. For example, the outer web 6850 may comprise a tea bag material having a basis weight of about 16.5 g/m² with a wet tensile CD strength of 68 N/m. In another example embodiment, the outer web 58 may be formed of a paper having a wet MD tensile strength of about 45 N/mm to about 52 N/mm.

In at least one example embodiment, the outer web 6850 is formed of a hydrophobic paper or material. The hydrophobic paper may be formed of a cellulosic material. The hydrophobic paper may be non-woven material, and may include any hydrophobic materials. The hydrophobic materials may be synthetic materials and/or semi-synthetic materials. The hydrophobic materials may include viscose, rayon, lyocell, polyester, polyurethane, polypropylene, polyethylene, and/or modal fibers. The outer web 6850 may be treated to make the outer web 6850 hydrophobic. In other example embodiments, the hydrophobic material may be a woven material.

The material used to form the outer web 6850 may have a neutral or pleasant taste and/or aroma. Further, the outer web 6850 may be substantially white in color. However, in other example embodiments, the outer web 6850 may be colored so as to indicate a flavor of the inner filling material 6910 contained therein.

In at least one example embodiment, the outer web 6850 may be impregnated or coated with at least one flavorant, at least one cannabis material, at least one tobacco material, at least one binder, at least one sensate or chemesthesis agent, at least one functional ingredient, at least one salivation inducing ingredient, or any combination thereof so as to enhance a flavor of the filling material 6910 (shown in FIG. 23) contained within the oral pouch product 6800. A substantially continuous coating including the at least one flavorant and/or the at least one cannabis material and/or at least one tobacco material and/or the at least one binder and/or the at least one sensate or chemesthesis agent may be coated on outer (exterior facing) surfaces of the outer web 6850. In at least one example embodiment, the coating may be formed on only a portion of the outer web 6850, such as only along the seams or only on one side of the pouch 6800. The coating can provide an initial flavor burst upon placement of the oral pouch product 6800 in an oral cavity, while the inner filling material 6910 provides a later flavor release so as to prolong flavor release during placement in an adult consumer's mouth.

The at least one flavorant may be any flavorant disclosed herein for inclusion in the liquid mixture including nicotine and a triglyceride. In at least one example embodiment, the at least one flavorant may be coated on or impregnated in the outer web 6850 in an amount ranging from about 0.01 % by weight to about 5 % by weight based on the weight of the oral pouch product 800 (*e.g*., about 0.1 wt.% to about 4.5 wt.%, about 1 wt.% to about 4 wt.%, about 1.5 wt.% to about 3.5 wt.%, about 2 wt.% to about 3 wt.%).

In at least one example embodiment, the at least one cannabis or tobacco material may be coated on or impregnated in the outer web 6850. The at least one cannabis or tobacco material may include, for example only, a ground cannabis or tobacco material, cannabis or tobacco plant fibers, and/or any extract thereof. The at least one cannabis or tobacco material may be coated on or impregnated in the outer web 6850 in an amount ranging from about 0.01% by weight to about 5% by weight based on the weight of oral pouch product 6800 (*e.g*., about 0.1 wt.% to about 4.5 wt.%, about 1 wt.% to about 4 wt.%, about 1.5 wt.% to about 3.5 wt.%, about 2 wt.% to about 3 wt.%).

In at least one example embodiment, the at least one binder may be coated on or impregnated in the outer web 6850. The at least one binder is a food grade adhesive, gum, or other binder. For example, in some example embodiments, the at least one binder includes, without limitation, sodium alginate, sugar, agar, guar gum, and the like. The at least one binder may be coated on or impregnated in the outer web in an amount ranging from about 0.01% by weight to about 5% by weight based on the weight of the oral pouch product 6800 (*e.g*., about 0.1 wt.% to about 4.5 wt.%, about 1 wt.% to about 4 wt.%, about 1.5 wt.% to about 3.5 wt.%, about 2 wt.% to about 3 wt.%).

The at least one sensate or chemesthesis agent may be coated on or impregnated in the outer web 6850. The at least one sensate or chemesthesis agent may include mint, menthol, cinnamon, pepper, jambu, or any combination thereof. In certain embodiments, the at least one sensate or chemesthesis agent may include any soothing, cooling, and/or warming agent. For example, in some example embodiments, the at least one sensate or chemesthesis agent may include capsaicin, pipeline, alpha-hydroxy-sanshool, and (8)-gingerole,/ which may be selected so as to provide a warm, tingling or burning sensation. In other example embodiments, the at least one sensate or chemesthesis agent may include menthol, menthyl lactate, WS-3 (N- Ethyl-p-menthane-3-carboxamide), WS-23 (2-Isopropyl-N,2,3-trimethylbutyramide) and Evercool 180^{™} (available from Givaudan SA), which may be selected so as to provide a cooling sensation. The at least one sensate or chemesthesis agent may be coated on or impregnated in the outer web 6850 in an amount ranging from about 0.01% by weight to about 5% by weight based on the weight of the oral pouch product 6800 (*e.g.*, about 0.1 wt.% to about 4.5 wt.%, about 1 wt.% to about 4 wt.%, about 1.5 wt.% to about 3.5 wt.%, about 2 wt.% to about 3 wt.%).

In at least one example embodiment, the at least one functional ingredient may include an antioxidant, a soothing agent, an energizing agent, an effervescent, or any combination thereof. The antioxidant may include, for example, vitamin C, vitamin B, magnesium, calcium, or any combination thereof. The soothing agent may include, for example only, chamomile, lavender, jasmine, theanine, melatonin, soursop, cannabidiol, or any combination thereof. The soothing agent can be added as a flavorant and or aroma embedded in the product and/ or the package. The energizing agent may include, for example only, caffeine, taurine, guarana, vitamin B6, vitamin B12, and the like. The effervescent agent may include, for example, carbon dioxide embedded in the flavor and/or the filling material. In at least one example embodiment, the at least one functional ingredient may be coated on or impregnated in the outer web 6850 in an amount ranging from about 0.01% by weight to about 5% by weight based on the weight of the oral pouch product 6800 (*e.g*., about 0.1 wt.% to about 4.5 wt.%, about 1 wt.% to about 4 wt.%, about 1.5 wt.% to about 3.5 wt.%, about 2 wt.% to about 3 wt.%).

In at least one example embodiment, the at least one soothing ingredient may be coated on or impregnated in the outer web 6850 in an amount ranging from about 0.01% by weight to about 5% by weight based on the weight of the oral pouch product 6800 (*e.g*., about 0.1 wt.% to about 4.5 wt.%, about 1 wt.% to about 4 wt.%, about 1.5 wt.% to about 3.5 wt.%, about 2 wt.% to about 3 wt.%).

FIG. 23 is a cross-sectional view of the oral pouch product along line II-II of FIG. 22 according to at least one example embodiment.

FIG. 24 is a cross-sectional view of the oral pouch product along line III-III of FIG. 22 according to at least one example embodiment.

In at least one example embodiment, as shown in FIG. 23, opposing layers of the outer web 6850, the longitudinal seal 6830, and the fin seals 6840 define an inner cavity 6900 therebetween. The inner filling material 6910 may be held within the inner cavity 6900. In at least one example embodiment, the inner filling material 6910 completely fills the interior (*e.g.*, cavity) 6900 of the oral pouch product 6800. In other example embodiments, the inner filling material 6910 only partially fills the interior (*e.g.*, cavity) 6900 of the oral pouch product 6800.

In at least one example embodiment, the inner filling material 6910 has a moisture content ranging from about 4% by weight based on the weight of the oral pouch product to about 8% by weight based on the weight of the oral pouch product 6800 (*e.g*., about 7 wt.% to about 90 wt.%, about 10 wt.% to about 90 wt.%, about 10 wt.% to about 50 wt.%).

In at least one example embodiment, the inner filling material 6910 includes a dry mixture and the liquid mixture, the liquid mixture including liquid nicotine and a triglyceride, such as MCT. In at least one example embodiment, the dry mixture includes a non-tobacco cellulose material, and the liquid mixture is absorbed in the cellulose. The dry mixture may further include dry flavor enhancers, such as a salt and pH adjusters, such as sodium bicarbonate, in addition to the cellulose. High intensity sweeteners, such as sucralose, palatinose, stevia, Acesulfame K, and any combination thereof may be pre-blended with liquid humectants, such as glycerin, propylene glycol, and any combination thereof. The pre-blended mixture can be mixed with the dry mixture. The liquid mixture including MCT and nicotine can be added to the dry mixture last so as to reduce exposure to oxygen. Antioxidants may be further added to the liquid mixture to extend shelf-life and/or stabilize nicotine.

For example, in at least one example embodiment, the inner filling material 6910 includes about 20% to about 80% by weight of a non-dissolvable cellulose (e.g., about 30% to about 70%, about 40% to about 60%, or about 45% to about 55%), about 0% to about 5% of a pH adjuster (e.g., about 1% to about 4% or about 2% to about 3%), such as sodium bicarbonate, about 10% to about 30% of a triglyceride (e.g., about 15% to about 25%, about 18% to about 23%, or about 20% to 22%) such as a medium chain triglyceride, and a sweetener, such as stevia or a combination of sucralose and palatinose.

By including a higher level of triglyceride, such as MCT, the oral pouch product 6800 maintains a relatively moist mouth feel, while maintaining a moisture content of about 4% to about 8% and a water activity level below about 0.6, which can aid in preventing and/or reducing microbial growth and increasing stability of nicotine in the oral pouch product 6800. Further, when a hydrophobic pouch wrapper is used, since the inner filling material predominately includes oils instead of water, the flavors from the inner filling material 6910 more readily pass through the pouch wrapper than where a hydrophilic pouch wrapper is used.

In at least one example embodiment, the oral pouch product 6800 may further include cannabis. The cannabis can include any portion of the cannabis plant and/or any extract therefrom. For example, in some example embodiments, the plant material may include a mixture of *Cannabis sativa* and *Cannabis indica*, such as, for example only, a mixture of about 70% sativa and about 30% indica. In at least one example embodiment, the oral pouch product 6800 may include a cannabis extract applied to cellulose. In at least one example embodiment, the oral pouch product 6800 may include cannabis in addition to other plant material. Alternatively, the oral pouch product 6800 may include other plant materials, such as herbs, vegetables, and the like. In at least one example embodiment, the inner filling material 6910 can also include tobacco material in addition to or in lieu of the cannabis material.

In at least one example embodiment, prior to placement of the cannabis (or other plant material) in the oral pouch product 6800, the cannabis (or other plant material) may be heated to a temperature sufficient to decarboxylate a compound within the cannabis (or other plant material). For example, cannabis may be maintained at the heated temperature for a time period that is sufficient to cause decarboxylation (i.e., to convert tetrahydrocannabinolic acid ("THCA") that is present in the cannabis to tetrahydrocannabinol ("THC"), and/or to convert cannabidiolic acid ("CBDA") to cannabidiol ("CBD")). For some applications, maintaining the plant material at the heated temperature causes the decarboxylation of the cannabis in accordance with an article by Dussy, et al., entitled "Isolation of Delta9-THCA-A from hemp and analytical aspects concerning the determination of Delta9-THC in cannabis products (Forensic Sci. Int. 2005 Apr 20;149(1):3-10), the entire disclosure of which is incorporated herein by reference, and/or an article by Veress, et al., entitled "Determination of cannabinoid acids by high-performance liquid chromatography of their neutral derivatives formed by thermal decarboxylation: I. Study of the decarboxylation process in open reactors" (Journal of Chromatography A 520:339-347, November 1990), the entire disclosure of which is incorporated herein by reference. For example, the cannabis (or other plant material) may be maintained at approximately 225 degrees Celsius for approximately 35 to 45 minutes.

As shown in FIG. 23, in at least one example embodiment, the inner filling material 6910 includes the plant material (*e.g.*, cannabis, tobacco, herbs, vegetables, or any combination thereof) and an additive. The additive may include at least one binder, at least one sensate or chemesthesis agent, at least one functional ingredient, at least one salivation inducing ingredient, at least one humectant, at least one sweetener, or any combination thereof (*e.g*., at least one flavorant and/or at least one sensate or chemesthesis agent and/or at least one humectant and/or at least one sweetener). The color of the pouch wrapper 6820 may be selected so as to identify contents thereof. For example, a green pouch wrapper 6820 may be used to identify an oral pouch product 6800 including a mint flavorant, while a red pouch wrapper 6820 may be used to identify an oral pouch product 6800 including cinnamon. In other example embodiments, the pouch wrapper 6820 may be white and may include the liquid mixture absorbed in cellulose.

In at least one example embodiment, the at least one flavorant may be any of the flavorants used in a coating of the pouch wrapper 6820 as described herein. The inner filling material 6910 can include an amount of the at least one flavorant of about 0.01% to about 25% by weight based on the weight of the oral pouch product 6800 (e.g., about 0.1 wt.% to about 20 wt.%, about 1 wt.% to about 15 wt.%, about 5 wt.% to about 10 wt.%).

The inner filling material 6910 can include the at least one sensate or chemesthesis agents used in a coating of the pouch wrapper 6820 as described herein. The sensates and/or chemesthesis agents may be included to provide a cooling sensation. The inner filling material 6910 can include an amount of the at least one sensate or chemesthesis agent of about 0.01 % to about 25 % by weight based on the weight of the oral pouch product 6800 (e.g., about 0.1 wt.% to about 20 wt.%, about 1 wt.% to about 15 wt.%, about 5 wt.% to about 10 wt.%).

The inner filing material 6910 can include the at least one humectant so as to help maintain the moisture levels of the inner filling material 6910 of the oral pouch product 6800. Examples of humectants that can be used include glycerol and propylene glycol. The inner filling material 6910 can include an amount of the at least one humectant of about 0.01 % to about 15 % by weight based on the weight of the oral pouch product 6800 (e.g., about 1 wt.% to about 12 wt.%, about 5 wt.% to about 10 wt.%, about 7 wt.% to about 9 wt.%).

In at least one example embodiment, as set forth above, the inner filling material 6910 can include the at least one sweetener. The at least one sweetener may include natural or artificial sweeteners. In at least one embodiment, sweeteners include, without limitation, water soluble sweeteners such as monosaccharides, disaccharides, and polysaccharides (such as xylose, ribose, sucrose, maltose, fructose, glucose, and mannose). In other example embodiments, sugar alcohols such as xylitol, mannitol, sorbitol and maltitol can be included. Non-nutritive artificial sweeteners, such as sucralose may also be used. The inner filling material 6910 can include at least one sweetener in an amount ranging from about 0.01 % to about 15 % by weight based on the weight of the oral pouch product 6800 (e.g., about 1 wt.% to about 12 wt.%, about 5 wt.% to about 10 wt.%, about 7 wt.% to about 9 wt.%).

In at least one embodiment, the inner filling material 6910 of the oral pouch product 6800 may include at least one functional ingredient. The at least one functional ingredient may include an antioxidant, a soothing agent, an energizing agent, an effervescent, or any combination thereof. For example, the inner filling material 6910 may include an amount of the at least one functional ingredient of about 0.01 % to about 15 % by weight based on the weight of the oral pouch product 6800 (e.g., about 1 wt.% to about 12 wt.%, about 5 wt.% to about 10 wt.%, about 7 wt.% to about 9 wt.%). The antioxidant, the soothing agent, the energizing agent, and/or the effervescent agent may be any of those used in a coating for the pouch wrapper 6820 as described herein.

In at least one embodiment, the one or more materials disposed in the cavity 6900 as the inner filling material 6910, including, for example only, the one or more additives and/or one or more functional ingredients, may be provided in the form of a plurality of capsules, microcapsules, and/or beads of various sizes. The capsules, microcapsules, and/or beads may have a size that is determined by the desired size of the final product (*e.g.*, oral pouch product 6800). For example, the capsules, microcapsules, and/or beads may range in size from about 0.1 mm to about 8 mm depending on the ingredients contained therein.

In each instance, each capsule, microcapsule, and/or bead may include an outer shell and an inner core. Varying the thicknesses of the outer shells of the capsules, microcapsules, and/or beads included can allow for the ingredients contained in each of the capsules, microcapsules, and/or beads to be released at varying rates so as to prolong the flavor and/or functional experience of the oral pouch product 6800. In some example embodiments, the shells range in thickness from about 0.1 mm to about 7 mm, depending on the size of the capsules, microcapsules, and/or beads and a desired dissolution rate. The capsules, microcapsules, and/or beads having the thinnest shells dissolve first to release the enclosed flavors and functional ingredients. Capsules, microcapsules, and/or beads having thicker shells dissolve at a slower rate so as to provide continued flavor and functional ingredients.

In at least one example embodiment, as shown in FIG. 24, the pouch wrapper 6820 can include at least one longitudinal seal 6830 and one or more fin seals 6840 (shown in FIGS. 22 and 23). As illustrated in FIG. 24, the longitudinal seal 6830 may include overlapping edge portions of the outer web 6850 that are sealed together. The sealing function can be accomplished by a food grade adhesive or by mutually sealing the overlapping edge portions, using thermal or sonic techniques.

As illustrated in FIG. 23, each fin seal 6840 is formed by bringing together an inner surface of the outer web 6850 of the pouch wrapper 6820 and another section of the inner surface of the outer web 6850 in a superposed relation to form a fin seal. The fin seal can then be sealed using any method such as detailed above to form a fin seal 6840. Though not illustrated, in certain embodiments, integrated fin and longitudinal seals may be used, as would be recognized by the skilled artisan. By overlapping a fin seal, the oral pouch product 6800 may be made more comfortable for insertion in the adult consumer's mouth because there are no loose, unsealed edges that may stick out and snag the consumer's mouth during enjoyment. In addition, integrated fin and longitudinal seals may be stronger so as to reduce and/or prevent breakage during manufacture, packaging, shipment, placement, and/or use of the oral pouch product 6800.

Methods of making the oral pouch product 6800 may generally include forming a wrapper into an open pouch using a vertical or horizontal fill machine and filling the open pouch with an inner filling material 6910. The pouch may then be sealed to contain the inner filling material 6910 and form the oral pouch product 6800. In at least one example embodiment, a series of oral pouch product 6800 are formed with a space between seals of adjacent pouch products 6800 and then cut apart to form individual pouch products 6800. For instance, the oral pouch product 6800 may be cut with a die at a location between adjacent seals so as to form a soft edge on each pouch product 6800. Alternatively, a first strip of pouch wrapper material can be advanced along a feed path, filling material can be placed on the strip, a second strip can be placed over the first strip, a sealing die can be used to press the strips together and form a seam such as a heat seal or adhesive seal around the filling, and a cuffing die can be used to cut the first and second strips outwardly of the seam to form the soft edge.

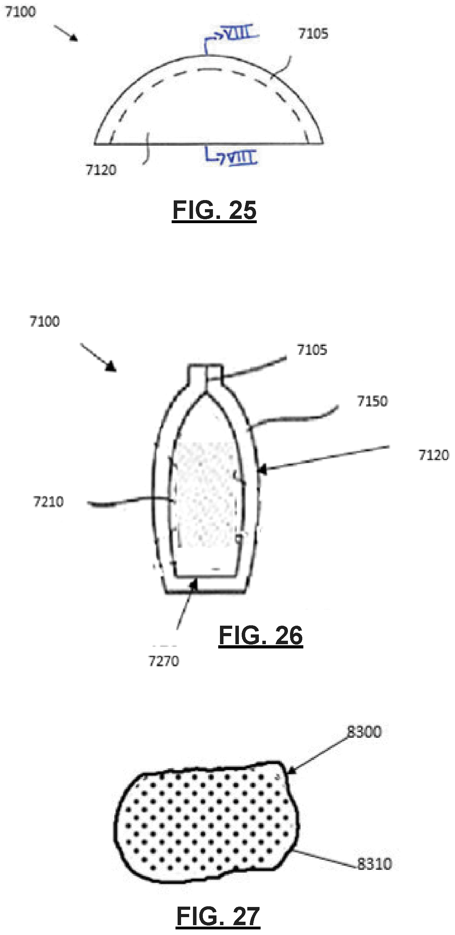

FIG. 25 is a side view of an oral pouch product according to at least one example embodiment.

FIG. 26 is a cross-sectional view along line VII-VII of the oral pouch product of FIG. 25 according at least one example embodiment.

In at least one example embodiment, as shown in FIGS. 25 and 26, the oral pouch product 7100 is the same as that of FIGS. 22-24 except that the oral pouch product 7100 has a single seam or seal 7105 along the wrapper 7120. In at least one example embodiment, as shown, the oral pouch product 7100 has a half moon shape. In some example embodiments, the pouch oral pouch product 7100 has a D-shape, boomerang, crescent, or other shape.

In at least one example embodiment, the pouch wrapper 7120 can be sealed along the seam or seal 7105 so as to define the inner cavity 7270 that is configured to contain or hold the filling material 7210, which is the same as described with respect to FIGS. 22-24. The single seam or seal 7105 can be formed by bringing together an inner surface of the outer web 7150 of the pouch wrapper 7120 and another section of the inner surface of the outer web 7150 in a superposed relation. The sealing function can be accomplished by a food grade adhesive or by mutual sealing the adjacent portions, using thermal or sonic techniques.

FIG. 27 is a cross-sectional view of an oral pouch product according to at least one example embodiment.

In other example embodiments, an oral pouch product 8300 is the same as the pouch product 6800 of FIGS. 22-27, except that the outer web 8310 is a non-woven material formed of a polymer, including synthetic or natural polymer. For example, the outer web 8310 may be formed of a mesh material formed of spun or melt-blown fibers, such as polyurethane fibers as described in U.S. Patent No. 10,448,669, U.S. Patent No. 10,463,070, and/or U.S. Patent No. 9,414,624, the entire contents of each of which is incorporated herein by reference thereto. The mesh material may be at least partially elastomeric. Further, because of the material used to form the pouch product 8300, the pouch product 8300 may exclude seams so as to provide a softer pouch. In at least one example embodiment, the mesh material encloses a filling material including cellulose and the liquid mixture including a triglyceride and liquid nicotine.

While some example embodiments have been disclosed herein, it should be understood that other variations may be possible. Such variations are not to be regarded as a departure from the spirit and scope of the present disclosure, and all such modifications as would be obvious to one skilled in the art are intended to be included within the scope of the following claims.

Although described with reference to specific examples and drawings, modifications, additions and substitutions of example embodiments may be variously made according to the description by those of ordinary skill in the art. For example, the described techniques may be performed in an order different with that of the methods described, and/or elements such as the described system, architecture, devices, circuit, and the like, may be connected or combined to be different from the above-described methods, or results may be appropriately achieved by other elements or equivalents.

The invention may further be described without limitation and by way of example only by the following embodiments.
1. An oral pouch product comprising:
   an outer wrapper defining a cavity, the outer wrapper including,
      a hydrophobic material; and
   an inner filling material in the cavity, the inner filling material including,
      a dry mixture including a cellulosic material, and
      a liquid mixture including,
         a triglyceride, and
         liquid nicotine dissolved in the triglyceride, the triglyceride included in the oral pouch product in an amount of 10% to 30% by weight based on the weight of the oral pouch product.
2. The oral pouch product of claim 1, wherein the triglyceride includes a medium-chain triglyceride.
3. The oral pouch product of claim 1, wherein the liquid mixture is free of water.
4. The oral pouch product of claim 1, where the oral pouch product has a moisture content of 4% to 8%.
5. The oral pouch product of claim 1, wherein the liquid mixture is absorbed in the cellulosic material.
6. The oral pouch product of claim 1, wherein the cellulosic material includes microcrystalline cellulose.
7. The oral pouch product of claim 1, wherein the liquid nicotine is present in the oral pouch product in an amount ranging from 4 mg per pouch to 12 mg per pouch.
8. The oral pouch product of claim 1, wherein the filler further comprises at least one additive.
9. The oral pouch product of claim 8, wherein the additive comprises a sweetener, a pH adjuster, a flavorant, a sensate, or any combination thereof.
10. The oral pouch product of claim 1, wherein the outer wrapper includes at least one seam.
11. The oral pouch product of claim 1, wherein the oral pouch product weighs 0.25 gram to 2.0 grams.
12. The oral pouch product of claim 1, wherein the outer wrapper is a non-woven material.
13. An oral pouch product comprising:
   an outer wrapper defining a cavity, the outer wrapper including,
      an elastomeric material; and
   an inner filling material in the cavity, the inner filling material including,
      a dry mixture including a cellulosic material, and
      a liquid mixture including,
         a triglyceride, and
         liquid nicotine dissolved in the triglyceride, the triglyceride included in the oral pouch product in an amount of 10% to 30% by weight based on the weight of the oral pouch product, and the oral pouch product having a moisture content of 4% to 8%.
14. The oral pouch product of claim 13, wherein the triglyceride includes a medium-chain triglyceride.
15. The oral pouch product of claim 13, wherein the liquid mixture is free of water.
16. The oral pouch product of claim 13, wherein the elastomeric material is a non-woven material including polyurethane.
17. The oral pouch product of claim 13, wherein the outer wrapper has no seams.
18. The oral pouch product of claim 13, wherein the filler further comprises at least one additive.
19. The oral pouch product of claim 18, wherein the additive comprises a sweetener, a pH adjuster, a flavorant, a sensate, or any combination thereof.
20. The oral pouch product of claim 13, wherein the oral pouch product weights about 0.25 gram to 2.0 grams.

## Claims

1. An oral pouch product comprising:
an outer wrapper defining a cavity, the outer wrapper including,
a polymeric material; and
an inner filling material in the cavity, the inner filling material including,
a dry mixture including a cellulosic material, and
a liquid mixture including,
a triglyceride, and
nicotine dissolved in the triglyceride,
the oral pouch product having a moisture content of less than 8 weight percent.

2. The oral pouch product of claim 1, wherein the inner filling material includes the triglyceride in an amount ranging from 10 weight percent to 30 weight percent.

3. The oral pouch product of any one of claims 1 and 2, wherein the inner filling material includes the triglyceride in an amount ranging from 15 weight percent to 30 weight percent.

4. The oral pouch product of any one of the preceding claims, wherein the polymeric material includes a hydrophobic material.

5. The oral pouch product of any one of the preceding claims, wherein the outer wrapper further includes a nonwoven material including the polymeric material.

6. The oral pouch product of any one of the preceding claims, wherein the polymeric material includes polyurethane.

7. The oral pouch product of any one of the preceding claims, wherein the outer wrapper includes a seam.

8. The oral pouch product of any one of the preceding claims, wherein the liquid mixture includes water in an amount of less than or equal to 5 weight percent.

9. The oral pouch product of any one of claims 1 to 7, wherein the liquid mixture is free of water.

10. The oral pouch product of any one of the preceding claims, wherein the oral pouch product has a water activity of less than or equal to 0.6.

11. The oral pouch product of any one of the preceding claims, wherein the liquid mixture further includes an antioxidant.

12. The oral pouch product of any one of the preceding claims, wherein the cellulosic material includes microcrystalline cellulose.

13. The oral pouch product of any one of the preceding claims, wherein the inner filling material further includes at least one of a plasticizer, a flavorant and a sweetener.

14. The oral pouch product of any one of the preceding claims, wherein the inner filling material further includes a pH adjuster.

15. The oral pouch product of any one of the preceding claims, wherein the oral pouch product weighs 0.25 gram to 2.0 grams.
